# EUROPEAN PATENT SPECIFICATION

(11) **EP 1 289 980 B1**
(45) Date of publication and mention of the grant of the patent: **17.11.2004**
(21) Application number: 01945174.9
(22) Date of filing: 21.05.2001
(51) Int. Cl.: C07D 333/60, C07D 409/12, C07D 307/81, C07D 215/14, A61K 31/381, A61K 31/343, A61K 31/47, A61P 29/00, A61P 37/00

(54) **BICYCLYL OR HETEROBICYCLYLMETHANESULFONYLAMINO-SUBSTITUTED N-HYDROXYFORMAMIDES**
BICYCLYL ODER HETEROBICYCLYL METHANESULFONYLAMINO-SUBSTITUIERTE N-HYDROXYFORMAMIDE
N-HYDROXYFORMAMIDES A SUBSTITUTION BICYCLYLE OU HETEROBICYCLYLMETHANESULFONYLAMINO

(30) Priority: 25.05.2000 GB 0012809; 28.02.2001 GB 0104970
(43) Date of publication of application: 12.03.2003
(73) Proprietor: SMITHKLINE BEECHAM PLC, Brentford, Middlesex TW8 9GS (GB)
(72) Inventor: BEST, Desmond John GlaxoSmithKline, Harlow Essex CM19 5AW (GB); BRUTON, Gordon GlaxoSmithKline, Harlow Essex CM19 5AW (GB); ORLEK, Barry Sidney GlaxoSmithKline, Harlow Essex CM19 5AW (GB); RANA, Kishore GlaxoSmithKline, Harlow Essex CM19 5AW (GB); WALKER, Graham GlaxoSmithKline, Harlow Essex CM19 5AW (GB)
(74) Representative: West, Vivien
(86) International application number: PCT/EP2001/005798
(87) International publication number: WO 2001/090100

(56) References cited:
- WO-A-00/12478
- WO-A-98/42659
- WO-A-99/06361
- WO-A-99/38843
- WO-A-99/40080

## Description

This invention relates to novel inhibitors of the formation of soluble human CD23 and their use in the treatment of conditions associated with excess production of soluble CD23 (s-CD23) such as autoimmune disease and allergy.

CD23 (the low affinity IgE receptor FceRII, Blast 2), is a 45 kDa type II integral protein expressed on the surface of a variety of mature cells, including B and T lymphocytes, macrophages, natural killer cells, Langerhans cells, monocytes and platelets (Delespesse *et al, Adv Immunol*, 49 [1991] 149-191). There is also a CD23-like molecule on eosinophils (Grangette *et al*, J Immunol, 143 [ 1989] 3580.3588). CD23 has been implicated in the regulation of the immune response (Delespesse *et al*, Immunol Rev, 125 [1992] 77-97). Human CD23 exists as two differentially regulated isoforms, a and b, which differ only in the amino acids at the intracellular N-terminus (Yokota *et al*, Cell, 55 [1988] 611-618). In man the constitutive a isoform is found only on B-lymphocytes, whereas type b, inducible by IL4, is found on all cells capable of expressing CD23.

Intact, cell bound CD23 (i-CD23) is known to undergo cleavage from the cell surface leading to the formation of a number of well-defined soluble fragments (s-CD23), which are produced as a result of a complex sequence of proteolytic events, the mechanism of which is still poorly understood (Bourget *et al J Biol Chem,* 269 [1994] 6927-6930). Although not yet proven, it is postulated that the major soluble fragments (Mr 37, 33, 29 and 25 kDa) of these proteolytic events, all of which retain the C-terminal lectin domain common to i-CD23, occur sequentially via initial formation of the 37 kDa fragment (Letellier *et al*, *J Exp Med,* 172 [1990] 693-700). An alternative intracellular cleavage pathway leads to a stable 16 kDa fragment differing in the C-terminal domain from i-CD23 (Grenier-Brosette *et al, Eur J Immunol*, 22 [1992] 1573-1577).

Several activities have been ascribed to membrane bound i-CD23 in humans, all of which have been shown to play a role in IgE regulation. Particular activities include: a) antigen presentation, b) IgE mediated eosinophil cytotoxicity, c) B cell homing to germinal centres of lymph nodes and spleen, and d) downregulation of IgE synthesis (Delespesse *et al*, *Adv Immunol*, 49, [1991] 149-191). The three higher molecular weight soluble CD23 fragments (Mr 37, 33 and 29 kDa) have multifunctional cytokine properties which appear to play a major role in IgE production. Thus, the excessive formation of s-CD23 has been implicated in the overproduction of IgE, the hallmark of allergic diseases such as extrinsic asthma, rhinitis, allergic conjunctivitis, eczema, atopic dermatitis and anaphylaxis (Sutton and Gould, *Nature,* 366, [1993] 421-428).

Other biological activities attributed to s-CD23 include the stimulation of B cell growth and the induction of the release of mediators from monocytes. Thus, elevated levels of s-CD23 have been observed in the serum of patients having B-chronic lymphocytic leukaemia (Sarfati *et al*, *Blood,* 71 [1988] 94-98) and in the synovial fluids of patients with rheumatoid arthritis (Chomarat *et al*, *Arthritis and Rheumatism,* 36 [1993] 234-242). That there is a role for CD23 in inflammation is suggested by a number of sources. First, sCD23 has been reported to bind to extracellular receptors which when activated are involved in cell-mediated events of inflammation. Thus, sCD23 is reported to directly activate monocyte TNF, IL-1, and IL-6 release (Armant *et al*, vol 180, J.Exp. Med., 1005-1011 (1994)). CD23 has been reported to interact with the B2-integrin adhesion molecules, CD11b and CD11c on monocyte/macrophage (S. Lecoanet-Henchoz *et al, Immunity*, vol 3; 119-125 (1995)) which trigger NO2⁻, hydrogen peroxide and cytokine (IL-1, IL-6, and TNF) release. Finally, IL-4 or IFN induce the expression of CD23 and its release as sCD23 by human monocytes. Ligation of the membrane bound CD23 receptor with IgE/anti-IgE immune complexes or anti CD23 mAb activates cAMP and IL-6 production and thromboxane B2 formation, demonstrating a receptor-mediated role of CD23 in inflammation.

Because of these various properties of CD23, compounds which inhibit the formation of s-CD23 should have twofold actions of a) enhancing negative feedback inhibition of IgE synthesis by maintaining levels of i-CD23 on the surface of B cells, and b) inhibiting the immunostimulatory cytokine activities of higher molecular weight soluble fragments (Mr 37, 33 and 29 kDa) of s-CD23. In addition, inhibition of CD23 cleavage should mitigate sCD23-induced monocyte activation and mediator formation, thereby reducing the inflammatory response.

WO 99/06361 (Abbott) and WO 00/12478 (Zeneca Limited) describe a range of compounds which includes reverse hydroxamate sulfonyl and sulfonamide compounds, for use as metalloproteinase inhibitors.

WO 99/38843 (Darwin Discovery Limited) discloses a generic scope of compounds useful in the treatment of *inter alia* conditions mediated by enzymes involved in the shedding of CD23, which covers compounds of the formula (A): 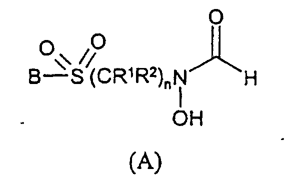 wherein B, R¹ and R² may be one of a range of organic groups.

According to the present invention, there is provided a compound of formula (I): 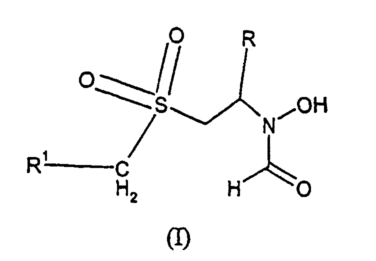 wherein
R is hydrogen, alkyl, alkenyl, alkynyl, aryl, heteroaryl or heterocyclyl; and
R¹ is bicyclyl or heterobicyclyl.

Alkyl, alkenyl and alkynyl groups referred to herein either alone or as part of another group may be straight, branched or cyclic.

Alkyl, alkenyl and alkynyl groups referred to herein in the definition of the R group contain up to eight carbon atoms and are optionally substituted by one or more groups selected from the group consisting of aryl, heterocyclyl, (C1-6)alkylthio, (C2-6)alkenylthio, (C2-6)alkynylthio, aryloxy, arylthio, heterocyclyloxy, heterocyclylthio, (C1-6)alkoxy, aryl(C1-6)alkoxy, aryl(C1-6)alkylthio, amino, mono- or di-(C1-6)alkylamino, acylamino and sulfonylamino in which the amino group may optionally be substituted by (C1-6)alkyl, cycloalkyl, cycloalkenyl, carboxylic acid (C1-6) esters, hydroxy, halogen and carboxamide : CONR²R³ where R² and R³ are independently selected from the group consisting of hydrogen, alkyl, aryl, arylalkyl and heterocyclyl, and includes R² and R³ as part of a heterocyclyl group.

Cycloalkyl and cycloalkenyl groups referred to herein in the definition of the R group include groups having between three and eight ring carbon atoms and are optionally substituted as described hereinabove for alkyl, alkenyl and alkynyl groups.

When used herein in the definition of the R group the term "aryl" includes phenyl. Suitably any aryl group, including phenyl, may be optionally substituted by up to five, preferably up to three substituents. Suitably, any two substituents may optionally together form a fused ring and may optionally be interrupted by up to three heteroatoms in the ring, each of which is selected from oxygen, nitrogen and sulphur. Suitable substituents include halogen, halo(C1-6)alkyl or polyhalo(C1-6)alkyl e.g. CF₃, halo(C1-6)alkyloxy or polyhalo(C1-6)alkyloxy, e.g. OCF₃, CN, (C₁₋₆)alkyl, (C ₁₋₆)alkoxy, hydroxy, amino, mono- and di-N-(C1-6)alkylamino, acylamino (e.g. acetylamino) in which the amino group may optionally be substituted by (C1-6)alkyl, acyloxy, carboxy, (C1-6)alkoxycarbonyl, aminocarbonyl, mono- and di-N-(C1-6)alkylaminocarbonyl, mono- and di-N-(C1-6)alkylaminoalkyl, (C1-6)alkylsulfonylamino in which the amino group may optionally be substituted by (C1-6)alkyl, aryl(C1-6)alkoxycarbonylamino in which the amino group may optionally be substituted by (C1-6)alkyl, (C1-6)alkoxycarbonylamino in which the amino group may optionally be substituted by (C1-6)alkyl, aminosulfonyl, (C1-6)alkylthio, (C1-6)alkylsulfonyl, (C1-6)alkylsulfonyloxy, mono- and di-N-(C1-6)alkylaminosulfonyl, heterocyclyl, heterocyclyl(C1-6)alkyl, aminosulfonyloxy and (C1-6)mono- and dialkylaminosulfonyloxy. The term "aryl" includes single and fused rings, of which at least one is aromatic, which rings may be unsubstituted or substituted by, for example, up to three substituents as set out above. Each ring suitably has from 4 to 7, preferably 6 or 7, ring atoms.

When used herein in the definition of the R group the term "heteroaryl" suitably includes any heterocyclyl group which incorporates at least one aromatic ring (heterocyclic or carbocyclic). Suitable heteroaryl groups include thiophene, such as thiophen-2-yl and thiophen-3-yl; furan, such as furan-2-yl and furan-3-yl; benzothiophene, such as benzothiophen-2-yl; pyrazole, such as pyrazol-3-yl; and isoxazole, such as isoxazol-3-yl.

When used herein in the definition of the R group the terms "heterocyclyl" and "heterocyclic" suitably include, unless otherwise defined, aromatic and non-aromatic, single and fused, rings, one or more rings suitably containing up to four heteroatoms in each ring, each of which is selected from oxygen, nitrogen and sulphur, which rings, may be unsubstituted or substituted by, for example, up to three substituents. Each ring suitably has from 4 to 7, preferably 5 or 6, ring atoms. A fused heterocyclic ring system may include carbocyclic rings and need include only one heterocyclic ring. Suitable heteroaryl groups include benzodioxan, such as 2,3-dihydrobenzo[1,4]dioxin-6-yl; benzodioxepine, such as 3,4-dihydro-2*H*-benzo[b][1,4]dioxepin-7-yl; and benzoxazine, such as 3-oxo-3,4-dihydro-*2H*-benz[1,4]oxazin-6-yl.

Suitable substituents for a heteroaryl or heterocyclyl group include halogen, halo(C1-6)alkyl or polyhala(C1-6)alkyl e.g. CF₃, halo(C1-6)alkyloxy or polyhalo(C1-6)alkyloxy, e.g. OCF₃, (C1-6)alkyl, (C1-6)alkoxy, hydroxy, CN, amino, mono-and di-N-(C1-6)alkylamino, acylamino (e.g. acetylamino) in which the amino group may optionally be substituted by (C1-6)alkyl, acyloxy, carboxy, (C1-6)alkoxycarbonyl, aminocarbonyl, mono- and di-N-(C1-6)alkylaminocarbonyl, (C1-6)alkylsulfonylamino, aminosulfonyl, mono- and di-N-(C1-6)alkylaminosulfonyl, (C1-6)alkylthio and (C1-6)alkylsulfonyl.

When used herein in the definition of the R¹ group "bicyclyl" means fused bicyclic rings suitably containing 4 to 7, preferably 5 or 6 ring atoms in each ring. One ring of the bicyclyl may be saturated or partially saturated. Suitable bicyclyl groups include naphthyl such as 2-naphthyl, tetrahydronaphthyl such as 1,2,3,4-tetrahydronaphthalen-2-yl, and indanyl such as 2-indanyl.

When used herein in the definition of the R¹ group, heterobicyclyl means fused bicyclic aromatic and non-aromatic rings containing up to 4 heteroatoms in each ring, each of which is selected from oxygen, nitrogen and sulphur. Each ring suitably has from 4 to 7, preferably 5 or 6, ring atoms. The fused bicyclic ring system may include one carbocyclic ring and one of the rings may be saturated or partially saturated. Suitable heterobicyclyl groups include benzothiophene, such as benzothiophen-5-yl and benzothiophen-6-yl; benzofuran such as benzofuran-2-yl, benzofuran-5-yl and benzofuran-6-yl; quinoline such as quinolin-3-yl; thienopyridine such as thieno[2,3-b]pyridin-5-yl and thieno[3,2-b]pyridin-6-yl; isoquinoline such as isoquinolin-3-yl; quinoxaline such as quinoxalin-2-yl; and benzothiazole such as benzothiazol-6-yl.

Aromatic rings in bicyclyl and heterobicyclyl ring systems may be optionally substituted with up to three substituents. Suitable substituents include fluorine. Examples of substituted heterobicyclyl groups include 2-fluorobenzothiophen-5-yl and 3-fluorobenzothiophen-5-yl.

Preferably, R is selected from phenyl optionally substituted by up to three groups selected independently from halogen, (C1-6)alkoxy, di-N-(C1-6)alkylaminosulfonyl, (C1-6)acylamino, aryl(C1-6)alkoxycarbonylamino, amino, (C1-6)alkoxycarbonyl, carboxy, hydroxy and (C1-6)alkyl or two groups which together form a fused ring; benzothiophene, thiophene optionally substituted by (C1-6)alkyl, carboxy or (C1-6)alkoxycarbonyl; furanyl; pyrazole optionally substituted by up to two (C1-6)alkyl groups; (C1-6)alkyl optionally substituted by (C1-6)alkoxy, aryl(C1-6)alkoxy or phenyl; isoxazole optionally substituted by (C1-6)alkyl; benzodioxan; benzodioxepine; and benzoxazine.

Preferably R¹ is selected from benzothiophene optionally substituted by fluorine; indanyl; benzofuranyl; quinolyl; naphthyl; benzothiazole; thienopyridyl; isoquinolyl; and quinoxalyl.

More preferably, R is selected from phenyl optionally substituted by one or two groups independently selected from chlorine, fluorine, -OCH₃, -SO₂N(CH₃)₂, -NHCOCH₃, NHCO₂CH₂Ph, amino, ethoxycarbonyl, methoxycarbonyl, carboxy, hydroxy and methyl; benzothiophen-2-yl; thiophen-2-yl optionally substituted by methyl, carboxy or methoxycarbonyl; pyrazol-3-yl optionally substituted by methyl and/or t-butyl; furan-2-yl; furan-3-yl; methyl, ethyl, *n*-propyl, isopropyl, isobutyl or neopentyl each optionally substituted by methoxy, isopropoxy, benzyloxy or phenyl; isoxazol-3-yl optionally substituted by methyl; 3-oxo-3,4-dihydro-*2H*-benz[1,4]oxazin-6-yl; 3,4-dihydro-2*H*-benzo[b][1,4]dioxepin-7-yl; and 2,3-dihydrobenzo[1,4]dioxin-6-yl

More preferably, R¹ is selected from benzothiophene-5-yl optionally substituted by F; indan-2-yl; benzofuran-2-yl; benzofuran-5-yl; benzofuran-6-yl; quinolin-3-yl; 2-naphthyl; benzothiazol-6-yl; thieno[2,3-b]pyridin-5-yl; thieno[3,2-b]pyridin-6-yl; isoquinolin-3-yl; and quinoxalin-2-yl.

Even more preferably, R is phenyl; phenyl substituted by one or two groups independently selected from chlorine, fluorine, -OCH₃, -SO₂N(CH₃)₂, -NHCOCH₃, NHCO₂CH₂Ph, amino, ethoxycarbonyl, methoxycarbonyl, carboxy, hydroxy and methyl; thiophen-2-yl optionally substituted by methyl; 3-oxo-3,4-dihydro-*2H*-benz[1,4]oxazin-6-yl; 3,4-dihydro-2*H*-benzo[b][1,4]dioxepin-7-yl; or 2,3-dihydrobenzo[1,4]dioxin-6-yl; methyl optionally substituted by methoxy or isopropoxy; ethyl; *n*-propyl; isopropyl; or isobutyl.

Even more preferably, R¹ is benzothiophen-5-yl; quinolin-3-yl; thieno[2,3-b]pyridin-5-yl; or thieno[3,2-b]pyridin-6-yl.

Yet more preferably, R and/or R¹ are selected from the group consisting of the values ascribed to it in the Examples hereinbelow.

Still more preferably, the compound of formula (I) of the invention is selected from the group consisting of the compounds described in the Examples hereinbelow.

According to a second aspect, the present invention provides the use of a compound of formula (I) for the production of a medicament for the treatment or prophylaxis of disorders such as allergy, allergic asthma, atopic dermatitis and other atopic diseases; inflammatory disorders; and autoimmune disease, in which the overproduction of s-CD23 is implicated.

In a third aspect the invention provides a method for the treatment or prophylaxis of disorders such as allergy, allergic asthma, atopic dermatitis and other atopic diseases; inflammatory disorders; and autoimmune disease, in which the overproduction of s-CD23 is implicated, which method comprises the administration of a compound of formula (I), to a human or non-human mammal in need thereof.

The invention also provides a pharmaceutical composition for the treatment or prophylaxis of disorders such as allergy, allergic asthma, atopic dermatitis and other atopic diseases; inflammatory disorders; and autoimmune disease, in which the overproduction of s-CD23 is implicated which comprises a compound of formula (I) and optionally a pharmaceutically acceptable carrier therefor.

Particular inflammatory disorders include CNS disorders such as Alzheimer's disease, multiple sclerosis, and multi-infarct dementia, as well as the inflammation mediated sequel of stroke and head trauma.

The present inventors have surprisingly found that the compounds of the invention are potent and selective inhibitors of CD23 processing, whilst having little or no activity as inhibitors of collagenase.

It is to be understood that the pharmaceutically acceptable salts, solvates and other pharmaceutically acceptable derivatives of the compound of formula (I) are also included in the present invention.

Salts of compounds of formula (I) include for example acid addition salts derived from inorganic or organic acids, such as hydrochlorides, hydrobromides, hydroiodides, p-toluenesulphonates, phosphates, sulphates, acetates, trifluoroacetates, propionates, citrates, maleates, fumarates, malonates, succinates, lactates, oxalates, tartrates and benzoates.

Salts may also be formed with bases. Such salts include salts derived from inorganic or organic bases, for example alkali metal salts such as sodium or potassium salts, and organic amine salts such as morpholine, piperidine, dimethylamine or diethylamine salts.

The compounds of the invention may be prepared by use of any appropriate conventional method.

Accordingly, a further aspect of the invention provides a process for preparing a compound of formula (I) as defined hereinabove, which process comprises:
(a) deprotecting a compound of formula (II): 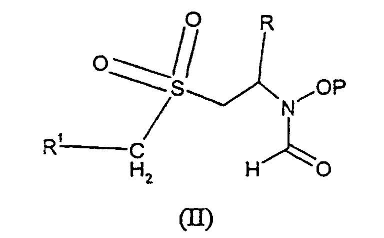 wherein R and R¹ are as defined hereinabove, and P is a protecting group such as allyl, allyloxycarbonyl, benzyl, benzyloxycarbonyl, tetrahydropyranyl, p-methoxybenzyl, acyl such as acetyl, or benzoyl; or
(b) converting a compound of formula (I) to a different compound of formula (I) as defined hereinabove, or
(c) formylating a compound of formula (III) to give a compound of formula (I) as defined hereinabove, 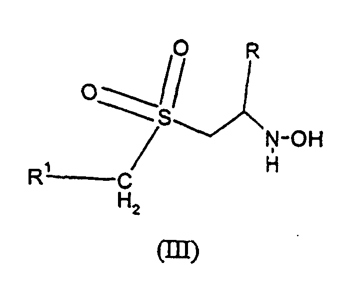 or
(d) oxidising a compound of formula (X) to give a compound of formula (I) as defined hereinabove

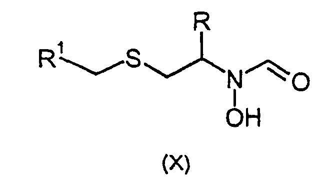

Compounds of formula (II) and (III) are novel and form a further aspect of the invention.
The following reaction schemes illustrate the procedures that may be used to prepare compounds of formula (I).

### Reaction Schemes

One procedure for preparing compounds of formula (I) is shown in Scheme 1. The thiol (VIII) may be prepared from the corresponding halide such as the bromide (IX) using the methods described by Choi and Yoon, Synthesis, 1995, 373, and converted into (VII) by reaction with a suitable halomethyl ketone such as the bromomethyl ketone in the presence of a base such as triethylamine. The ketone (VII) can be reacted with a suitably O-protected hydroxylamine. For example, when the protecting group (P) is benzyl, reaction with O-benzyl hydroxylamine under standard conditions can be used to prepare oxime (VI) which can be reduced to (V) with a suitable reducing agent eg sodium borohydride or sodium cyanoborohydride in acetic acid. Formylation of (V) using formic acetic anhydride followed by oxidation with meta chloroperbenzoic acid affords (II) which can be deprotected under conditions which depend on the nature of R¹. For example when R¹ is 5-benzo[b]thiophene, and the protecting group is benzyl, the deprotection can be effected using boron trichloride dimethyl sulfide in the presence of anisole.

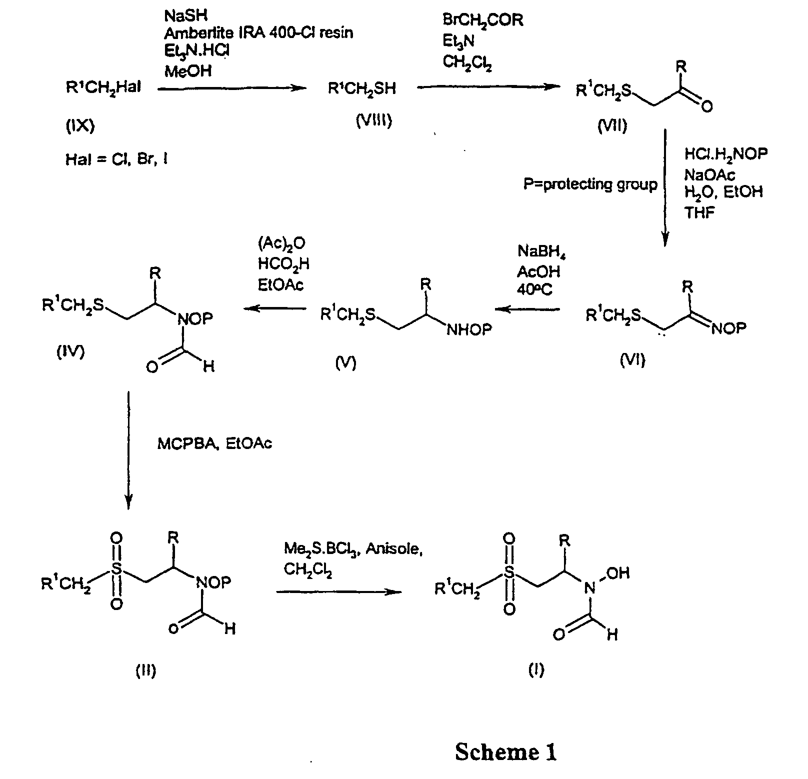

Compounds of formula (I) may also be prepared as described in Scheme 2. The ketone (VII) may be reacted with hydroxylamine under standard conditions to give an oxime (XII )which upon reduction with a suitable reducing agent such as sodium cyanoborohydride in acetic acid yields the hydroxylamine (XI) which can be formylated by treatment with formic acetic anhydride followed by potassium carbonate and methanol, and oxidised as described previously for Scheme 1.

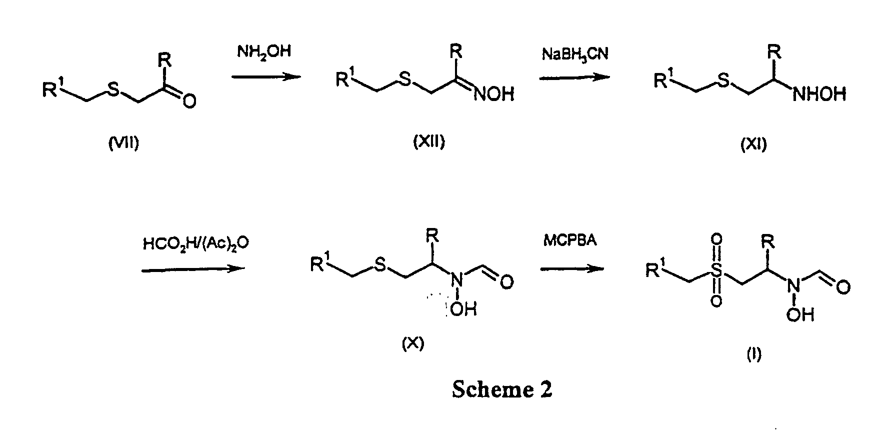

Procedures for preparing compounds of formula (I), where R = aryl or heteroaryl, in chirally pure form are shown in Scheme 3. The thioacetate (XVI) can be prepared from the corresponding halide (IX), converted in situ to the thiol and reacted with a bromomethylketone to give (VII). Alternatively, the procedures in Scheme 1 may be employed to obtain (VII). Chiral reduction of the ketone (VII) with (S)-CBS/BH₃ affords the alcohol (XVA) which is reacted with a suitably protected and activated hydroxylamine derivative such as bis-tert-butoxycarbonyl hydroxylamine or *bis*-benzyloxycarbonyl hydroxylamine, to give (XIVA). Oxidation to the sulfone (XIIIA) is carried out with a suitable oxidising agent such as MCPBA, followed by deprotection and formylation. Protecting groups (P) are selected in order to ensure compatability between deprotection conditions and any other chemically labile functional groups in the molecule. Deprotection of a bis-tert-butoxycarbonyl protected hydroxylamine may be effected with trifluoroacetic acid. Compounds of formula (I) containing acid sensitive groups may be prepared using bis-benzyloxycarbonyl protection which can be removed using hydrogenolysis and/or reaction with trimethylsilyl iodide.

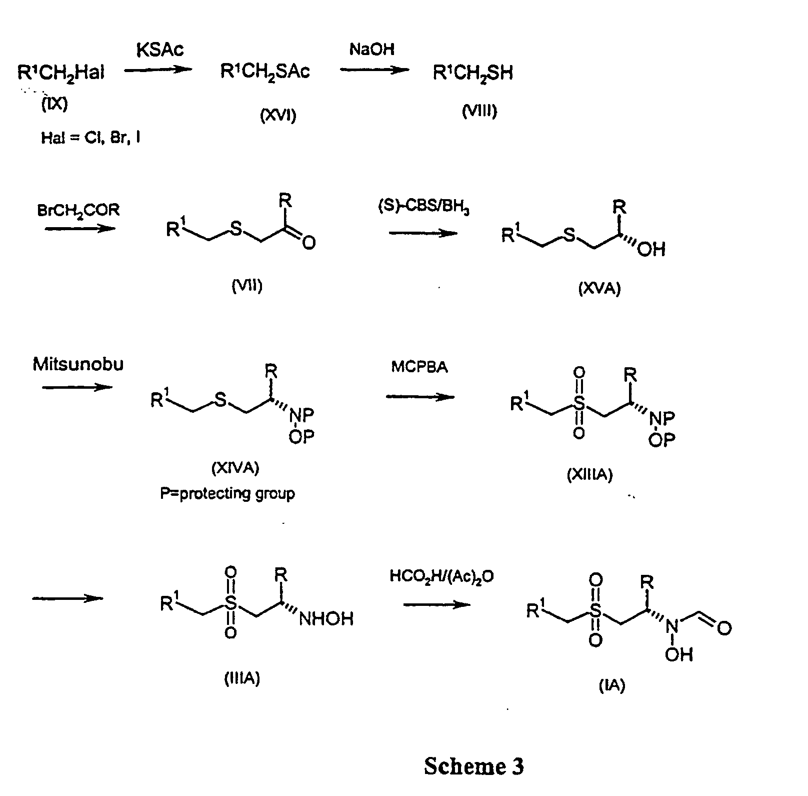

Alternative methods for preparing compounds of formula (IA) where R = aryl or heteroaryl are shown in Scheme 4. These routes allow the introduction of the chiral centre at an earlier stage in the synthesis. Route A, which relies on the ring opening of an (S)-aryl epoxide (XVIIIA), results in an approximately 1:1 mixture of alcohols (XVIIA) and (XVA). Route B affords exclusively alcohol (XVIIA). Mitsunobu reaction of either (XVIIA), (XVA) or a mixture of (XVIIA) and (XVA) with a suitably protected hydroxylamine derivative such as bis Boc hydroxylamine affords a single product (XIVA) which can be progressed to the final product using the methods described for Scheme 3. Route A is preferred for less stable R¹CH₂Hal intermediates. Where R=Ph preparation of the thiol (XIXA) from (S)-ethyl mandelate is described by Aversa et al, J. Org. Chem., 1997, 62 (13), 4376. Chiral epoxides (XVIIIA) are either commercially available, as in the case of R=Ph, or can be prepared via established asymmetric routes.

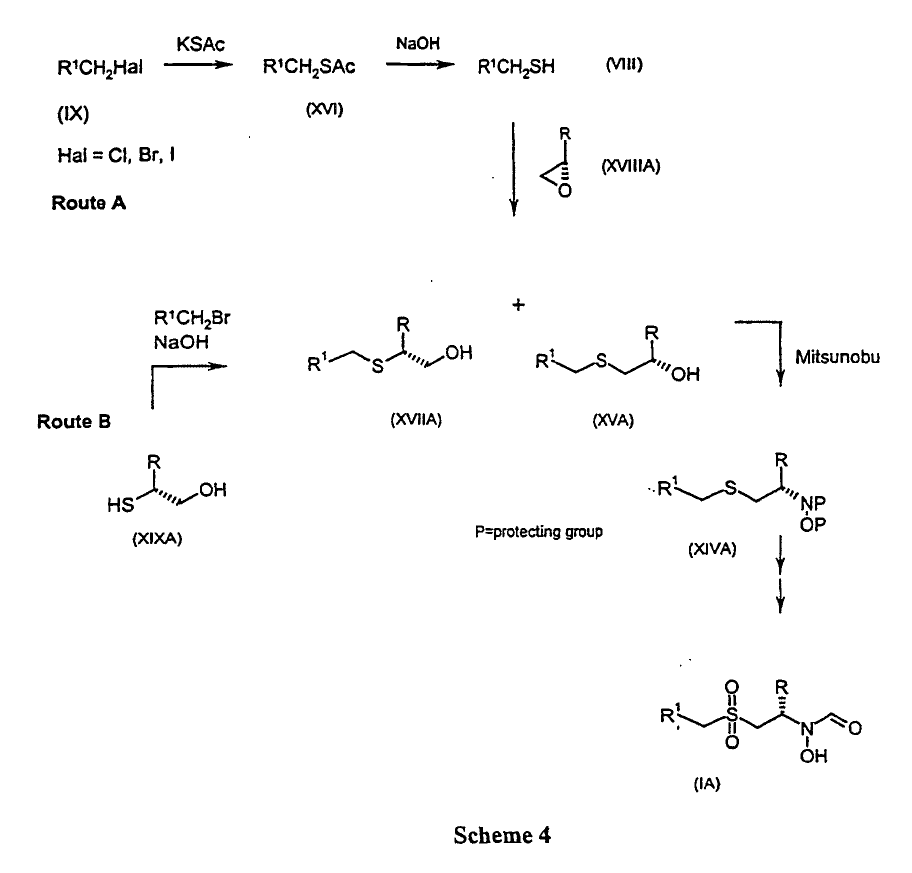

Compounds of formula (IA) can be prepared in chirally pure form using the procedures described in Scheme 5. A suitably protected amino alcohol (XXVIA) can be converted into the corresoponding thioacetate (XXVA) under Mitsunobu conditions eg using triphenylphosphine or tributylphosphine in combination with di-t-butylazodicarboxylate or diethylazodicarboxylate. The thioacetate (XXVA) can be converted into (XXIVA) using previously desribed methods (Scheme 3). Alternatively, the alcohol group in (XXVIA) may be converted into a leaving group such as a tosylate or bromide and reacted with (XVI) using previously described conditions to give (XXIVA). Oxidation of (XXIVA) to the sulfone (XXIIIA) can be carried out as previously described. Deprotection under standard conditions such as trifluoroacetic acid or hydrogen chloride in dioxan affords (XXIIA) which can be converted into (XXIA) and oxidised to the oxaziridine (XXA) using established procedures eg meta chloroperbenzoic acid.

Conversion of the oxaziridine (XXA) into (IIIA) is preferably carried out using hydrochloric acid. Formylation of (IIIA) can be carried out as previously described.

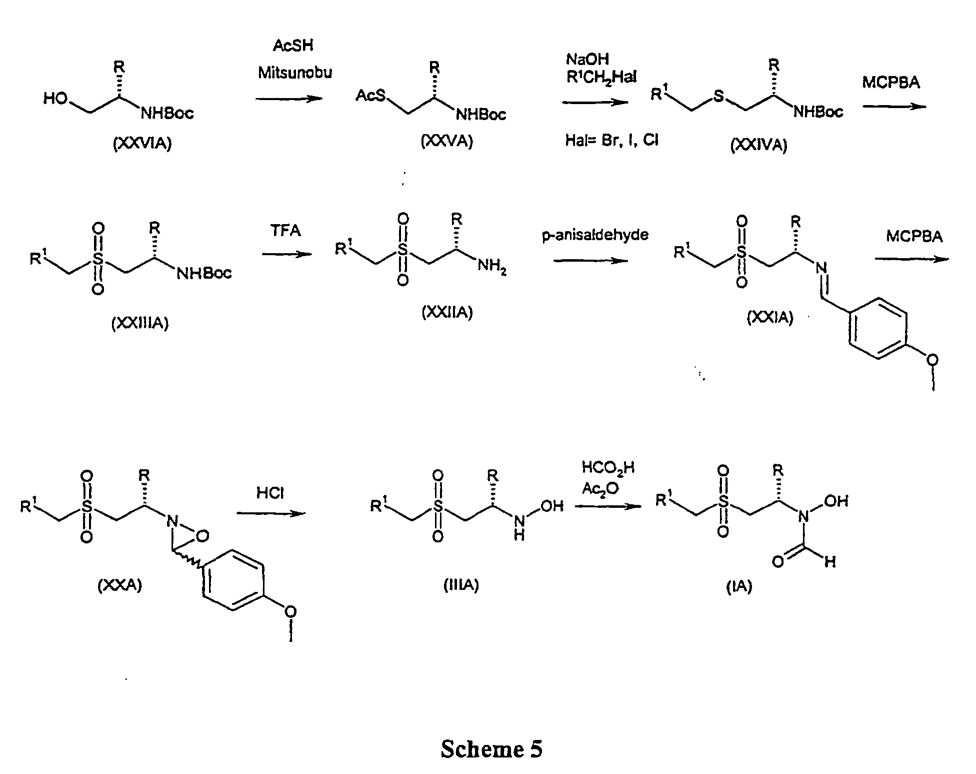

An alternative procedure for preparing compounds of formula (IIIA) in chirally pure form is shown in Scheme 6. The amine (XXIIA) can be alkylated with cyanomethyl bromide or cyanomethyl iodide to give the cyanomethylamine (XXVIIA) which is converted into (IIIA) using the methods described by Tokuyama et al., Synthesis, 2000, 9, 1299.

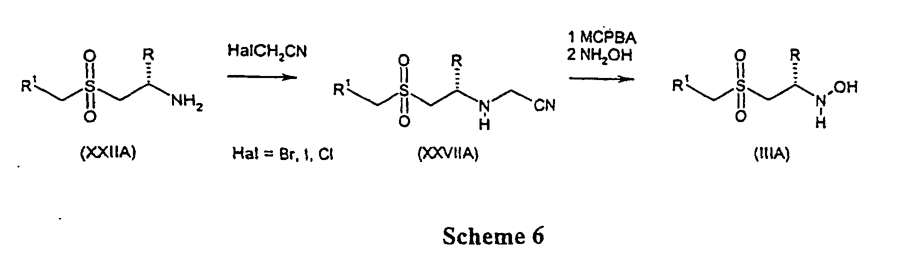

Alternatively, the amine (XXIIA) may be oxidised directly with benzoyl peroxide to give a benzoyl hydroxylamine (XXXA) as shown in Scheme 7, using the method described by Phanstiel, J. Org. Chem., 1997, 62,8104. The latter compound can be formylated with formic acetic anhydride and then deprotected, for example with ammonia in methanol, to give (IA).

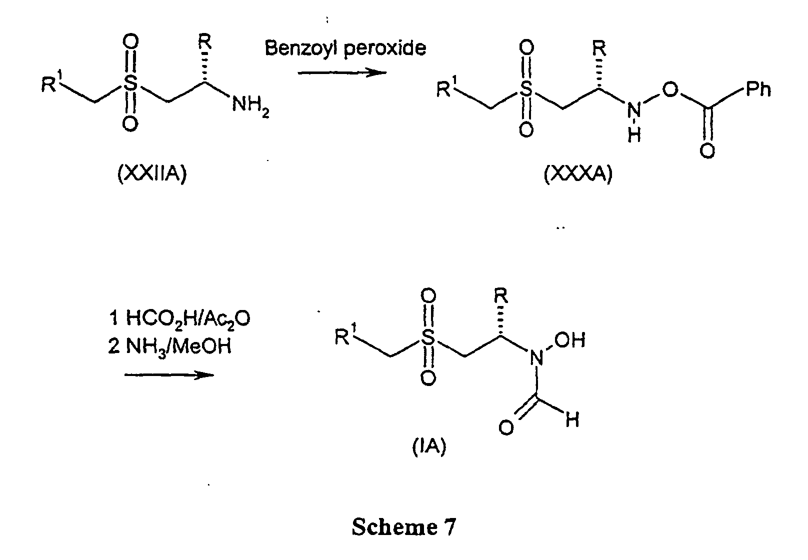

Compounds of formula (III) where R = alkyl can also be prepared using the route shown in Scheme 8. The alcohol (XV) can be obtained by reduction of the ketone (VII) under standard conditions, for example borane in THF. Where appropriate, the alcohol (XV) may be prepared by reaction of the halo alcohol (XXXI) with (XVI). Oxidation to the sulfone (XXIX) can be carried out as previously described followed by elimination using methanesulfonyl chloride and triethylamine, or Mitsunobu conditions to give (XXVIII). Addition of hydroxylamine to the unsaturated sulfone (XXVIII) affords (III).

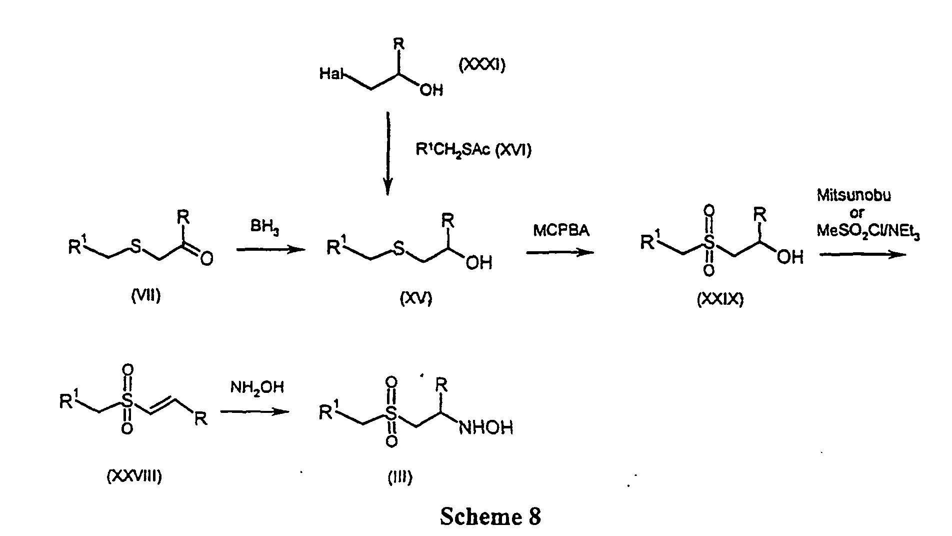

Compounds of formula (IA) where R = aryl or heteroaryl may also be prepared from a chiral halo alcohol (XXXIA) as shown in Scheme 9. For example, bromo alcohols of formula (XXXIA) (Hal = Br) may be obtained from the corresponding ketone by chiral reduction with (S)-CBS/BH₃ (Corey, Halal, Angew. Chem. Int. Ed., 1998, 37,1986). Subsequent reaction with (XVI) in the presence of sodium methoxide or sodium hydroxide affords a mixture of alcohols (XVIIA) and (XVA) which can be converted into (IA) as shown in Scheme 4.

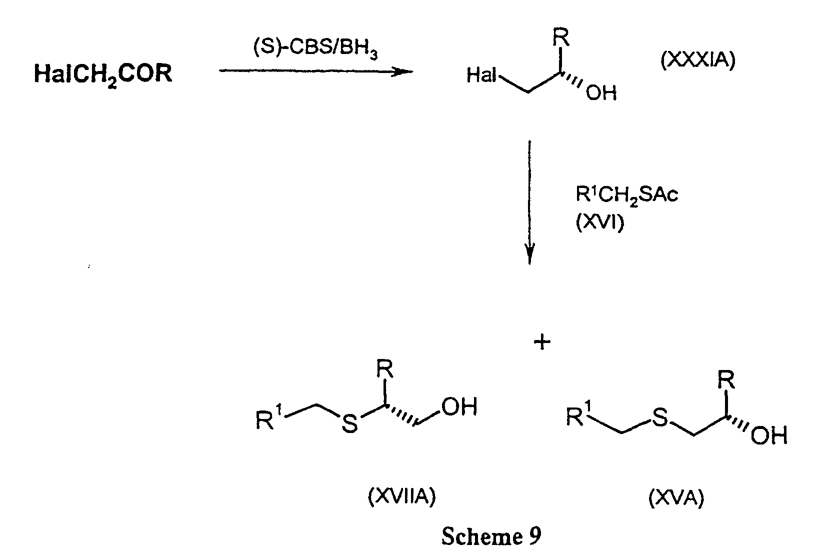

Precursors are either commercially available or may be prepared via standard methods. Halomethyl ketones can be obtained by bromination of a methyl ketone using bromine in methanol as described by Gaudry and Marquet, Org. Synth., 1976, 55, 24. Alternatively, bromomethylketones can be obtained from the corresponding diazoketones by reaction with hydrogen bromide using standard methods. Compounds of formula (IX) can be obtained by standard methods, for example by bromination of a compound of formula R¹CH₃ with N-bromosuccinimide in carbon tetrachloride. Bromination of quinoline containing precursors with N-bromosuccinimide is preferably carried out in the presence of an acid such as acetic acid. Alternatively a compound R¹CH₂OH may be converted into a compound of formula (IX) using standard halogenation procedures e.g. using phosphorous pentachloride or thionyl chloride, or by conversion into the mesylate followed by treatment with lithium bromide in acetone.

Protected amino alcohol precursors (XXVIA) are either commercially available or may be prepared via standard routes e.g. from the corresponding amino acids as described by Ho *et al*, Tet. Lett., 1993, 34(41), 6513.

Suitable amino acid derivatives may be prepared from aziridine precursors e.g. as described by Nakajima *et al*., Bull. Soc. Chim. Japan, 1982, 55, 3049.

The isomers, including stereoisomers, of the compounds of the present invention may be prepared as mixtures of such isomers or as individual isomers. The individual isomers may be prepared by any appropriate method, for example individual stereoisomers may be prepared by stereospecific chemical synthesis starting from chiral substrates or by separating mixtures of enantiomers or mixtures of diastereomers using known methods such as chiral preparative HPLC. For example, separation of compounds of formula (I) which are racemic into single enantiomers can be achieved by conversion into a suitable ester derivative such as the O-methyl mandelic acid derivative followed by separation using standard chromatographic procedures and then deprotection.

In a preferred aspect, the invention provides compounds of formula (IA):

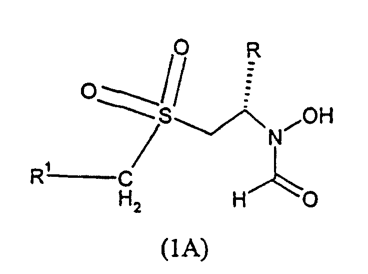

It is preferred that the compounds are isolated in substantially pure form.

As stated herein an inhibitor of the formation of soluble human CD23 has useful medical properties. Preferably the active compounds are administered as pharmaceutically acceptable compositions.

The compositions are preferably adapted for oral administration. However, they may be adapted for other modes of administration, for example in the form of a spray, aerosol or other conventional method for inhalation, for treating respiratory tract disorders; or parenteral administration for patients suffering from heart failure. Other alternative modes of administration include sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring-agents.

For parenteral administration, fluid unit dosage forms are prepared utilising the compound and a sterile vehicle, and, depending on the concentration used, can be either suspended or dissolved in the vehicle. In preparing solutions the compound can be dissolved in water for injection and filter sterilised before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns for example diameters in the range of 1-50 microns, 1-10 microns or 1-5 microns. Where appropriate, small amounts of other anti-asthmatics and bronchodilators, for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included.

The compositions may contain from 0.1 % to 99% by weight, preferably from 10-60% by weight, of the active material, depending upon the method of administration. A preferred range for inhaled administration is 10-99%, especially 60-99%, for example 90, 95 or 99%.

Microfine powder formulations may suitably be administered in an aerosol as a metered dose or by means of a suitable breath-activated device.

Suitable metered dose aerosol formulations comprise conventional propellants, cosolvents, such as ethanol, surfactants such as oleyl alcohol, lubricants such as oleyl alcohol, desiccants such as calcium sulphate and density modifiers such as sodium chloride.

Suitable solutions for a nebulizer are isotonic sterilised solutions, optionally buffered, at for example between pH 4-7, containing up to 20mg/ml of compound but more generally 0.1 to 10mg/ml, for use with standard nebulisation equipment.

An effective amount will depend on the relative efficacy of the compounds of the present invention, the severity of the disorder being treated and the weight of the sufferer. Suitably, a unit dose form of a composition of the invention may contain from 0.1 to 1000mg of a compound of the invention (0.001 to 10mg via inhalation) and more usually from 1 to 500mg, for example 1 to 25 or 5 to 500mg. Such compositions may be administered from 1 to 6 times a day, more usually from 2 to 4 times a day, in a manner such that the daily dose is from 1mg to 1g for a 70 kg human adult and more particularly from 5 to 500mg. That is in the range of about 1.4 x 10m2 mg/kg/day to 14 mg/kg/day and more particularly in the range of about 7 x 10-2 mg/kg/day to 7 mg/kg/day.

The following examples illustrate the invention but do not limit it in any way.

### BIOLOGICAL TEST METHODS

**Procedure 1:** The ability of test compounds to inhibit the release of soluble CD23 was investigated by use of the following procedure.

### RPMI 8866 Cell membrane CD23 cleavage activity assay:

Plasma membranes from RPMI 8866 cells, a human Epstein-Barr virus transformed B-cell line (Sarfati et al., Immunology 60 [1987] 539-547) expressing high levels of CD23 are purified using an aqueous extraction method. Cells resuspended in homogenization buffer (20mM HEPES pH 7.4, 150 mM NaCl, 1.5 mM MgCl2, 1 mM DTT) are broken by N₂ cavitation in a Parr bomb and the plasma membrane fraction mixed with other membranes is recovered by centrifugation at 10,000Xg. The light pellet is resuspended in 0.2 M potassium phosphate, pH 7.2 using 2 ml per 1-3 g wet cells and the nuclear pellet is discarded. The membranes are further fractionated by partitioning between Dextran 500 (6.4% w/w) and polyethylene glycol (PEG) 5000 (6.4% w/w) (ref), at 0.25 M sucrose in a total of 16 g per 10-15 mg membrane proteins [Morre and Morre, BioTechniques 7, 946-957 (1989)]. The phases are separated by brief centrifugation at 1000Xg and the PEG (upper) phase is collected, diluted 3-5 fold with 20 mM potassium phosphate buffer pH 7.4, and centrifuged at 100,000Xg to recover membranes in that phase. The pellet is resuspended in phosphate-buffered saline and consists of 3-4 fold enriched plasma membranes as well as some other cell membranes (e.g. lysosomes, Golgi). The membranes are aliquoted and stored at -80°C. Fractionation at 6.6 % Dextran/PEG yields plasma membranes enriched 10-fold.

The fractionated membranes are incubated at 37°C for times up to 4 hrs to produce fragments of CD23 which are separated from the membrane by filtration in 0.2 micron Durapore filter plates (Millipore) after quenching the assay with a non-selecitve MMP inhibitor, e.g. 5 uM Preparation 1 from WO 95/31457 ([4-(N-Hydroxyamino)-2-(R)-isobutyl-3-(S)-(2-thiophenethiomethyl)succinyl]-(S)-phenylalanine-N-methylamide sodium salt, prepared according to the procedure described in Example 11 of WO 90/05719). sCD23 released from the membrane is determined using the EIA kit from The Binding Site (Birmingham, UK) or a similar one utilising MHM6 anti-CD23 mAb [Rowe et al., Int. J. Cancer, 29, 373-382 (1982)] or another anti-CD23 mAb as the capture antibody in a sandwich EIA.. The amount of soluble CD23 made by 0.5 ug membrane protein in a total volume of 50 ul phosphate-buffered saline is measured by EIA and compared to the amount made in the presence of various concentrations of inhibitors. Inhibitors are prepared in solutions of water or dimethylsulfoxide (DMSO) and the final DMSO concentration is not more than 2 %. IC50's are determined by curve fitting as the concentration where 50 % inhibition of production of sCD23 is observed relative to the difference in sCD23 between controls incubated without inhibitor.

### Results

The compounds of Examples 1-69 were tested and all showed IC₅₀ values of ≤ 1 uM.

**Procedure 2:** The ability of test compounds to inhibit collagenase was investigated using the following procedure.

### Collagenase inhibition assay:

The potency of compounds to act as inhibitors of collagenase was determined by the method of Cawston and Barrett (Anal. Biochem. **99**, 340-345, 1979), hereby incorporated by reference, whereby a 1 mM solution of the inhibitor being tested or dilutions thereof, was incubated at 37 °C for 18 h with collagen and human recombinant collagenase, from synovial fibroblasts cloned, expressed and purified from E. Coli, (buffered with 150 mM Tris, pH 7.6, containing 15 mM calcium chloride, 0.05% Brij 35, 200 mM sodium chloride and 0.02% sodium azide). The collagen was acetylated ³H type 1 bovine collagen prepared by the method of Cawston and Murphy (methods in Enzymology 80, 711,1981) The samples were centrifuged to sediment undigested collagen and an aliquot of the radioactive supernatant removed for assay on a scintillation counter as a measure of hydrolysis. The collagenase activity in the presence of 1mM inhibitor, or dilution thereof, was compared to activity in a control devoid of inhibitor and the results reported as that concentration effecting 50% of the collagenase (IC₅₀).

### Results

The compounds of Examples 1-5, 14,15, 16, 22, 23, 24, 26, 35, 42, 48, 49, 50, 54 and 58 were tested and all showed IC₅₀ values of ≥10uM.

### Preparation of Intermediates

### Preparation 1: Thioacetic acid S-benzo[b]thiophen-5-yl-methyl ester

**Step 1: 5-Bromomethylbenzo*[b]*thiophene -** 5-Methylbenzo*[b]*thiophene (37g) in carbon tetrachloride (400ml) with N-bromosuccinimide (46g) and azo-isobutyronitrile (0.3g) was brought to reflux. After 3h at reflux the reaction was cooled, filtered, evaporated and crystallised from hexane to give the subtitle compound as a solid, (56g).
**Step 2 : Thioacetic acid *S*-benzo*[b]*thiophen-5-yl-methyl ester** - To 5-bromomethylbenzo*[b]*thiophene (56g) in acetone (600ml) was added finely crushed potassium thioacetate (34g). The reaction was stirred and sonicated for 20 min and then left stirring for 4h. Iced water and EtOAc were then added and the reaction washed with brine/sodium bicarbonate solution and water (3x). The EtOAc layer was dried (MgSO₄), evaporated and chromatographed (silica gel, step gradient 0-10% EtOAc / hexane) to give the title compound as a crystalline solid (38g).

### Preparation 2: 2-Acetylthiomethylindane

**Step 1: 2-Bromomethylindane -** A solution of 2-hydroxymethylindane (1.0g) (J. Kenner, *J. Chem. Soc.,* 1914, 2685) in MDC (25ml) at 0°C was treated with triethylamine (1.0ml) and methanesulfonyl chloride (0.6ml). After 30min the solution was washed with dilute hydrochloric acid, aqueous sodium hydrogen carbonate, and brine, dried (MgSO₄), and evaporated. The resulting crude mesylate was dissolved in acetone (25ml) and lithium bromide (1.8g) added. After refluxing overnight the mixture was cooled and filtered, and the filtrate evaporated then partitioned between water and hexane. The hexane layer was filtered through silica gel and evaporated to provide the subtitle compound (0.49g).
**Step 2: 2-Acetylthiomethylindane** - A mixture of 2-bromomethylindane (1.1g), potassium thioacetate (0.7g), and acetone (5ml) was stirred at rt for 4h then partitioned between water and MDC. The organic layer was dried (MgSO₄) and evaporated to give the title compound (1.0g).
In like manner was prepared 3-acetylthiomethylquinoline from 3-chloromethylquinoline hydrochloride (Z.-Z. Ma et al, *Heterocycles,* 1999, *51(8)*, 1883) - see Preparation 6.

**Preparation 3: 5-Bromomethylbenzo*[b]*furan -** Phosphorus oxybromide (2.0g) was added to a solution of 5-hydroxymethylbenzo*[b]*furan (0.5g) (K. Hiroya et al, *Heterocycles*, 1994, *38(11)*, 2463) in ether (50ml) at reflux. After 3h at reflux the solution was washed with water, aqueous saturated sodium hydrogen carbonate, and brine, dried (MgSO₄) and evaporated to give the title compound (0.7g).

### Preparation 4: 2-Fluoro-5-bromomethylbenzo[b]thiophene

**Step 1: 2-Fluoro-5-methylbenzo*[b]*thiophene -** A solution of 5-methylbenzo*[b]*thiophene (4.0g) in diethyl ether (50ml) at -10°C was treated with n-butyllithium (19ml, 1.6M in hexane). After 1h at -10°C N-fluorobenzenesulfonimide (10.4g) in THF (20ml) was added. After 1h at rt the mixture was partitioned between aqueous saturated ammonium chloride and hexane, and the organic layer was dried (MgSO₄) and chromatographed (silica gel, hexane) to give the subtitle compound (1.9g).
**Step 2: 2-Fluoro-5-bromomethylbenzo*[b]*thiophene -** A solution containing 2-fluoro-5-methylbenzo*[b]*thiophene (0.60g), N-bromosuccinimide (0.63g) in carbon tetrachloride (20ml) was refluxed for 4h, cooled, and filtered. The filtrate was evaporated to give the title compound as an oil (0.36g).

### Preparation 5: 3-Fluoro-5-bromomethylbenzo[b]thiophene

**Step 1: 5-Methylbenzo*[b]*thiophene-2-carboxylic acid -** A solution of 5-methylbenzo*[b]*thiophene (6.1g) in diethyl ether (50ml) at -10°C was treated with n-butyllithium (25ml, 1.6M in hexane). After 1h at -10°C the solution was poured onto solid carbon dioxide then left to evaporate. Water and diethyl ether were then added. The aqueous layer was acidified with dilute HCl and extracted with more diethyl ether to give the subtitle compound (5.4g).
**Step 2: 3-Fluoro-5-methylbenzo*[b]*thiophene-2-carboxylic acid -** A solution of 5-methylbenzo*[b]*thiophene-2-carboxylic acid (1.0g) in THF (15ml) at -70°C was treated with n-butyllithium (7.0ml, 1.6M in hexane). After 1h at -70°C N-fluorobenzenesulfonimide (2.4g) in THF (5ml) was added. After 1h without cooling the mixture was partitioned between dilute hydrochloric acid and diethyl ether. The organic layer was dried (MgSO₄) and evaporated and the residue crystallised from MDC to give the subtitle compound (0.75g).
**Step 3: 3-Fluoro-5-methylbenzo*[b]*thiophene -** A mixture of 5-methyl-3-fluorobenzo*[b]*thiophene-2-carboxylic acid (0.75g), copper powder (0.50g), and quinoline (5ml) was heated at 180°C for 30min then cooled and partitioned between dilute HCl and hexane. The organic layer was dried (MgSO₄) and evaporated and chromatographed (silica gel, hexane) to give the subtitle compound (1.9g).
**Step 4: 3-Fluoro-5-bromomethylbenzo*[b]*thiophene -** Prepared by the method of Preparation 4, Step 2.

### Preparation 6: 3-Acetylthiomethylquinoline

### Method A

**Step 1: 3-Quinolylmethanol -** Quinoline-3-carboxaldehyde (13.18g) in ethanol (260ml) was cooled to 0°C followed by the addition of sodium borohydride (1.62g) portionwise. The temperature was maintained at 0°C for 15min followed by the addition of 6N HCl (28ml) during which time the temperature of the reaction was maintained between 0-5°C. The solution was then neutralised with 1M NaOH. The crude reaction mixture was stripped to dryness to remove ethanol and the residue was partitioned between water and EtOAc. The EtOAc layer was then dried (MgSO₄) and absorbed onto silica gel and chromatographed (flash silica gel, step gradient: 0-100% EtOAc/hexane) to give the subtitle compound as a white solid (9.85g).
**Step 2 : 3-Chloromethylquinoline hydrochloride -** 3-Quinolylmethanol (9.85g) was taken up in dry benzene (200ml) and stirred followed by the addition of thionyl chloride (14.69ml). An immediate yellow precipitate was obtained. Stirring was maintained at rt for 2 h. A light yellow solid was filtered off and dried to give the subtitle compound (13g).
**Step 3 : 3-Acetylthiomethylquinoline -** 3-Chloromethylquinoline hydrochloride (5.2g) was taken up in acetone (100ml) followed by the addition of potassium thioacetate (1.8g) and allowed to stir at rt overnight. The reaction mixture was absorbed onto silica gel and chromatographed (silica gel, step gradient 0-50% ether/petroleum ether) to give the title compound as an orange solid (4.2g). ¹H NMR δ(DMSO-d6): 8.85 (1H, d, J=2Hz), 8.25(1H, d, J=2Hz), 8.01(1H, d, J=8.4Hz), 7.95 (1H, d, J=8.4Hz), 7.74 (1H, t, J=8.4Hz), 7.61 (1H, t, J=8.4Hz), 4.33 (2H, s), 2.38 (3H, s).

### Method B

3-Methylquinoline (5g) in CCl₄ (50ml) was treated with glacial acetic acid (1.85ml), NBS (8.5g) and AIBN (1.5g). The reaction was brought to reflux using a 100W halogen light and refluxed for 10min. After cooling, EtOAc (60ml) was added and the reaction was filtered through a plug of silica, concentrated to half volume and added to potassium thioacetate (10g) dissolved in DMF (150ml) with potassium carbonate (2g). The reaction was then further concentrated to 150ml by evaporation. After 2h the reaction was diluted with EtOAc (300ml) and washed with saturated sodium hydrogen carbonate solution and saturated brine (8x). The organic phase was evaporated and the residue chromatographed (silica gel, step gradient 0-50% ether/petroleum ether) to afford the title compound (3.1g). In similar manner 3-acetylthiomethylisoquinoline was prepared from 3-methylisoquinoline, and 2-acetylthiomethylquinoxaline was prepared from 2-methylquinoxaline.

### Preparation 7: 6-Bromomethylbenzo[b]furan

**Step 1: 6- Methylbenzo*[b]*furan -** A mixture of 2-(3-methylphenoxy)acetaldehyde (C.A. Lipinski et al., J.Med.Chem., 1980, 23, 1026) (14g), polyphosphoric acid (25g), and benzene (150ml) was heated at reflux for 2h then filtered through silica gel, evaporated, and chromatographed (silica gel, step gradient: 0-5% ethyl acetate/pentane) to give the subtitle compound containing 25% of 4-methylbenzo*[b]*furan, (5g).
**Step 2: 6-Bromomethylbenzo*[b]*furan -** Prepared from 6-methylbenzo*[b]*furan by the method of Preparation 4, Step 2, containing 25% of 4-bromomethylbenzo*[b]*furan.

**Preparation 8: 2-Acetylthiomethylnaphthalene -** Prepared by the method of Preparation 2, Step 2 from 2-bromomethylnaphthalene.

**Preparation 9: 6-Chloromethylbenzothiazole -** A mixture of 6-hydroxymethylbenzothiazole (A. Burger and S.N. Sawney, *J.Med.Chem.,* 1968, 11, 270) (0.8g) and pyridine (1.0ml) in dichloromethane (20ml) was treated with phosphorous pentachloride (1.0g). After 10min at 20°C the solution was washed with aqueous saturated sodium hydrogen carbonate, dried (MgSO4), and evaporated to give the title compound (0.7g).

### Preparation 10: 5-Acetylthiomethylthieno[2,3-b]pyridine

**Step 1: 5-Hydroxymethylthieno*[2,3-b]*pyridine -** A solution of thieno*[2,3-b]*pyridine-5-carboxylic acid (J. Bourgignon et al., *Tetrahedron*, 1988, 44, 1079) (1.6g) in THF (10ml) at 0°C was treated with triethylamine (1.3ml) and isobutyl chloroformate (1.2ml). After 5min at 0°C the mixture was filtered and the filtrate at 0°C treated with a solution of sodium borohydride (0.5g) in water (5ml). After a further 5min the reaction was made strongly acidic with 11M hydrochloric acid and after 30min more, partitioned between 2M aqueous sodium hydroxide and ether. The organic layer was dried (MgSO₄), evaporated, and chromatographed (silica gel, step gradient, 50-100% ethyl acetate/hexane) to give the subtitle compound (0.5g). ¹H NMR δ (CDCl₃): 8.1 and 8.5 (2H, 2d, J=1Hz), 7.2 and 7.5 (2H, 2d, J=6Hz), and 4.8 (2H, s).
**Step 2: 5-Acetylthiomethylthieno*[2,3-b]*pyridine -** Thionyl chloride (3ml) was added to a solution of 5-hydroxymethylthieno*[2,3-b]*pyridine (1.5g) in toluene (15ml). A black solid precipitated, the mixture was stirred for 18h then evaporated and redissolved in acetone (15ml). Potassium thioacetate (3g) was then added and after 3h the mixture was partitioned between aqueous saturated sodium hydrogen carbonate and ether. The organic layer was dried (MgSO₄), evaporated, and chromatographed (silica gel, step gradient: 25-50% ethyl acetate/hexane) to give the title compound (0.85g). ¹H NMR δ (CDCl₃) 8.0 and 8.5 (2H, 2d, J=1Hz), 7.2 and 7.5 (2H, 2d, J=6Hz), 4.2 (2H, s) and 2.4 (3H, s).

### Preparation 11: 6-Acetylthiomethylthieno[3,2-b]pyridine

**Step 1: 6-Hydroxymethylthieno*[3,2-b]*pyridine** - Prepared from thieno*[3,2-b]*pyridine-6-carboxylic acid (J. Bourgignon et al., *Tetrahedron*, 1988, 44, 1079) by the method of Preparation 10, Step 1. ¹H NMR δ (CDCl₃): 82 and 8.6 (2H, 2d, J=1Hz), 7.5 and 7.7 (2H, 2d, J=6Hz), and 4.9 (2H, s).
**Step 2: 6-Acetylthiomethylthieno*[3,2-b]*pyridine -** Prepared by the method of Preparation 10, Step 2. ¹H NMR δ (CDCl₃) 8.1 and 8.6 (2H, 2d, J=1Hz), 7.5 and 7.7 (2H, 2d, J=6Hz), 4.2 (2H, s) and 2.4 (3H, s).

**Preparation 12: 2-Fluoro-5-acetylthiomethylbenzo*[b]*thiophene -** Prepared from 2-fluoro-5-bromomethylbenzo*[b]*thiophene according to the method of Preparation 1, step 2.

### Examples

### Example 1: N-[2-(Benzo[b]thiophen-5-yl-methanesulfonyl)-1-(3,4-dichlorophenyl)-ethyl]-N-hydroxy-formamide.

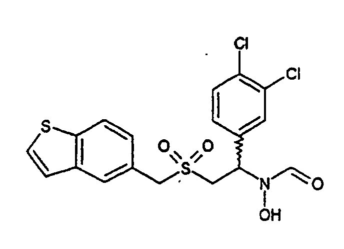

**Step 1: Benzo*[b]*thiophen-5-yl-methanethiol-** Sodium hydrosulfide (4.7g) in methanol (120ml) was added to Amberlite IRA-400-Cl resin (28g). To this stirred mixture was added triethylamine hydrochloride (3.8g) in methanol (28ml) followed after 10min by 5-bromomethylbenzo*[b]*thiophene (6g) in methanol (50ml). The mixture was left stirring until all 5-bromomethylbenzo*[b]*thiophene had been consumed (approx. 3h). The solution was then filtered through silica, which was washed with further quantities of methanol. The methanolic solutions were combined and evaporated. The residue was extracted with MDC, which was filtered and evaporated to give the subtitle compound as a solid (4g) from diethyl ether/hexane.
**Step 2: 2-(Benzo*[b]*thiophen-5-yl-methanesulfanyl)-1-(3,4-dichlorophenyl)-ethanone** - Benzo*[b]*thiophen-5-yl-methanethiol (0.5g) in MDC (15ml) was added to 2-bromo-1-(3,4-dichlorophenyl)-ethanone (0.797g) followed by triethylamine (0.41ml). The stirred solution was evaporated after 18h and purified by chromatography (silica gel, step gradient 15-80% MDC/hexane) to afford the subtitle compound (0.33g).
**Step 3: 2-(Benzo*[b]*thiophen-5-yl-methanesulfanyl)-1-(3,4-dichlorophenyl)-ethanone-*O*-benzyl-oxime -** 2-(Benzo*[b]*thiophen-5-yl-methanesulfanyl)-1-(3,4-dichlorophenyl)-ethanone (0.33g) in water/ethanol/THF (10:45:45; 10ml) was treated with O-benzyl-hydroxylamine (0.42g) and sodium acetate (0.22g) and heated to 80°C for 4h. Pyridine (2ml) was then added and heating continued for 10h. After cooling the reaction was evaporated and re-evaporated twice from toluene (25ml). The residue was taken up into EtOAc and washed with dilute HCl and brine. Evaporation gave the subtitle compound (0.4g).
**Step 4: *N*-[2-(Benzo*[b]*thiophen-5-yl-methanesulfanyl)-1-(3,4-dichlorophenyl)-ethyl)]-*O*-benzyl-hydroxylamine -** 2-(Benzo*[b]*thiophen-5-yl-methanesulfanyl)-1-(3,4-dichloro-phenyl)-ethanone-*O*-benzyl-oxime (0.4g) in acetic acid (20ml) at 40°C was treated periodically with portions of sodium borohydride (1.2g) over 4 days. At the end of this time the reaction was cooled, evaporated, extracted with EtOAc and washed with sodium hydrogen carbonate and brine. Evaporation and chromatography (silica gel, step gradient 3-80% EtOAc/hexane) afforded the subtitle compound (0.1g).
**Step 5: *N*-[2-(Benzo*[b]*thiophen-5-yl-methanesulfanyl)-1-(3,4-dichlorophenyl)-ethyl]-*N*-benzyloxy-formamide -** *N*-2-(Benzo*[b]*thiophen-5-yl-methanesulfanyl)-1-(3,4-dichlorophenyl)-ethyl)]-*O*-benzyl-hydroxylamine (0.1g) in EtOAc (1ml) was treated with premixed formic acid/acetic anhydride (2:1, 2ml). After 3h the reaction was evaporated and then re-evaporated three times from toluene to afford the subtitle compound (0.11g).
**Step 6: *N*-[2-(Benzo*[b]*thiophen-5-yl-methanesulfonyl)-1-(3,4-dichlorophenyl)-ethyl]-*N*-benzyloxy-formamide-** *N*-[2-(Benzo*[b]*thiophen-5-yl-methanesulfanyl)-1-(3,4-dichlorophenyl)-ethyl]-*N*-benzyloxy-formamide (0.11g) in EtOAc (1.5ml) was treated with MCPBA (86%, 0.09g) with rapid stirring at room temperature. After 10 min dimethyl sulphide (1ml) was added and after a further 10 min the reaction was evaporated and then re-evaporated twice from toluene. Chromatography (silica gel, step gradient 5% - 80% EtOAc/hexane) afforded the subtitle compound (0.094g).
**Step 7: *N*-[2-(Benzo*[b]*thiophen-5-yl-methanesulfonyl)-1-(3,4-dichloro-phenyl)-ethyl]-*N*-hydroxy-formamide -** *N*-[2-(Benzo*[b]*thiophen-5-yl-methanesulfonyl)-1-(3,4-dichlorophenyl)-ethyl]-*N*-benzyloxy-formamide (0.094g) was treated, under argon, with boron trichloride:dimethylsulfide complex (0.15g) dissolved in MDC (1ml). To the reaction was then added anisole (0.5ml) and stirring was continued for 3 days. The reaction was then diluted with EtOAc and washed with sodium hydrogen carbonate and brine. Evaporation and purification by prep-HPLC afforded the title compound as a solid (0.02g). MS electrospray (+ve ion) 444 (MH⁺), 461 (MH⁺ + NH₃), 904 (2MH⁺ + NH₃), 909 (2MNa⁺); MS electrospray (-ve ion) 441.9 (M-H⁻); ¹H NMR δ(CDCl₃ ), 7.1-8.5 (10H, m, aromatics + CHONOH rotamers), 5.89-6 and 5.2-5.38 (1H, m x 2, rotamers), 4.5 and 4.35 (2H, br s x 2, rotamers), 4.0 and 3.15 plus 3.6 and 3.22 (2H, br AB x 2, rotamers).

The compounds of the following examples were prepared by the procedures described in Example 1. ¹H NMR and mass spectra were consistent with the proposed structures.

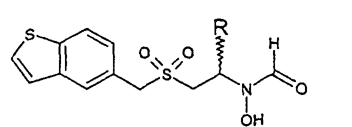

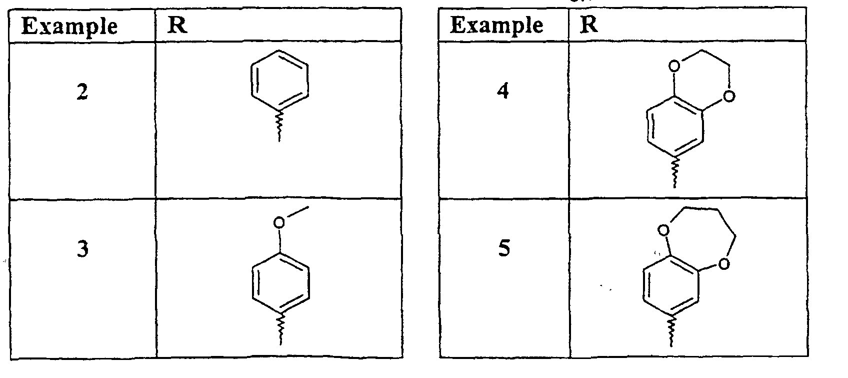

### Example 6: (S)-N-[2-(Benzo[b]thiophen-5-yl-methanesulfonyl)-1-(4-methoxyphenyl)-ethyl]-N-hydroxyformamide.

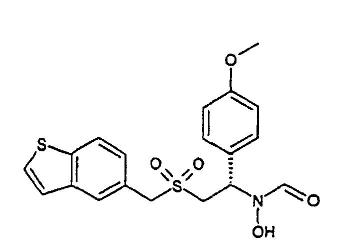

**Step 1: 2-Benzo*[b]*thiophen-5-yl-methanesulfanyl)-1-(4-methoxyphenyl)ethanone-** Thioacetic acid *S*-benzo*[b]*thiophen-5-yl-methyl ester (5.9g) in methanol (60ml) was treated with 1 equivalent of sodium methoxide as a solution in methanol. After 5min 2-bromo-1-(4-methoxy-phenyl)-ethanone (5.1g) in THF (25ml) was added. After a further 3h the reaction was diluted with EtOAc, washed with brine, sodium bicarbonate solution and water. The EtOAc layer was separated, dried (MgSO₄) and evaporated.
   Chromatography (silica gel, step elution: 10-50% EtOAc/hexane) afforded the subtitle compound as a crystalline solid (6.9g).
**Step 2: (S)-2-(Benzo*[b]*thiophen-5-yl-methanesulfanyl)-1-(4-methoxyphenyl)ethanol-** (S)-2-Methyl-CBS-oxazaborolidine (21ml of a 1M solution in toluene) and borane dimethyl sulfide complex (10.5ml of a 2M solution in toluene) were mixed together under argon for 15 min. The reaction was then cooled to -30°C and 2-benzo*[b]*thiophen-5-ylmethanesulfanyl)-1-(4-methoxyphenyl)ethanone (6.9g) in toluene (35ml) was added dropwise over *circa* 20 min to maintain the reaction temperature at < -20°C. After 1h methanol (10ml) was added carefully, with consequential hydrogen evolution. The reaction was allowed to warm to rt and evaporated from toluene (30ml), diluted with EtOAc and washed with diluted HCl and saturated sodium hydrogen carbonate solution. Evaporation of the EtOAc layer provided a solid which could either be crystallised from EtOAc/ether/hexane or chromatographed (silica gel, step elution: 5-45% EtOAc/hexane) to give the subtitle product (6.86g).
**Step 3: (S)-*N*-[2-(Benzo*[b]*thiophen-5-yl-methanesulfanyl)-1-(4-methoxyphenyl)ethyl]-N,O-bis-tert-butoxycarbonyl-hydroxylamine-** (S)-2-(Benzo*[b]*thiophen-5-yl-methanesulfanyl)-1-(4-methoxy-phenyl)-ethanol (6.1g) in THF/toluene (60ml,1:1) under argon was treated with N,O-bis-tert-butoxycarbonyl-hydroxylamine (9g) and 1,1'-azobis(N,N-dimethylformamide) (6.4g). After cooling to 0°C tributylphosphine (9.1ml) was added dropwise and the reaction then allowed to warm to rt. After 4h the reaction was quenched with water (10ml) diluted with EtOAc and washed with brine. After filtering through a short plug of silica gel the EtOAc layer was evaporated and the residue chromatographed on (silica gel, step elution: 3-10% EtOAc/hexane) to give the subtitle compound as a gum (5.18g). Also obtained was 6.84g of material as a 50:50 mixture of title compound and N,O-bis-tert-butoxycarbonyl-hydroxylamine which could be repurified.
**Step 4: (S)-*N*-[2-(Benzo*[b]*thiophen-5-yl-methanesulfonyl)-1-(4-methoxyphenyl)ethyl]-N,O-bis-tert-butoxycarbonyl-hydroxylamine-** (S)-*N*-[2-(Benzo*[b]*thiophen-5-yl-methanesulfanyl)-1-(4-methoxy-phenyl)ethyl]-N,O-bis-tert-butoxycarbonyl-hydroxylamine (8.6g) in EtOAc (100ml) at 0°C was treated with MCPBA (86% purity, 9.6g). After 15min the reaction was quenched with dimethylsulfide (3ml), diluted with EtOAc and washed with saturated sodium hydrogen carbonate solution. Evaporation of the EtOAc layer and chromatography (silica gel, step elution: 5-25% EtOAc/hexane) gave the subtitle compound as a foam (6.29g).
**Step 5: (S)-*N*-[2-(Benzo*[b]*thiophen-5-yl-methanesulfonyl)-1-(4-methoxy-phenyl)ethyl]-hydroxylamine-** (S)-*N*-[2-(Benzo*[b]*thiophen-5-yl-methanesulfonyl)-1-(4-methoxy-phenyl)ethyl]-N,O-bis-tert-butoxycarbonyl-hydroxylamine (6g) was dissolved into 95% TFA/water (100ml) and left for 1 h. Toluene (30ml) was added and the reaction was evaporated and re-evaporated from toluene (30ml × 2), dissolved into EtOAc and treated with saturated sodium hydrogen carbonate solution until the EtOAc layer was neutral. Evaporation of the organic layer provided the subtitle compound (3.82g).
**Step 6: (S)-*N*-[2-(Benzo*[b]*thiophen-5-yl-methanesulfonyl)-1-(4-methoxy-phenyl)ethyl]-*N*-hydroxyformamide-** Acetic anhydride (60ml) and formic acid (180ml) were mixed at room temperature for 15min. To this was added (S)-*N*-[2-(Benzo*[b]*thiophen-5-yl-methanesulfonyl)-1-(4-methoxy-phenyl)ethyl]-hydroxylamine (3.8g) in formic acid (20ml). After 4h the reaction was evaporated from toluene (30ml, × 3) taken up into methanol (60ml) and evaporated. The residue was dissolved into EtOAc/methanol (200ml, 3:1) and stirred with sodium carbonate (2g) for two days. Dilution with EtOAc, washing with brine and evaporation afforded a solid which was chromatographed (silica gel, step elution: 5-25% EtOAc/Hexane folowed by 80% EtOAc/acetone) to afford a solid which was triturated with MDC to give the title compound with >98% ee (2.43g). MS electrospray (+ve ion) 406 (MH⁺), 833 (2MNa⁺); MS electrospray (-ve ion) 404 (M-H⁻), 808.9 (2M-H⁻); ¹H NMR δ [(CD₃)₂CO], 6.9-8.9 (9H, m, aromatics + CHONOH rotamers), 5.4-5.63 and 5.9-6.15 (1H, m x 2, rotamers), 4.55 (2H, s), 3.9-4.29 and 3.45-3.74 (2H, br AB x 2, rotamers) and 3.8 (3H, s).

**The compounds of the following examples were prepared by the procedures described in Example 6.** ^{**1**}**H NMR and mass spectra were consistent with the proposed structures.**

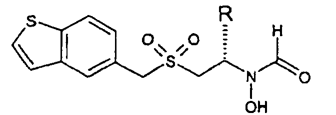

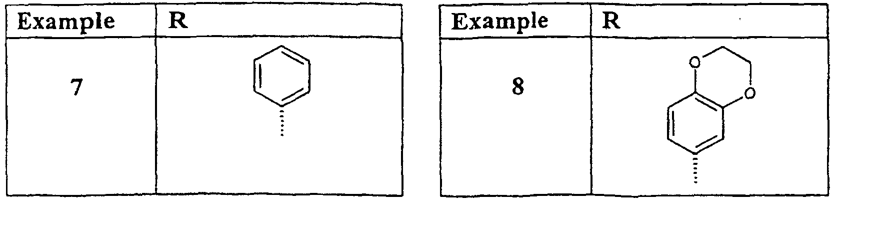

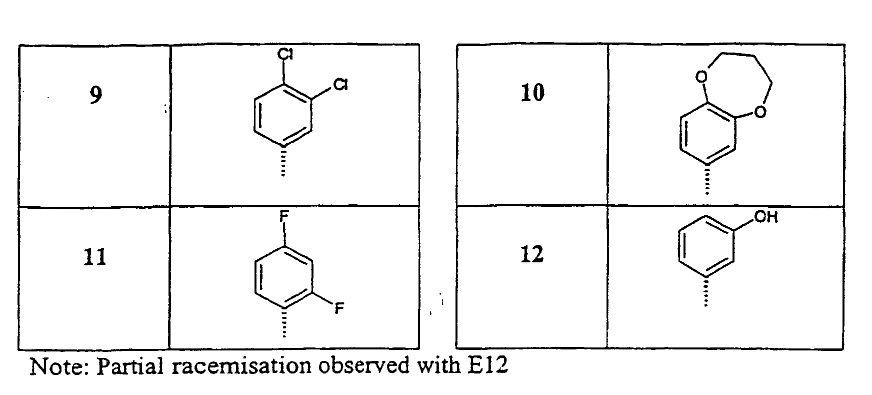

### Example 7 (S)-N-[2-(Benzo[b]thiophen-5-ylmethanesulfonyl)-1-phenyl)ethyl]-N-hydroxyformamide (Alternative Synthetic Route)

**Step 1: (S)-*N*-[2-(4-Toluenesulfonyloxy)-1-phenylethyl]-N-tert-butoxycarbonylamine** - A solution of (S)-*N*-(*tert*-butoxycarbonyl)-2-phenylglycinol (0.86g) in MDC (20ml) was stirred with *p*-toluenesulfonyl chloride (1.39g), triethylamine (0.56ml) and dimethylaminopyridine (catalytic amount) at rt overnight. The mixture was diluted with MDC (20ml) and washed with water (3x10ml), brine (10ml) and then dried (MgSO₄).
   The organic layer was then evaporated to a crude which was purified by chromatography (silica gel; step gradient: 30-50% ether/hexane) to give the subtitle compound (1.18g).
**Step 2: (S)-*N*-[2-(Benzo*[b]*thiophen-5-yl-methanesulfanyl)-1-phenylethyl]-N-tert-butoxycarbonylamine** - (S)-*N*-[2-(4-Toluenesulfonyloxy)-1-phenylethyl]-N-tert-butoxycarbonylamine (1.00g) and thioacetic acid *S*-benzo*[b]*thiophen-5-yl-methyl ester (0.68g) were reacted to form the subtitle compound (0.51g) in a similar manner to Example 6, step 1.
**Step 3: (S)-*N*-[2-(Benzo*[b]*thiophen-5-yl-methanesulfonyl)-1-phenylethyl]-N-tert-butoxycarbonylamine** - (S)-*N*-[2-(Benzo*[b]*thiophen-5-yl-methanesulfanyl)-1-phenylethyl]-N-tert-butoxycarbonylamine (0.51g) was reacted with MCPBA (0.51g) in a similar manner to Example 6, step 4 to give the subtitle compound (0.43g).
**Step 4: (S)-*N*-[2-(Benzo*[b]*thiophen-5-ylmethanesulfonyl)-1-phenylethyl]amine -** (S)-*N*-[2-(Benzo*[b]*thiophen-5-yl-methanesulfonyl)-1-phenylethyl]-*N*-tert-butoxycarbonylamine (0.38g) was treated with 95% TFA/water (5ml). After 4h the solution was concentrated. The residue was re-evaporated from toluene (3x5ml) and then purified by chromatography (silica gel; 2% methanol/MDC) to give the subtitle compound (0.26g).
**Step 5: (S)-*N*-[2-(Benzo*[b]*thiophen-5-ylmethanesulfonyl)-1-phenylethyl]-O-benzoyl-hydroxylamine** - (S)-*N*-[2-(Benzo[b]thiophen-5-ylmethanesulfonyl)-1-phenylethyl]amine (0. 10g) was suspended in degassed pH 10.5 buffer solution (3ml, solution made up from 222ml 0.75M sodium bicabonate with 78ml 1.5M sodium hydroxide) and treated with benzoyl peroxide (0.104g, 70% in water) in MDC (3ml). Argon was bubbled through the mixture for 5min and stirring continued for 6 days. A further 1ml of buffer was added after 3 days and 2ml of MDC added after 5 days. The reaction mixture was diluted with MDC (25ml) and water (10ml) and the aqueous layer extracted with MDC (2x10ml). The combined organic layers were washed with saturated sodium bicarbonate solution (10ml), water (10ml) and brine (10ml) and then dried (MgSO₄). The solvent was evaporated and the crude purified by chromatography (silica gel; step gradient; 0-50% ethyl acetate/hexane) to give the subtitle compound (0.022g)
**Step 6 : (S)-*N*-[2-(Benzo*[b]*thiophen-5-ylmethanesulfonyl)-1-phenylethyl]-*N*-hydroxy formamide -** (S)-*N*-[2-(Benzo[b]thiophen-5-ylmethanesulfonyl)-1-phenylethyl-O-benzoyl-hydroxylamine (0.012g) was dissolved in formic acid (0.25ml) and treated with a premixed solution of formic acid/acetic anhydride (1ml; 3:1 formic acid: acetic anhydride). The solution was stirred overnight and then evaporated to dryness. The residue was re-evaporated from toluene (3x) and then treated with 2% ammonia in methanol solution (1ml). The solution was stirred for 45min and the solvent evaporated. The residue was evaporated from toluene (3x) and purified by reverse phase preparative HPLC chromatography to give the title compound (0.01g). MS electrospray (+ve ion) 376 (MH⁺); ¹H NMR δ(DMSO-d6): 10.18 and 9.85 (1H, s, rotamers), 8.20 (1H, s), 8.38 (1H, d, J=8.0Hz), 7.87 (1H, s), 7.82 (1H, d, J=5.2Hz), 7.49 (1H, d, J=5.3Hz), 7.35 (6H, m), 5.93 and 5.56 (1H, m +d, rotamers), 4.64 (2H, s), 4.10 - 3.63 (2H, m, rotamers). Analytical Chiral HPLC : Single peak.

### Example 13: (S)-N-[2-(Benzo[b]thiophen-5-ylmethanesulfonyl)-1-(4-dimethylaminosulfonylphenyl)ethyl]-N-hydroxyformamide

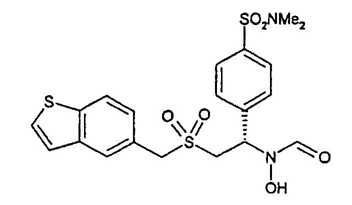

**Step 1: 4-Acetyl-N,*N*-dimethylbenzenesulfonamide -** 4-Acetylbenzenesulfonyl chloride (1.00g) was stirred in water (50ml) at 5°C and then dimethylamine (1.30ml, 33% solution in ethanol) added dropwise. The reaction was stirred at 5°C for 30 min and then allowed to warm to room temperature over night. The reaction mixture was then evaporated down to a minimum and redissolved in ethyl acetate (100ml) and washed with 1M aqueous HCl (200ml), brine (400ml) and water (100ml). The organic layer was dried (magnesium sulfate) and then evaporated to give the subtitle compound as a white solid (0.96g).
**Step 2: 4-(2-Bromoacetyl)-*N,N*-dimethylbenzenesulfonamide -** 4-Acetyl- *N,N*-dimethyl-benzenesulfonamide (0.96g) was stirred with copper bromide (1.57g) in ethyl acetate (25ml) and heated to reflux for 2.5 h. The reaction mixture was allowed to cool to rt then filtered through activated charcoal. The filtrate was then evaporated give the subtitle compound as a yellow oil (0.52g).
**Step 3: 4-[2-(Benzo[b]thiophen-5-ylmethylsulfanyl)ethanoyl]-N,N-dimethylbenzenesulfonamide -** 4-(2-Bromoacetyl)-*N,N*-dimethylbenzenesulfonamide was converted to the subtitle compound using the procedure described in Example 6, step 1. ¹H NMR δ (CDCl₃), 8.05-7.29 (9H, aromatics), 3.87 (2H, s), 3.67 (2H, s), 2.73 (6H, s).
**Step 4: (S)-4-[2-(Benzo[*b*]thiophen-5-ylmethylsulfanyl)-1-hydroxyethyl]-*N,N*-dimethyl-benzenesulfonamide -** 4-[2-(Benzo[*b*]thiophen-5-ylmethylsulfanyl)ethanoyl]-N,N-dimethyl-benzenesulfonamide was converted to the subtitle compound using the procedure described in Example 6, step 2. ¹H NMR δ (CH₃OD), 7.82-7.12 (9H, aromatics), 4.77 (1H, m), 3.90 (2H, s), 3.00-2.73 (2H, m), 2.64 (6H, s).
**Step 5: (S)-*N*-[2-(Benzo[*b*]thiophen-5-ylmethanesulfonyl)-1-(4-dimethylaminosulfonylphenyl)ethyl]-N,O-bis-tert-butoxycarbonylhydroxylamine -** (S)-4-[2-(Benzo[*b*]thiophen-5-ylmethylsulfanyl)-1-hydroxyethyl]-*N*,*N*-dimethylbenzenesulfonamide was converted to the subtitle compound using the procedures described in Example 6, steps 3 and 4.
   MS electrospray (+ve ion) 655 (MH⁺).
**Step 6: (S)-*N*-[2-(Benzo*[b]*thiophen-5-ylmethanesulfonyl)-1-(4-dimethylaminosulfonylphenyl)ethyl]hydroxylamine -** (S)-N-[2-(Benzo[*b*]thiophen-5-ylmethanesulfonyl)-1-(dimethylaminosulfonylphenyl)ethyl]-N,O-bis-tert-butoxycarbonylhydroxylamine was converted to the subtitle compound using the procedure described in Example 6, step 5. ¹H NMR δ (CH₃OD), 7.94-7.15 (9H, aromatics), 4.75 (1H, m), 4.54 (2H, m), 4.22-3.84 and 3.82-3.55 (2H, m), 2.67 (6H, s).
**Step 7: (S)-*N*-[2-(Benzo[*b*]thiophen-5-ylmethanesulfonyl)-1-(4-dimethylaminosulfonyl-phenyl)ethyl]-*N*-hydroxyformamide** - (S)-*N*-[2-(Benzo[*b*]thiophen-5-ylmethanesulfonyl)-1-(4-dimethylaminosulfonylphenyl)ethyl]hydroxylamine was converted to the title compound using the procedure described in Example 6, step 6, (0.01g). Chiral purity: 75%ee. ¹H NMR δ (CD₃OD), 8.29 and 8.09 (1H, 2 x s, rotamers), 7.93-7.31(9H, m), 6.10 and 5.56 (1H, m x 2, rotamers), 4.61 (2H, s), 4.41-3.99 and 3.73-3.59 (2H, m x 2, rotamers) and 2.64 (6H, s).

**The compounds of the following examples were prepared by the procedures described in Example 6.** ^{**1**}**H NMR and mass spectra were consistent with the proposed structures.**

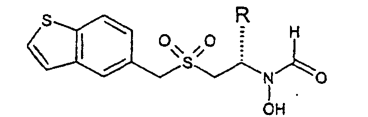

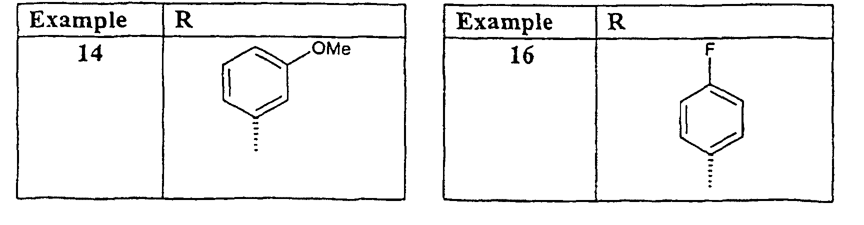

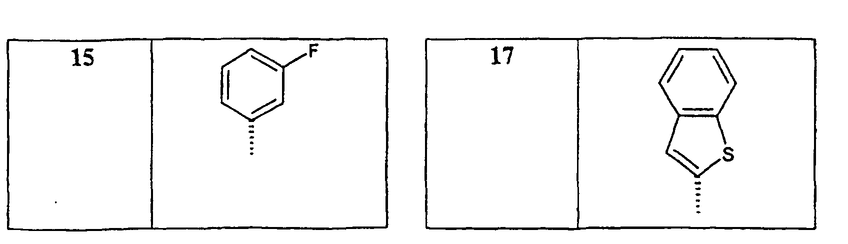

### Example 18: (S)-N-[2-(Benzo[b]thiophen-5-yl-methanesulfonyl)-1-(3-methylthiophen-2-yl)ethyl]-N-hydroxyformamide.

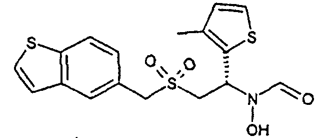

A solution of 2-acetyl-3-methylthiophene (1.25g) in methanol (15ml) was stirred at room temperature and treated dropwise with a solution of bromine (0.46ml) in methanol (5ml) added over 10min. After being stirred for a further 1h, the volatile components were removed under reduced pressure to afford 2-bromo-1-(3-methylthiophen-2-yl)-ethanone as a pale oil. This was converted into the title compound using the procedures described in Example 6 steps 1-6.

### Example 19: (S)-N-[2-(Benzo[b]thiophen-5-yl-methanesulfonyl)-1-(1-t-butyl-5-methylpyrazol-3-yl)ethyl]-N-hydroxyformamide.

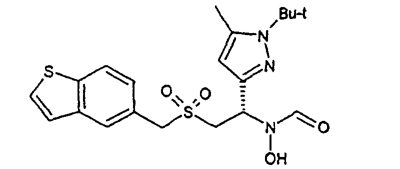

**Step 1: 2-Diazo-1-(1-t-butyl-5-methylpyrazol-3-yl)-ethanone -** A stirred solution of 1-t-butyl-3-carboxy-5-methylpyrazole (0.50g) in MDC (10ml) was treated with *N,N*-dimethylformamide (1 drop) and oxalyl chloride (0.288ml) and stirred at rt for 1 h. The volatile components were then removed under reduced pressure. Re-evaporation from toluene afforded the acid chloride. A solution of trimethylsilyldiazomethane (2.0M solution in hexane, 1.72ml) in THF (5ml) and acetonitrile (5ml) was stirred under argon at 0°C and treated with polybase (1.075g) followed by a solution of the acid chloride in MDC (3ml) added dropwise. The mixture was stirred at 0°C for 3h and then at rt overnight. Filtration of the resin and removal of the solvent under reduced pressure gave the crude product which was chromatographed (silica gel, EtOAc/hexane 1:4) to give the subtitle compound as a yellow solid (245mg).
**Step 2: 2-Bromo-1-(1-t-butyl-5-methyl-pyrazol-3-yl)-ethanone**
   A solution of 2-diazo-1-(1-t-butyl-5-methylpyrazol-3-yl)-ethanone (240mg) in acetic acid (5ml) was stirred at rt and treated with a 30% solution of hydrogen bromide in acetic acid (0.28ml). Nitrogen was immediately evolved and the solution was stirred for a further 45 min. The volatile components were then removed under reduced pressure and the resulting oil re-evaporated from toluene (3x) to afford the subtitle compound as an oil.
**Step 3: (S)-*N*-[2-(Benzo*[b]*thiophen-5-yl-methanesulfonyl)-1-(1-t-butyl-5-methylpyrazol-3-yl)ethyl]-*N*-hydroxyformamide -** Prepared from 2-bromo-1-(1-t-butyl-5-methyl-pyrazol-3-yl)-ethanone using the procedures described in Example 6 steps 1-6.

### Example 20: (S)-N-[2-(Benzo[b]thiophen-5-ylmethanesulfonyl)-1-(furan-2-yl)ethyl]-N-hydroxyformamide.

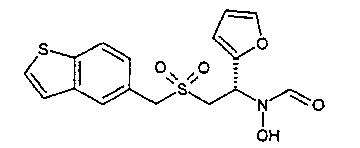

**Step 1: 2-Diazo-1-(furan-2-yl)-ethanone -** Prepared from furan-2-carboxylic acid as described in Example 19 Step 1.
**Step 2: 2-Bromo-1-(furan-2-yl)-ethanone -** Prepared from 2-diazo-1-(furan-2-yl)-ethanone as desribed in Example 19 Step 2.
**Step 3: 2-(Benzo*[b]*thiophen-5-yl-methanesulfanyl)-1-(furan-2-yl)-ethanone -** Prepared from 2-bromo-1-(furan-2-yl)-ethanone as described in Example 6 Step 1.
**Step 4: (S)-2-(Benzo*[b]*thiophen-5-yl-methanesulfanyl)-1-(furan-2-yl)-ethanol -** Prepared from 2-(benzo*[b]*thiophen-5-yl-methanesulfanyl)-1-(furan-2-yl)-ethanone as described in Example 6 Step 2.
**Step 5: (S)-*N*-[2-(Benzo*[b]*thiophen-5-yl-methanesulfanyl)-1-(furan-2-yl)ethyl]-N,O-bis-benzyloxycarbonyl-hydroxylamine -** (S)-2-(Benzo*[b]*thiophen-5-ylmethanesulfanyl)-1-(furan-2-yl)-ethanol (0.23g) in toluene (5ml) under argon was treated with N,O-bis-benzyloxycarbonyl-hydroxylamine (0.47g) and 1,1'-azobis(N,N-dimethylformamide) (0.28g). After cooling to 0°C tributyl-phosphine (0.41ml) was added dropwise and the reaction was then allowed to warm to rt. After 2h the reaction mixture was evaporated to dryness and the residue chromatographed (silica gel, 25% EtOAc/hexane) to give the crude product as a colourless gum (0.61g).
**Step 6: (S)-*N*-[2-(Benzo*[b]*thiophen-5-yl-methanesulfonyl)-1-(furan-2-yl)ethyl]-N,O-bis-benzyloxycarbonyl-hydroxylamine -** Prepared from (S)-*N*-[2-(benzo*[b]*thiophen-5-yl-methanesulfanyl)-1-(furan-2-yl)ethyl]-N,O-bis-benzyloxycarbonyl-hydroxylamine as described in Example 6 Step 4.
**Step 7: (S)-*N*-[2-(Benzo*[b]*thiophen-5-yl-methanesulfonyl)-1-(furan-2-yl)ethyl]hydroxylamine -** A solution of (S)-*N*-[2-(benzo*[b]*thiophen-5-ylmethanesulfonyl)-1-(furan-2-yl)ethyl]-N,O-bis-benzyloxycarbonyl-hydroxylamine (156mg) in methanol (12ml) was hydrogenated over 10% palladium on charcoal (180mg) for three days. The catalyst was removed by filtration and the solvent evaporated to afford an oil. This monoprotected intermediate (123mg) was dissolved in dry MDC, under argon, cooled to 0°C and treated with trimethylsilyl iodide (0.11ml). The mixture was stirred at 0°C for 1h followed by 1h at rt. Saturated sodium hydrogen carbonate solution was then added followed by EtOAc, and the organic layer dried (MgSO₄). Removal of the solvent gave the subtitle compound as a dark oil.
**Step 8: (S)-*N*-[2-(Benzo*[b]*thiophen-5-yl-methanesulfonyl)-1-(furan-2-yl)ethyl]-*N*-hydroxy-formamide -** Prepared from (S)-*N*-[2-(Benzo*[b]*thiophen-5-ylmethanesulfonyl)-1-(furan-2-yl)ethyl]hydroxylamine as described in Example 6 Step 6. The crude product was purified by chromatography (silica gel, EtOAc/hexane 2:1) to give the title compound as a pale solid. ¹H NMR δ(CDCl₃), 8.23 (1H, CHO), 7.9 - 7.3 and 6.4 (8H, aromatics), 5.78 (1H, dd), 4.09 (2H, m, retamers), 3.62 (1H, dd), 3.35 (1H, dd).

### Example 21: (S)-N-[2-(Benzo[b]thiophen-5-yl-methanesulfonyl)-1-(furan-3-yl)ethyl]-N-hydroxyformamide.

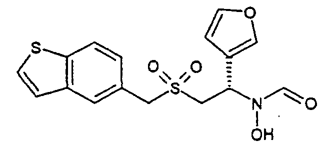

Prepared as described for Example 20, except that in Step7 deprotection was carried out by direct treatment of the N,O-bis-benzyloxycarbonyl-hydroxylamine with trimethylsilyl iodide without prior hydrogenation. The crude product was purified by chromatography (silica gel, EtOAc/hexane 2:1) to give the title compound as a pale solid. ¹H NMR δ(DMSO-d₆), 10.2 and 9.89 (1H, NOH, rotamers), 8.32 and 8.21 (1H, CHO), 8.0-7.3 and 6.52 (8H, aromatics), 5.90 and 5.52 (1H, m, rotamers), 4.61 (2H, s), 3.84 (1H, m), 3.57 (1H, m).

### Example 22: (S)-N-[1-Phenyl-2-(2-indanylmethanesulfonyl)ethyl]-N-hydroxyformamide

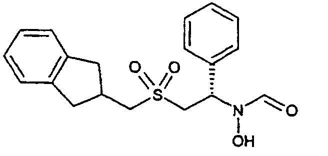

The title compound was prepared using the method of Example 6. MS (+ve ion electrospray) 382 (MNa⁺, 100%), ¹H NMR δ(CDCl₃), 8.44 and 8.15 (1H, 2s), 7.0-7.4 (10H, m), 5.4 and 6.0 (1H, 2m), and 2.8-4.2 (9H, m).

### Example 23: (S)-N-[1-Phenyl-2-(benzo[b]furan-5-yl-methanesulfonyl)ethyl]-N-hydroxyformamide

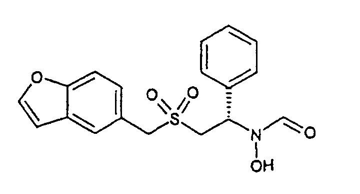

**Step 1: α-(5-Benzo*[b]*furylmethylthio)acetophenone ―** A mixture of 5-bromomethylbenzo*[b]*furan (0.85g), α-mercaptoacetophenone (0.60g), triethylamine (0.56ml), and MDC (20ml) was stirred at rt for 18h then directly chromatographed (silica gel, step gradient 0-25% EtOAc/hexane) to give the subtitle compound (0.70g). Converted to the title compound using the method of Example 6, Steps 2-6. MS (+ve ion electrospray) 382 (MNa⁺, 44%), 131 (100%); ¹H NMR δ(CD₃OD), 8.27 (1H, s), 7.88 (1H, s), 7.78 (1H, s), 7.3-7.6 (7H, m), 6.82 (1H, s), 5.4 and 6.0 (1H, 2m), 4.54 (2H, s), and 3.4-4.2 (2H, m).

### Example 24: (S)-N-[1-Phenyl-2-(2-fluoro-benzo[b]thiophen-5-yl-methanesulfonyl)ethyl]-N-hydroxyformamide

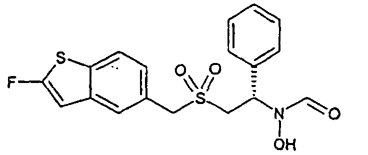

A mixture of 2-fluoro-5-bromomethylbenzo*[b]*thiophene (0.35g), (R)-2-mercapto-2-phenylethanol (0.23g) (M. C. Aversa et al, *J*. *Org. Chem*., 1997, *62(13)*, 4376), sodium hydroxide (0.06g), ethanol (5ml), and water (1ml), was stirred at rt for 18h then partitioned between saturated aqueous ammonium chloride and diethyl ether. The organic layer was dried (MgSO₄) and evaporated and the residue chromatographed (silica gel, step gradient 10-30% EtOAc/hexane) to give (R)-2-phenyl-2-(2-fluorobenzo*[b]*thiophen-5-yl-methanesulfanyl)ethanol (0.25g). This was converted into the title compound using the method of Example 6, Steps 3-6. MS (+ve ion electrospray) 411 (MNH₄⁺, 54%), 457 (100%); ¹H NMR δ(CDCl₃), 8.23 and 8.36 (1H, 2s), 7.88 (1H, s), 7.3-7.5 (7H, m), 6.73 (1H, s), 5.4 and 6.0 (1H, 2m), and 3.2-4.4 (4H, m).

### Example 25: (S)-N-[1-Phenyl-2-(3-fluoro-benzo[b]thiophen-5-ylmethanesulfonyl)ethyl]-N-hydroxyformamide

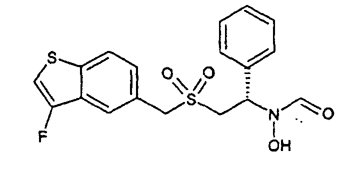

The title compound was prepared from 3-fluoro-5-bromomethylbenzo*[b]*thiophene by the method of Example 24. MS (+ve ion electrospray) 394 (MNa⁺, 62%), 165 (100%); ¹H NMR δ(DMSO-d₆), 10.23 and 9.86 (1H, 2bs), 8.27 (1H, s), 8.1 (1H, m), 7.78 (1H, s), 7.3-7.5 (7H, m), 5.5 and 6.0 (1H, 2m), and 3.6-4.2 (4H, m).

### Example 26: (S)-N-[1-Phenyl-2-(3-quinolylmethanesulfonyl)ethyl]-N- hydroxyformamide

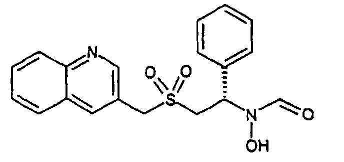

**Step 1: (S)-1-Phenyl-2-(3-quinolylmethanesulfanyl)ethanol and (R)-2-phenyl-2-(3-quinolylmethanesulfanyl)ethanol -** To 3-acetylthiomethylquinoline (5.0g) in methanol (50ml) was added aqueous sodium hydroxide (11.5ml, 2M). After 15min at rt (S)-phenyloxirane (2.8ml) was added, and after another 15min the solution was partitioned between saturated aqueous ammonium chloride and diethyl ether. The organic layer was dried (MgSO₄) and evaporated and the residue chromatographed (silica gel, step gradient: 25-75% ethyl acetate/hexane) to give a 1:1 mixture of the two subtitle compounds (6.1g). ¹H NMR δ(CDCl₃) 8.7 and 8.8 (1H, 2d, J=2Hz), 7.5-8.1 (5H, m), 7.2-7.3 (5H, m), 4.8 (0.5H, m), 3.7-3.9 (3.5H, m), 3.3(0.5H, d, J=3Hz), 2.7 (1H, m), and 2.6 (1H, bs).
**Step 2: (S)-N-[2-(3-Quinolylmethanesulfanyl)-1-phenylethyl]-N,O-bis-tert-butoxycarbonylhydroxylamine -** The mixture of (S)-1-phenyl-2-(3-quinolylmethanesulfanyl)ethanol and (R)-2-phenyl-2-(3-quinolylmethanesulfanyl)ethanol (6.1g) in toluene (100ml) under argon was treated with N,O-bis-tert-butoxycarbonyl-hydroxylamine (7.0g) and 1,1'-azobis(N,N-dimethylformamide) (6.9g). After cooling to 0°C tributylphosphine (10.0ml) was added dropwise and the reaction then allowed to warm to rt. After 1.5h the reaction was evaporated and the residue chromatographed twice (silica gel, step gradient, 25-50% ethyl acetate/hexane) to give the subtitle compound as a gum (5.7g). MS (+ve ion electrospray) 511 (MH⁺, 100%).
**Step 3: (S)-N-[2-(3-Quinolylmethanesulfonyl)-1-phenylethyl]-N,O-bis-tert-butoxycarbonyl-hydroxylamine** - (S)-N-[2-(3-Quinolylmethanesulfanyl)-1-phenylethyl]-N,O-bis-tert-butoxycarbonylhydroxylamine (5.7g) in dichloromethane (100ml) at 0°C was treated with MCPBA (65% purity, 5.5g). After 30min the reaction was washed with saturated sodium hydrogen carbonate solution containing 1% sodium sulfite. Evaporation of the organic layer and chromatography (silica gel, step gradient: 25-50% ethyl acetate/hexane) gave the subtitle compound as a foam (2.5g). MS (+ve ion electrospray) 343 (MH⁺-2Boc, 52%), and 143 (100%).
**Step 4: (S)-*N*-[1-Phenyl-2-(3-quinolylmethanesulfonyl)ethyl]-*N*-hydroxyformamide** - A solution of (S)-N-[2-(3-quinolylmethanesulfonyl)-1-phenylethyl]-N,O-bis-tert-butoxycarbonyl-hydroxylamine (2.5g) in dichoromethane (50ml) was treated with trifluoroacetic acid (20ml). After 30min the solution was evaporated and redissolved in formic acid (60ml) and acetic anhydride (20ml) was added. After a further 1.5h the solution was again evaporated and redissolved in methanol (50ml). Potassium carbonate (2g) was then added and after 15min water was added and the pH adjusted to 7 with hydrochloric acid. Extraction with ethyl acetate and chromatography (silica gel, step gradient: 0-5% methanol/ethyl acetate) gave the title compound as a solid after trituration with ether (0.75g). MS (+ve ion electrospray) 371 (MH⁺, 100%); ¹H NMR δ(CDCl₃): 8.9 (1H, m), 8.3 (2H, m), 8.09 (1H, d, J=8Hz), 7.85 (1H, d, J=8Hz), 7.78 (1H, t, J=8Hz), 7.62 (1H, t, J=8Hz),7.3-7.5 (5H, m), 5.4 and 6.1 (1H, 2m), and 3.2-4.4 (4H, m); [α]_{D} +35° (c, 0.3, ethanol).

### Example 27 : (S)-N-[2-(Benzo[b]furan-5-yl-methanesulfonyl)-1-(4-methoxy-phenyl)-ethyl]-N-hydroxyformamide

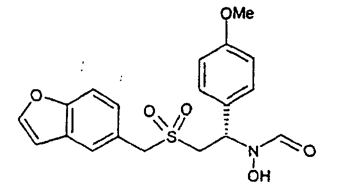

The title compound was prepared as for Example 6 Steps 1-6; followed by either crystallisation from ethanol (Method A), or additional Steps 1,2 (Method B). ¹H NMR δ(CDCl₃): 6.8-8.2 (9H, m, aromatics + CHONOH rotamers), 4.15 (2H, s), 3.8-4.1 (1H, m), 3.78 (2H, s), 3.34( 3H, s).

### Method B:

**Step 1: (S)-*N*-[2-(Benzo*[b]*furan-5-yl-methanesulfonyl)-1-(4-methoxyphenyl)ethyl]-*N*-((R)-2-methoxy-2-phenyl-ethanoyloxy)-formamide-** A solution of (S)-*N*-[2-(benzofuran-5-yl-methanesulfonyl)-1-(4-methoxyphenyl)ethyl]-*N*-hydroxyformamide (0.055g) in MDC (2.82ml) was treated with [R]-methoxyphenylacetic acid (23.4mg). CDI (0.022g) was then added and the reaction mixture left overnight. The reaction was evaporated then redissolved in toluene and chromatographed (silica gel, step gradient 2-20% EtOAc/toluene) to give the subtitle compound (2.1mg).
**Step 2: (S)-*N*-[(Benzo*[b]*furan-5-yl-methanesulfonyl)-1-(4-methoxyphenyl)ethyl]-*N*-hydroxyformamide-** A solution of (S)-*N*-[2-(benzo[*b*]furan-5-yl-methanesulfonyl)-1-(4-methoxyphenyl)ethyl]-*N*-(2-methoxy-2-phenylethanoyloxy)-formamide (2mg) in methanol/MDC (2ml) was treated with potassium carbonate (0.1g). The title compound was thus generated chirally pure (100% ee) as shown by chiral HPLC.

### Example 28: (S)-N-[1-Benzo[b]thiophen-5-yl-methanesulfonyl)-2-propyl]-N-hydroxyformamide

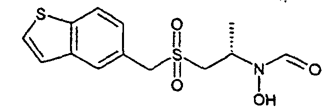

**Step 1: (S)-2-*N*-t-Butoxycarbonylaminopropyl thioacetate**-An ice-cold solution of triphenylphosphine (4.58g) in THF (80ml) was treated with di-t-butyl azodicarboxylate (4.02g) and stirred for 15 min. Thioacetic acid (1.24ml) and (S)-N-t-butoxycarbonylalaninol (1.53g) were added and the mixture allowed to gain room temperature. The solution was evaporated and the residue chromatographed (silica gel, step gradient: 3-50% EtOAc/hexane) to give the subtitle compound (2.5g) in approximately 50% purity.
**Step 2: (S)-1-(Benzo*[b]*thiophen-5-yl-methanesulfanyl)-2-(*N*-t-butoxycarbonylamino)propane-**A solution of (S)-2-N-t-butoxycarbonylaminopropyl thioacetate (2.5g) in methanol (30ml) at rt was treated dropwise with 1M sodium hydroxide solution (5.6ml) and stirred for 5 min. A solution of 5-bromomethylbenzo*[b]*thiophene (1.47g) in methanol (30ml) was added and the mixture stirred for 1h. This was then evaporated to low volume and diluted with EtOAc and water. The oganic layer was washed with water (twice), saturated brine, dried (MgSO₄), evaporated and the residue chromatographed (silica gel, step gradient 5-10% EtOAc/hexane) to give the subtitle compound (1.47g).
**Step 3: (S)-1-(Benzo*[b]*thiophen-5-yl-methanesulfonyl)-2-(*N*-t-butoxycarbonylamino)propane**-An ice-cold solution of (S)-1-(benzo*[b]*thiophen-5-ylmethanesulfanyl)-2-*N*-t-butoxycarbonylaminopropane (1.6g) in MDC (30ml) was treated with MCPBA (70%, 2.1g) and stirred for 2h. The mixture was then washed with saturated sodium hydrogen carbonate solution, water then saturated brine. The organic layer was dried (MgSO₄), evaporated and the residue chromatographed (silica gel, step gradient: 3-50% EtOAc/hexane) to give the subtitle compound (0.84g).
**Step 4: (S)-1-(Benzo*[b]*thiophen-5-yl-methanesulfonyl)-2-aminopropane -** A solution of (S)-1-(benzo*[b]*thiophen-5-yl-methanesulfonyl)-2-*N*-t-butoxycarbonylaminopropane (0.84g) in MDC (10ml) at rt was treated with trifluoroacetic acid (10ml). After 1h the solution was evaporated and the residue redissolved in MDC which was washed with saturated sodium hydrogen carbonate solution, water, saturated brine, dried (MgSO₄) and evaporated to give the subtitile compound (0.4g).
**Step 5: (S)-1-(Benzo*[b]*thiophen-5-yl-methanesulfonyl)-2-(4-methoxybenzylideneamino)propane -** A mixture of (S)-1-(benzo*[b]*thiophen-5-ylmethanesulfonyl)-2-aminopropane (0.4g) and 4-methoxybenzaldehyde (0.18ml) in benzene (20ml) was refluxed under Dean-Stark conditions for 2h, then cooled and evaporated to give the subtitle compound (0.575g).
**Step 6: (S)-2-[(Benzo*[b]*thiophen-5-yl-methanesulfonyl)-propan-2-yl]-3-(4-methoxyphenyl)oxaziridine-**An ice-cold solution of (S)-1-(benzo*[b]*thiophen-5-ylmethanesulfonyl)-2-(4-methoxybenzylideneamino)propane (0.575g) in MDC (15ml) was treated with MCPBA (70%, 0.321g). After 1.5h the mixture was filtered and evaporated. The residue was redissolved in EtOAc, washed with saturated sodium hydrogen carbonate solution, saturated brine, dried (MgSO₄)and evaporated. The residue was chromatographed (silica gel, step gradient: 3-50% EtOAc/hexane) to give the subtitle compound (0.274g).
Step 7: **(S)-1-(Benzo*[b]*thiophen-5-yl-methanesulphonyl)-2-hydroxyaminopropane** - A solution of (S)-2-[(benzo*[b]*thiophen-5-yl-methanesulfonyl)-propan-2-yl]-3-(4-methoxyphenyl)oxaziridine (0.264g) in MDC (5ml) at rt was treated with methanol (5ml) and 5M HCl (1ml), stirred for 1h and evaporated. The residue was chromatographed (silica gel, step gradient: 1-4% methanol/MDC) to give the subtitle compound (35mg).
**Step 8: (S)-*N*-[1-(Benzo*[b]*thiophen-5-yl-methanesulfonyl)-2-propyl]-*N*-hydroxyformamide** - A solution of (S)-1-(benzo*[b]*thiophen-5-yl-methanesulfonyl)-2-hydroxyaminopropane (33mg) in formic acid (3ml) acetic anhydride (1ml) was stood overnight at rt and evaporated, then re-evaporated from chloroform (twice). The residue was redissolved in methanol with stirring, and potassium carbonate (80mg) was added. After 3h the mixture was evaporated, treated with 2M HCl and extracted with MDC (twice). The combined extracts were dried (MgSO₄), evaporated and the residue chromatographed (acid washed silica gel, step gradient: 1-4% methanol/MDC) to give the title compound (21mg). MS (-ve ion electrospray) 314 (MH⁺); ¹H NMR δ [(CD₃)₂CO], 7.25-8.90 (7H, m), 4.25-4.97 (3H, m), 2.95-4.57 (2H, m), 1.25 (3H, m).

### Example 29: (S)-N-[1-Benzyloxy-3-(benzo[b]thiophen-5-yl-methanesulfonyl)-2-propyl]-N-hydroxyformamide

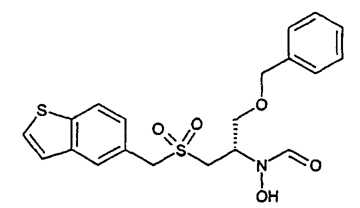

**Step 1: (S)-1-(Benzo*[b]*thiophen-5-yl-methanesulfonyl)-3-benzyloxy-2-(*N*- t-butoxycarbonylamino)propane ―** Prepared from (S)-3-benzyloxy-2-(*N*-t-butoxycarbonylamino)propanol using the method of Example 28 Steps 1-3.
**Step 2: (S)-1-(Benzo*[b]*thiophen-5-yl-methanesulfonyl)-3-benzyloxy-2-aminopropane hydrochloride -** A solution of (S)-1-(benzo*[b]*thiophen-5-yl-methanesulphonyl)-3-benzyloxy-2-(*N*-t-butoxycarbonylamino)propane (1.32g) in MDC (10ml) at rt was treated with 4M HCl in 1,4-dioxan (10ml). After 1h diethyl ether was added and the solid filtered off, washed with diethyl ether and dried to give the subtitle compound (1.11g).
**Step 3: (S)-1-(Benzo*[b]*thiophen-5-yl-methanesulfonyl)-3-benzyloxy-2-(*N*-cyanomethylamino)propane** -A stirred suspension of (S)-1-(benzo*[b]*thiophen-5-ylmethanesulfonyl)-3-benzyloxy-2-aminopropane hydrochloride (0.64g) in acetonitrile (10ml) at rt was treated with N,N-diisopropylethylamine (0.8ml) and bromoacetonitrile (0.12ml). The solution was refluxed for 16h, cooled and evaporated. The residue was treated with saturated sodium hydrogen carbonate solution and extracted with MDC (twice). The combined extracts were dried (MgSO₄) and evaporated. The residue was chromatographed (silica gel, step gradient: 5-50% EtOAc/MDC) to give the subtitle compound (0.64g).
**Step 4: (S)-*N*-[1-Benzyloxy-3-(benzo*[b]*thiophen-5-yl-methanesulfonyl)-2-propyl]hydroxylamine -** An ice-cold solution of (S)-1-(benzo*[b]*thiophen-5-ylmethanesulfonyl)-3-benzyloxy-2-(*N*-cyanomethylamino)propane (0.63g) in MDC (10ml) was treated with MCPBA (70%, 0.75g) and the stirred mixture allowed to warm to rt. After 30 min 10% sodium thiosulphate solution (10ml) and saturated sodium hydrogen carbonate solution (10ml) were added. After 10 minutes the aqueous phase was separated and extracted with MDC. The combined organic layers were dried (MgSO₄) and evaporated. The residue was redissolved in methanol (20ml) and hydroxylamine hydrochloride (2.1g) added. The mixture was stirred at 60°C for 1.5h cooled and evaporated. The residue was treated with saturated sodium hydrogen carbonate solution, extracted with MDC (twice), the combined extacts were dried (MgSO₄) and evaporated.The residue was chromatographed (silica gel, step gradient 5-50% EtOAc/MDC) to give the subtitle compound (0.4g).
**Step 5 : (S)-*N*-[1-Benzyloxy-3-(benzo*[b]*thiophen-5-yl-methanesulfonyl)-2-propyl]-*N*-hydroxyformamide** - (S)-*N*-[1-Benzyloxy-3-(benzo*[b]*thiophen-5-yl-methanesulfonyl)-2-propyl]hydroxylamine (0.39g) was converted to the title compound by the method described in Example 28 Step 8 (0,4g). MS (+ve ion electrospray) 420 (MH⁺, 32%), 237 (100%); ¹HNMR δ[(CD₃)₂CO], 8.45 and 8.95 (1H, 2s), 8.76 (1H, m), 7.22-8.11 (10H ,m), 4.45-5.32 (5H, m), 3.18-3.83 (4H, m).

### Example 30: (S)-N-[1-(Benzo[b]thiophen-5-yl-methanesulfonyl)-3-phenyl-2-propyl]-N-hydroxyformamide.

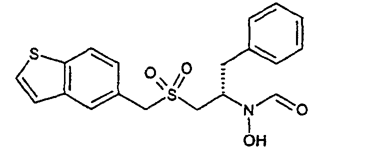

(S)-2-(*N*-t-Butoxycarbonylamino)-3-phenylpropanol was converted to the title compound by the methods described in Example 29. MS (+ve ion electrospray) 390 (MH⁺, 25%) 412 (MNa⁺, 45%) 147 (100%); ¹H NMR δ [(CD₃)₂CO], 8.38 and 9.05 (1H, 2s), 8.88 (1H, bs), 7.27-8.19 (10H, m), 4.45-4.80 and 5.32 (3H, 2m), 3.05-3.95 (4H, m).

### Example 31: (S)-N-[1-(Benzo[b]thiophen-5-yl-methanesulfonyl)-4,4-dimethyl-2-pentyl]-N-hydroxyformamide.

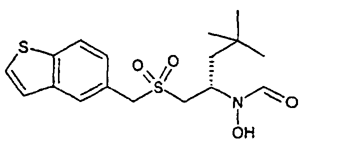

(S)-2-(*N*-t-Butoxycarbonylamino)-4,4-dimethylpentanol was converted to the title compound by the methods described in Example 29. MS (+ve ion electrospray) 370 (MH⁺,26%), 392 (MNa⁺,81%), 147 (100%); ¹H NMR δ [(CD₃)₂CO], 8.79 (1H, bs), 8.11 and 8.32 (1H, 2s), 7.62-8.07 (5H, m), 4.37-4.65 and 5.15 (3H, 2m), 3.05-3.73 (2H, m), 1.43 and 1.95 (2H, 2m), and 0.94 (9H, s).

### Example 32 : (S)-N-[1-(Benzo[b]thiophen-5-yl-methanesulfonyl)-3-methyl-2-butyl]-N-hydroxyformamide.

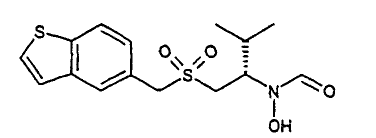

**Step 1 : 1-Bromo-3-methyl-butan-2-one-** Bromine (8.32g, 2.69ml) was added rapidly to a cooled solution (0°-5°C) of 3-methyl-2-butanone (4.5g) in anhydrous methanol (35ml). The temperature was allowed to rise to 10°C and maintained at 10°C for 2 h. Water was added (16ml), and the mixture stirred overnight at rt. The reaction mixture was diluted with water (16ml) and extracted with ether (3 x 100ml) The combined ether layers were then washed with 10% K₂CO₃ solution (100ml). The organic layer was dried (CaCl₂) and evaporated to give the subtitle compound (5.14g).
**Step 2 : 1-(Benzo*[b]*thiophen-5-yl-methanesulfanyl)-3-methyl-butan-2-one -** A solution of thioacetic acid *S*-benzo*[b]*thiophen-5-yl-methyl ester (6.91g) in methanol (100ml) was cooled in an ice bath and treated with 1N NaOH (31.1ml) followed immediately by 1-bromo-3-methyl-butane-2-one (5.15g). The reaction mixture was then allowed to stir at rt for 3 h. Methanol was removed by evaporation and the oily residue was partitioned between water (100ml) and ether (100ml). The organic layer was dried (MgSO₄) and evaporated. The crude reaction mixture was then chromatographed (silica gel, step gradient: 0-8% ether/petroleum ether) to give the subtitle compound as a white solid (5.58g).
**Step 3 : 1-(Benzo*[b]*thiophen-5-yl-methanesulfanyl)-3-methyl-butan-2-ol**-A solution of 1-(benzo*[b]*thiophen-5-yl-methanesulfanyl)-3-methyl-butane-2-one (2g) in THF (25ml) was treated dropwise with a 1M solution of borane in THF (7.6ml). The reaction mixture was stirred at rt overnight. Methanol (5ml) was added and the crude reaction mixture was chromatographed (silica gel, step gradient: 0-5% ether/petroleum ether) to give the subtitle compound as a white solid (1.62g).
**Step 4 :1-(Benzo*[b]*thiophen-5-yl-methanesulfonyl)-3-methyl-butan-2-ol-**A solution of 1-(benzo*[b]*thiophen-5-yl-methanesulfanyl)-3-methyl-butan-2-ol (2.3g) in dry MDC (30ml) was treated with MCPBA (100%, 0.34g) The reaction mixture was allowed to stir at rt overnight then diluted with EtOAc (50ml) and washed with saturated aqueous Na₂S₂O₃ followed by saturated aqueous sodium hydrogen carbonate The organic layer was dried (MgSO₄), evaporated and chromatographed (silica gel, step gradient: 5-70% ether/petroleum ether) to give the subtitle compound as a white solid (1.7g).
**Step 5 : (E)-[1-(Benzo*[b]*thiophen-5-yl-methanesulfonyl)-3-methylbutene**-A solution of 1-(benzo*[b]*thiophen-5-yl-methanesulfonyl)-3-methyl-butan-2-ol (0.3g) in anhydrous THF (30ml) was treated with triphenylphosphine (0.22g) and diethyl azodicarboxylate (0.145g, 0.13ml). The reaction mixture was allowed to stir at rt overnight. After being concentrated the crude reaction mixture was chromatographed (silica gel, step gradient: 3-30% ether/petroleum ether) to give the subtitle compound as an oil (0.52g).
**Step 6 : *N*-[1-(Benzo*[b]*thiophen-5-yl-methanesulfonyl)-3-methyl- 2-butyl]hydroxylamine-** A solution of (E)-[1-(benzo*[b]*thiophen-5-yl-methanesulfonyl)-3-methylbutene (0.52g) in anhydrous THF (15ml) was treated with hydroxylamine (50 wt% solution in water, 5ml) and allowed to stir at rt for 3h. Excess solvent was then removed by evaporation and the residue partitioned between EtOAc and water. The organic layer was dried (MgSO₄), evaporated and chromatographed (silica gel, step gradient: 3-100% ether/petroleum ether folowed by 1% methanol/ether) to give the subtitle compound as a white solid (50mg).
**Step 7 : (S)-*N*-[1-(Benzo*[b]*thiophen-5-yl-methanesulfonyl)-3-methyl-2-butyl]-*N*-hydroxyformamide -** *N-*[1-(Benzo*[b]*thiophen-5-yl-methanesulfonyl)-3-methyl-2-butyl]hydroxylamine (45mg) was treated with formic acid (0.75ml) and acetic anhydride (0.25ml) and left to stir at rt for 3h.The reaction mixture was stripped to dryness and taken up into methanol followed by the addition of K₂CO₃ (85mg). After stirring at rt for 3 h the reaction mixture was stripped to dryness, and then dissolved in water (20ml) and acidified with 1N HCl to pH 6. The precipitate which formed was extracted into EtOAc, dried (MgSO₄) and evaporated to give a crude racemic mixture which was separated into single enantiomers using chiral preparative HPLC (Chiralpak AD, isocratic, ethanol/hexane 50:50, 235nm). The slower running component was collected to give the title compound as a white solid (13.5mg), ee 94%. MS electrospray (+ve ion) 342.1(M+H⁺); ¹H NMR δ (MeOH-d₄), 8.34 (1H, s), 7.99 (1H, s), 7.94 (2H, d, J=8.4Hz), 7.63 (1H, d, J=5.6Hz), 7.43 (1H, d, J=8.4Hz), 7.40 (1H, d, J=5.6Hz), 4.59-4.47(2H, m), 3.90-3.85 (1H, m), 3.69-3.63 (1H, m),3.53-3.52 (1H, m), 2.0-1.96 (1H, m) and 0.97-0.87(6H, m).

### Example 33 : N-[1-(Benzo[b]thiophen-5-yl-methanesulfonyl)-4-methyl-2-pentyl]-N-hydroxyformamide

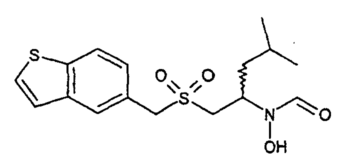

**Step 1 : 1-Bromo-4-methyl-pentan-2-one -** Bromine (3.03g, 0.978ml) was added rapidly to a cooled solution (0°-5°C) of 4-methyl-2-pentanone (2g) in anhydrous methanol (5ml). The temperature was allowed to rise to 10°C and maintained at 10°C for 30min, and then at rt for 15min. After the addition of water (30ml) the reaction mixture was extracted with ether (3 x 25ml). The combined ether layers were then washed with saturated NaHCO₃ solution and the organic layer was dried (MgSO₄) and evaporated to give the subtitle compound (3g).
**Step 2 : 1-(Benzo*[b]*thiophen-5-yl-methanesulfanyl)-4-methylpentan-2-one**-A solution of thioacetic acid *S*-benzo*[b]*thiophen-5-yl-methyl ester (2.5g) in methanol (40ml) was cooled in an ice bath and treated with 1N NaOH (31.1ml) followed by 1-bromo-4-methyl-pentan-2-one (2.01g). The reaction mixture was then allowed to stir at rt for 3h. Methanol was removed by evaporation and the oily residue was partitioned between water (100ml) and ether (100ml). The organic layer was dried (MgSO₄) and chromatographed (silica gel, step gradient: 0-4% ether/petroleum ether) to afford the subtitle compound (2.98g).
**Step 3 : 1-(Benzo*[b]*thiophen-5-yl-methanesulfanyl)-4-methylpentan-2-one oxime-** A solution of 1-(benzo*[b]*thiophen-5-yl-methanesulfanyl)-4-methyl-pentan-2-one (0.32g) in toluene (5ml) was treated with NaOAc.3H₂O (0.31g) and NH₂OH.HCl (0.16g) and allowed to reflux for 3h. The reaction mixture was then stripped to dryness and the crude products partitioned between water and EtOAc. The organic layer was dried (MgSO₄), concentrated and chromatographed (silica gel, step gradient 2-30% ether/petroleum ether) to give the subtitle compound as a white solid (0.3g).
**Step 4 : *N*-[1-(Benzo*[b]*thiophen-5-yl-methanesulfanyl)-4-methyl-2-pentyl]hydroxylamine-** A solution of 1-(benzo*[b]*thiophen-5-yl-methanesulfanyl)-4-methyl-pentan-2-one oxime (0.21g) in acetic acid/methanol (1:1, 4ml) was treated with NaBH₃CN (0.2g). The reaction mixture was then allowed to stir at rt overnight. The reaction mixture was then stripped to dryness and chromatographed (silica gel, step gradient: 5-70% ether/pet-ether) to afford the subtitle compound (0.18g).
**Step 5 : *N*-[1-(Benzo*[b]*thiophen-5-yl-methanesulfanyl)-4-methyl-2-pentyl]-*N*-hydroxyformamide -** *N*-[1-(Benzo*[b]*thiophen-5-yl-methanesulfanyl)-4-methyl-2-pentyl]hydroxylamine (0.13g) was treated with formic acid (2.1ml) and acetic anhydride (0.9ml) and left to stir at rt for 3h. The reaction mixture was stripped to dryness and taken up into methanol (3ml) followed by the addition of K₂CO₃ (90mg). After stirring at rt for 1h the reaction mixture was stripped to dryness and then dissolved in water (20ml) and acidified with 1N HCl to pH 6. This was extracted with EtOAc, dried (MgSO₄) and evaporated to give the subtitle compound as an oil (0.1g).
**Step 6: *N*-[1-(Benzo*[b]*thiophen-5-yl-methanesulfonyl)-4-methyl-2-pentyl]-*N*-hydroxyformamide** - A solution of *N*-[1-(benzo*[b]*thiophen-5-yl-methanesulfanyl)-4-methyl-2-pentyl)-*N*-hydroxyformamide (0.09g) in dry MDC (6ml) was treated with MCPBA (70%, 0.13g). The reaction mixture was allowed to stir at rt for 2h and then diluted with EtOAc (50ml) and washed with saturated aqueous Na₂S₂O₃ followed by saturated sodium hydrogen carbonate solution. The organic layer was dried (MgSO₄), evaporated and chromatographed (silica gel, step gradient 5-100% ether/petroleum ether folowed by 1-5% methanol/ether) and the isolated product was further purified by preparative HPLC to afford the title compound as a solid (3.8mg). MS electrospray (+ve ion) 378.3 (M+H⁺), 394 (MH⁺ + NH₃); ¹H NMR δ (MeOH-d₄), 8.31 (1H, s), 7.96-7.92 (2H, m), 7.64-7.61 (1H, m ), 7.45-7.38 (2H, m), 4.80-4.50 (2H, m), 3.63-3.53 (1H, m), 3.33-3.30 (1H, m), 1.79-1.71 (1H, m), 1.52-1.49 (1H, m), 1.28-1.19 (1H, m) , 0.91-0.89 (3H, d, J=10Hz) and 0.82-0.79 (3H, d, J=10Hz).

### Example 34: (S)-N-[2-(Benzo[b]thiophen-5-ylmethanesulfonyl)-1-(5-methyl-isoxazol-3-yl)-ethyl]-N-hydroxyformamide

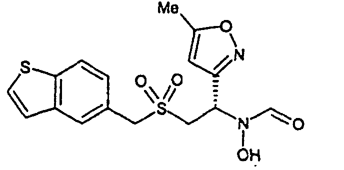

2-Bromo-1-(5-methyl-isoxazol-3-yl)-ethanone was prepared from 5-methylisoxazol-3-carboxylic acid in a similar manner to Example 19 steps 1 and 2, and then converted into the title compound using the procedures described in Example 6 steps 1-6. MS electrospray (+ve ion) 381 (MH⁺), MS electrospray (-ve ion) 379 (M-H⁻), ¹H NMR δ (CD₃OD): 8.47-7.38 (6H, aromatics+CHO rotamers), 6.17-6.11 and 5.65-5.48 (2H, 2 x m), 4.58 (2H, m), 4.03-3.69 (2H, m), 2.41 (3H, s).

### Example 35: (S)-N-[1-(4-Acetamidophenyl)-2-(benzo[b]thiophen-5-ylmethanesulfonyl)ethyl]-N-hydroxyformamide

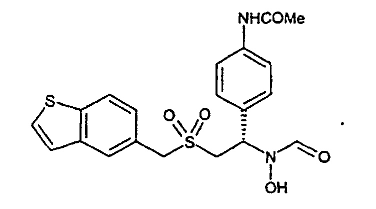

**Step 1: 1-(4-Acetamidophenyl)-2-bromoethanone -** 4-Acetamidoacetophenone (2.00g) and copper bromide (4.28g) were heated to reflux in ethyl acetate (30ml) for 2.5h. After cooling, the reaction mixture was filtered through activated charcoal, then evaporated, redissolved in ethyl acetate (18ml) and refiltered through silica. The filtrate was evaporated to give the subtitle compound as an oil (0.32g).
**Step 2: (S)-*N*-[1-(4-Acetamidophenyl)-2-(benzo*[b]*thiophen-5-ylmethanesulfonyl)ethyl]-*N*-hydroxyformamide -** 1-(4-Acetamidophenyl)-2-bromoethanone was converted into the title compound using the procedures described in Example 6, steps 1-6. The racemic product was separated into component isomers by preparative chiral HPLC (Chiralpak AD 250mm x 20mm i.d., ethanol). Collection of the slower eluting component afforded the title compound as a white solid (0.019g). MS electrospray (+ve ion) 433.2 (MH⁺), 455.2 (MH⁺ + Na), MS electrospray (-ve ion) 431.1 (M-H⁻). ¹H NMR δ(DMSO-d6) : 9.97 plus 9.76 (1H, s x2, rotamers), 8.25 - 7.34 (10H, m, aromatics plus CHO rotamers), 6.87 plus 5.47 (1H, s x2, rotamers), 4.09 (2H, m), 3.99 - 3.61 (2H, m, rotamers), 2.03 (3H, s). Chiral purity: 96.3%ee.

### Example 36: (S)-N-[2-(Benzo[b]thiophen-5-yl-methanesulfonyl)-1-(5-carboxythiophen-2-yl)ethyl]-N-hydroxyformamide.

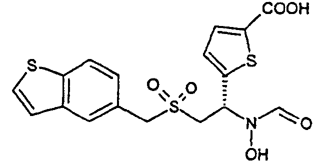

A solution of methyl 5-acetylthiophen-2-carboxylate (2.68g) in carbon tetrachloride (20ml), containing a catalytic amount of iron filings, was stirred at 60°C and treated dropwise with a solution of bromine (0.75ml) in methanol (10ml) added over 20min. After being stirred for a further 3h, the volatile components were removed under reduced pressure to afford 2-bromo-1-(5-methoxycarbonylthiophen-2-yl)-ethanone as a dark yellow solid which was converted into the methyl ester of the title compound using the procedures described in Example 6 steps 1-6. A solution of this ester (47 mg) in THF (0.5 ml) was treated with a solution of sodium sulphide nonahydrate (77 mg, 3 equiv.) in water (2 ml) and stirred under argon at rt for 2h. The reaction mixture was diluted with EtOAc and acidified with 2M HCl solution. The organic layer was washed with water, dried and evaporated to afford the title compound as a crisp foam (36 mg). MS electrospray (-ve ion) 424 (M-H⁻); 380, 319. ¹H NMR δ(CD₃OD) : 8.27 (1H, s), 8.00 - 7.3 (6H, aromatics), 7.15 (1H, s), 6.26 and 5.83 (1H, br d, rotamers), 4.56 (2H, s), 4.1 and 3.9 (2H, m).

### Example 37: (S)-N-[2-(Indan-2-ylmethanesulfonyl)-1-(5-methoxycarbonylthiophen-2-yl)ethyl]-N-hydroxyformamide.

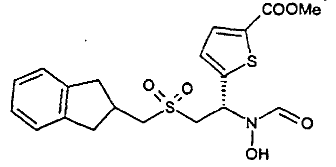

2-Acetylthiomethylindane was reacted with 2-bromo-1-(5-methoxycarbonylthiophen-2-yl)-ethanone (prepared as in Example 36) following the methods described in Example 6 steps 1-6 to afford the title compound as a pale solid (24 mg). MS electrospray (-ve ion) 422 (M-H⁻); 845 (2M-H). ¹H NMR δ(CDCl₃) : 8.17 and 8.42 (1H, s), 7.68 (1H, d, J=2Hz), 7.18 (4H, overlapping), 7.10 (1H, d, J=2Hz), 6.25 and 5.72 (1H, d, rotamers), 3.88 (3H, s), 3.2 (6H, overlapping m), 2.85 (3H, overlapping m).

### Example 38: (S)-N-[2-(Indan-2-ylmethanesulfonyl)-1-(3-benzyloxycarbonylaminophenyl)ethyl]-N-hydroxyformamide.

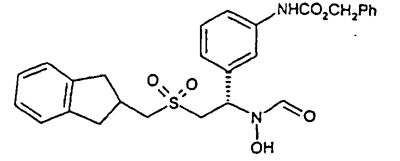

3-Benzyloxycarbonylamino-benzoic acid was prepared using the method described in *Synthesis,* 1974, 44, and converted into 2-bromo-1-(3-benzyloxycarbonylaminophenyl)-ethanone according to the procedures of Example 19, steps 1 and 2. Following the procedures of Example 6, steps 1-6, 2-bromo-1-(3-benzyloxycarbonylaminophenyl)ethanone and 2-acetylthiomethylindane afforded the title compound as a white solid. MS electrospray (+ve ion) 531 (MNa⁺); 509 (MH⁺). ¹H NMR δ(DMSO-d6) : 9.82 (1H, s), 8.30 (1H, s), 7.58 (1H, br s), 7.1-7.4 (13H), 5.84 and 5.49 (1H, br s, rotamers), 5.15 (2H, s), 4.02 (1H, br m), 3.66 (1H, br m), 3.3 (2H, obscured), 3.11 (2H, overlapping m), 2.89 (1H, m), 2.73 (2H, overlapping m).

### Example 39: (S)-N-[2-(Indan-2-ylmethanesulfonyl)-1-(3-aminophenyl)ethyl]-N- hydroxyformamide.

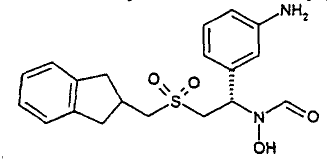

A solution of (S)-*N*-[2-(indan-2-yl-methanesulfonyl)-1-(3-benzyloxycarbonylaminophenyl)ethyl]-*N*-hydroxyformamide (40 mg) in methanol (10 ml) was treated with 5% palladium on charcoal and hydrogenated at ambient temperature and pressure for 4h. The catalyst was then filtered off and replaced with fresh catalyst and hydrogenated overnight. The catalyst was again removed by filtration through celite and the filtrate evaporated to afford the title compound as a yellow solid. ¹H NMR δ(CDCl₃) : 8.14 and 8.42 (1H, s), 7.1 (5H), 6.70 (2H, m), 6.59 (1H, m), 5.33 and 5.90 (1H, br m, rotamers), 4.11 (1H, br m), 3.90 (1H, br m), 3.2 (5H, overlapping m), 2.82 (2H, overlapping m).

### Example 40: (S)-N-[2-(Benzo[b]thiophen-5-ylmethanesulfonyl)-1-(4-ethoxycarbonylphenyl)ethyl]-N-hydroxyformamide.

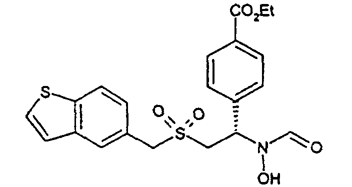

A stirred solution of ethyl 4-acetylbenzoate (1.92g) in carbon tetrachloride (20 ml) was treated with bromine (0.51ml) and heated to 40°C until the reaction initiated. Heating was then removed and the mixture was stirred for a further 1h. Removal of the solvent afforded a crude product which was chromatographed (silica gel, 10% EtOAc/hexane) to give ethyl 4-(2-bromoacetyl)-benzoate as a white solid (2.04g). Following the procedures described in Example 6, steps 1-6, ethyl 4-(2-bromoacetyl)-benzoate was converted into the title compound which was obtained as a white solid. MS electrospray (+ve ion) 470 (MNa⁺); (-ve ion) 446 (M-H). ¹H NMR δ(DMSO d6): 9.92 (1H, br s), 8.31 (1H, s), 8.04 (1H, d, J=8.3 Hz), 7.95 (2H, d, J=8.3Hz), 7.88 (1H, s), 7.82 (1H, d, J=5.5Hz), 7.58 (2H, br d), 7.49 (1H, d, J=5.5Hz), 7.37 (1H, dd, J=8.3 and 1.5Hz), 5.68 and 5.98 (1H, br m, rotamers), 4.66 (2H, s), 4.32 (2H, q), 4.03 (1H, br d), 3.74 (1H, br d), 1.32 (3H, t).

### Example 41: (S)-N-[2-(Benzo[b]thiophen-5-ylmethanesulfonyl)-1-(4-carboxyphenyl)ethyl]-N-hydroxyformamide.

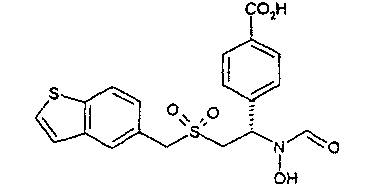

(S)-*N*-[2-(Benzo*[b]*thiophen-5-yl-methanesulfonyl)-1-(4-ethoxycarbonylphenyl)ethyl]-*N*-hydroxyformamide (Example 40) was treated with sodium sulphide following the procedure described for Example 36 to give the title compound as a white solid. MS electrospray (-ve ion) 418 (M-H), 357. ¹H NMR δ(DMSO-d6, 353°K) : 8.27 (1H, s), 8.02 (1H, d, J=8.0Hz), 7.91 (2H, d, J=8.3Hz), 7.88 (1H, s), 7.75 (1H, d, J=5.5Hz), 7.51 (2H, d, J=8.3Hz), 7.44 (1H, d, J=5.5Hz), 7.35 (1H, dd, J=8.0 and 1.0Hz), 5.75 (1H, br m), 4.61 (2H, s), 4.00 (1H, dd, J=14.8 and 8.3Hz), 3.72 (1H, dd, J= 14.8 and 4.6Hz).

### Example 42: (S)-N-[2-(3-Quinolylmethanesulfonyl)-1-(4- ethoxycarbonylphenyl)ethyl]-N-hydroxyformamide.

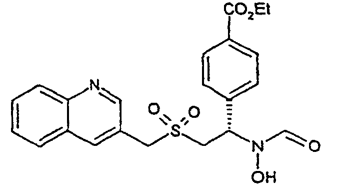

Ethyl 4-(2-bromoacetyl)benzoate, prepared as described in Example 40, and 3-acetylthiomethylquinoline were converted into the title compound according to the methods described in Example 6 steps 1-6, except that after reduction with borane dimethyl sulfide as described in step 2, the reaction was quenched with 2M HCl and left stirring for 1h. The title compound was obtained as a pale crisp foam. MS electrospray (+ve ion) 443 (MH⁺). ¹H NMR δ(CDCl₃) : 9.27 (1H, br s), 8.73 (1H, s), 8.35 and 8.50 (1H, s), 8.27 (2H, d), 8.00 (3H, overlapping m), 7.79 (1H, dd), 7.46 (2H, d), 5.57 and 6.18 (1H, br), 4.73 (2H, s), 4.35 (1H, obscured), 4.35 (2H, q), 3.43 and 3.59 (1H, br d), 1.37 (3H, t).

### Example 43: (S)-N-[2-(3-Quinolinylmethanesulfonyl)-1-(4-carboxyphenyl)ethyl]-N- hydroxyformamide.

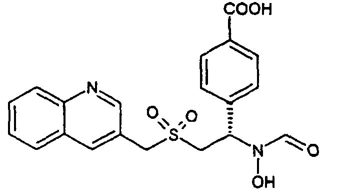

The title compound was obtained as a white solid upon treatment of (S)-*N*-[2-(3-quinolylmethanesulfonyl)-1-(4-ethoxycarbonylphenyl)ethyl]-*N*-hydroxyformamide (Example 42) with aqueous sodium sulphide using a similar procedure to that described in Example 36, except that phosphate buffer (pH = 6.0) was used in place of 2M HCl to acidify the reaction mixture. MS electrospray (+ve ion) 415 (MH⁺), (-ve ion) 413 (M-H), 352. ¹H NMR δ(DMSO-d6, 353°K) : 8.91 (1H, d, J=2Hz), 8.42 (1H, d, J=2Hz), 8.30 (1H, s), 8.07 (1H, d, J=8.0Hz), 8.00 (1H, d, J=8.0Hz), 7.92 (2H, d, J=8.4Hz), 7.82 (1H, dd, J=8.0 and 8.0Hz), 7.66 (1H, dd, J=8.0 and 8.0Hz), 7.54 (2H, d, J=8.4Hz), 5.81 (1H, br s), 4.79 (2H, s), 4.11 (1H, dd, J=14.7 and 8.3Hz), 3.84 (1H, dd, J= 14.7 and 4.4Hz).

### Example 44: (S)-N-[1-Phenyl-2-(benzo[b]furan-6-ylmethanesulfonyl)ethyl]-N-hydroxyformamide

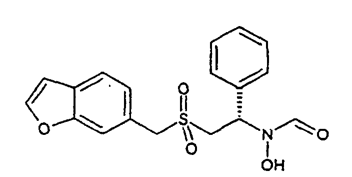

The title compound was prepared from 6-bromomethylbenzo*[b]*furan using the method of Example 24. Final purification was by preparative HPLC to remove the 4-benzofuranyl isomer. MS (+ve ion electrospray) 741 (M₂Na⁺,18%), and 131 (100%); ¹H NMR δ(CD₃OD): 8.3 (1H, s), 7.9 (1H, d, J=2Hz), 7.3-7.6 (8H, m), 6.9 (1H, d, J=2Hz), 5.4 and 6.0 (1H, 2m), 4.5 (2H, s), and 3.6 and 4.1 (2H, 2m).

### Example 45: (S)-N-[1-Phenyl-2-(2-naphthylmethanesulfonyl)ethyl]-N-hydroxyformamide

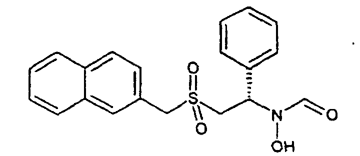

The title compound was prepared from 2-acetylthiomethylnaphthalene using the method of Example 26. MS (+ve ion electrospray) 370 (MH⁺, 32%), and 433 (100%); ¹H NMR δ(CD₃OD): 8.3 (1H, s), 7.8-8.0 (4H, m), 7.3-7.6 (8H, m), 5.4 and 6.0 (1H, 2m), 4.6 (2H, s), and 3.6 and 4.1 (2H, 2m).

### Example 46: (S)-N-[1-Phenyl-2-(benzothiazol-6-ylmethanesulfonyl)ethyl]-N-hydroxyformamide

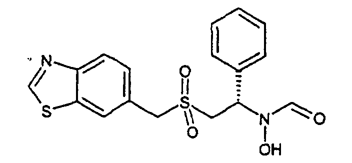

The title compound was prepared from 6-chloromethylbenzothiazole using the method of Example 24. MS (+ve ion electrospray) 377 (MH⁺,38%), and 148 (100%); ¹H NMR δ(CDCl₃): 9.0 (1H, bs), 8.0-8.5 (3H, m), 7.3-7.7 (6H, m), 5.4 and 6.0 (1H, 2m), and 3.2 - 4.6 (4H, m).

### Example 47: (S)-N-[1-Phenyl-2-(benzo[b]furan-2-ylmethanesulfonyl)ethyl]-N-hydroxyformamide

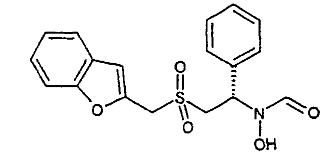

The title compound was prepared from 2-bromomethylbenzo*[b]*furan (J.H. Musser et al., *J.Med.Chem.,* 1987, 30, 400) using the method of Example 24. MS (+ve ion electrospray) 741 (M₂Na⁺,15%), and 131 (100%); ¹H NMR δ(CD₃OD): 8.3 (1H, s), 7.2-7.7 (9H, m), 6.9 (1H, s), 5.5 and 6.1 (1H, 2m), 4.7 (2H, s), and 3.7 and 4.2 (2H, 2m).

### Example 48: (S)-N-[1-Phenyl-2-(5-thieno[2,3-b]pyridylmethanesulfonyl)ethyl]-N-hydroxyformamide

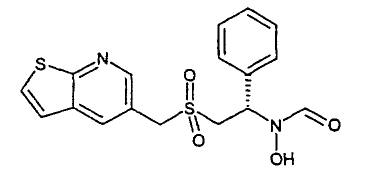

**Step 1: (S)-1-Phenyl-2-(5-thieno*[2,3-b]*pyridylmethanesulfanyl)ethanol and (R)-2-phenyl-2-(5-thieno*[2,3-b]*pyridylmethanesulfanyl)ethanol** - To 5-acetylthiomethylthieno*[2,3-b]*pyridine (0.9g) in methanol (10ml) was added aqueous sodium hydroxide (2.1ml, 2M). After 15min (S)-phenyloxirane (0.5ml) was added, and after another 15min the solution was partitioned between aqueous ammonium chloride and diethyl ether. The organic layer was dried (MgSO₄) and evaporated and the residue chromatographed (silica gel, step gradient: 25-100% ethyl acetate/hexane) to give a 1:1 mixture of the two subtitle compounds (0.82g). ¹H NMR δ(CDCl₃) 8.4 and 8.5 (1H, 2d, J=2Hz), 7.9 and 8.0 (1H, 2d, J=2Hz), 7.5 (1H, m), 7.2-7.3 (6H, m), 4.8 (0.5H, m), 3.7-3.9 (4H, m), and 2.7 (1.5H, m).
**Step 2: (S)-N-[2-(5-Thieno*[2,3-b]*pyridylmethanesulfanyl)-1-phenylethyl]-N,O-bis-tert-butoxycarbonylhydroxylamine -** The mixture of (S)-1-phenyl-2-(5-thieno*[2,3-b]*pyridylmethanesulfanyl)ethanol and (R)-2-phenyl-2-(5-thieno*[2,3-b]*pyridylmethanesulfanyl)ethanol (0.82g) in toluene (15ml) under argon was treated with N,O-bis-tert-butoxycarbonyl-hydroxylamine (1.0g) and 1,1'-azobis(N,N-dimethylformamide) (0.9g). After cooling to 0°C tributylphosphine (1.3ml) was added dropwise and the reaction then allowed to warm to rt. After 1.5h the reaction was evaporated and the residue chromatographed (silica gel, step gradient: 25-50% ethyl acetate/hexane) to give the subtitle compound as a gum (1.3g). MS (+ve ion electrospray) 517 (MH⁺, 15%), and 284 (100%).
**Step 3: (S)-N-[2-(5-Thieno*[2,3-b]*pyridylmethanesulfonyl)-1-phenylethyl]-N,O-bis-tert-butoxycarbonyl-hydroxylamine -** (S)-N-[2-(5-Thieno*[2,3-b]*pyridyl methanesulfanyl)-1-phenylethyl]-N,O-bis-tert-butoxycarbonylhydroxylamine (1.3g) in dichloromethane (20ml) at 0°C was treated with MCPBA (65% purity, 1.3g). After 30min the reaction was washed with saturated sodium hydrogen carbonate solution containing 1% sodium sulfite. Evaporation of the organic layer and chromatography (silica gel, step gradient, 25-100% ethyl acetate/hexane) gave the subtitle compound as a foam (0.42g). MS (+ve ion electrospray) 349 (MH⁺-2Boc, 57%), and 393 (100%).
**Step 4: (S)-*N*-[1-Phenyl-2-(5-thieno*[2,3-b]*pyridylmethanesulfonyl)ethyl]-*N*-hydroxyformamide -** A solution of (S)-N-[2-(5-thieno*[2,3-b]*pyridylmethanesulfonyl)-1-phenylethyl]-N,O-bis-tert-butoxycarbonyl-hydroxylamine (0.42g) in dichoromethane (8ml) was treated with trifluoroacetic acid (3ml). After 30min the solution was evaporated and redissolved in formic acid (10ml) and acetic anhydride (3ml) was added. After a further 1.5h the solution was again evaporated and redissolved in methanol (10ml). Potassium carbonate (0.3g) was then added and after 15min water was added and the pH adjusted to 7 with hydrochloric acid. Extraction with ethyl acetate and chromatography (silica gel, step gradient: 50-100% ethyl acetate/hexane) gave the title compound as a solid after trituration with ether (90mg). MS (+ve ion electrospray) 377 (MH⁺, 55%) and 148(100%); ¹H NMR δ(CD₃OD), 8.6 (1H, m), 8.3 (2H, m), 7.8 (1H, d, J=6Hz), 7.4-7.6 (6H, m), 5.6 and 6.1 (1H, 2m), 4.7 (2H, s), and 3.7 and 4.2 (2H, 2m).

### Example 49: (S)-N-[1-Phenyl-2-(6-thieno[3,2-b]pyridylmethanesulfonyl)ethyl]-N-hydroxyformamide

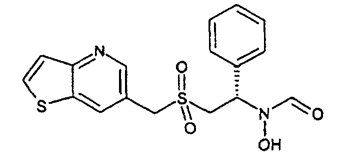

**Step 1: (S)-1-Phenyl-2-(6-thieno*[3,2-b]*pyridylmethanesulfanyl)ethanol and (R)-2-phenyl-2-(6-thieno*[3,2-b]*pyridylmethanesulfanyl)ethanol -** To 6-acetylthiomethylthieno*[3,2-b]*pyridine (0.7g) in methanol (10ml) was added aqueous sodium hydroxide (1.6ml, 2M). After 15min at rt (S)-phenyloxirane (0.4ml) was added, and after another 15min the solution was partitioned between aqueous saturated ammonium chloride and diethyl ether. The organic layer was dried (MgSO₄) and evaporated and the residue chromatographed (silica gel, step gradient: 25-100% ethyl acetate/hexane) to give a 1:1 mixture of the two subtitle compounds (0.53g). ¹H NMR δ(CDCl₃) 8.5 and 8.6 (1H, 2d, J=2Hz), 8.0 and 8.1 (1H, 2d, J=2Hz), 7.7 (1H, m), 7.5 (1H, m), 7.2-7.3 (5H, m), 4.8 (0.5H, m), 3.7-3.9 (3.5H, m), and 2.7 (1.5H, m).
**Step 2: (S)-N-[2-(6-Thieno*[3,2-b]*pyridylmethanesulfanyl)-1-phenylethyl]-N,O-bis-tert-butoxycarbonylhydroxylamine -** The mixture of (S)-1-phenyl-2-(6-thieno*[3,2-b]*pyridylmethanesulfanyl)ethanol and (R)-2-phenyl-2-(6-thieno*[3,2-b]*pyridylmethanesulfanyl)ethanol (0.53g) in toluene (10ml) under argon was treated with N,O-bis-tert-butoxycarbonyl-hydroxylamine (0.65g) and 1,1'-azobis(N,N-dimethylformamide) (0.6g). After cooling to 0°C tributylphosphine (0.85ml) was added dropwise and the reaction then allowed to warm to rt. After 1.5h the reaction was evaporated and the residue chromatographed (silica gel, step gradient: 25-75% ethyl acetate/hexane) to give the subtitle compound as a gum (0.76g). MS (+ve ion electrospray) 517 (MH⁺, 25%), and 148 (100%).
**Step 3: (S)-N-[2-(6-Thieno*[3,2-b]*pyridylmethaneulfonyl)-1-phenylethyl]-N,O-bis-tert-butoxycarbonyl-hydroxylamine -** (S)-N-[2-(6-Thieno*[3,2-b]*pyridylmethanesulfanyl)-1-phenylethyl]-N,O-bis-tert-butoxycarbonylhydroxylamine (0.76g) in dichloromethane (20ml) at 0°C was treated with MCPBA (65% purity, 0.75g). After 30min the reaction was washed with saturated sodium hydrogen carbonate solution containing 1% sodium sulfite. Evaporation of the organic layer and chromatography (silica gel, step gradient: 50-100% ethyl acetate/hexane) gave the subtitle compound as a foam (0.42g). MS (+ve ion electrospray) 349 (MH⁺-2Boc, 57%), and 393 (100%).
**Step 4: (S)-*N*-[1-Phenyl-2-(6-thieno*[3,2-b]*pyridylmethanesulfonyl)ethyl]-*N*-hydroxyformamide -** A solution of (S)-N-[2-(6-thieno*[3,2-b]*pyridylmethanesulfonyl)-1-phenylethyl]-N,O-bis-tert-butoxycarbonyl-hydroxylamine (0.42g) in dichoromethane (8ml) was treated with trifluoroacetic acid (3ml). After 30min the solution was evaporated and redissolved in formic acid (10ml) and acetic anhydride (3ml) was added. After a further 1.5h the solution was again evaporated and redissolved in methanol (10ml). Potassium carbonate (0.3g) was then added and after 15min water was added and the pH adjusted to 7 with hydrochloric acid. Extraction with ethyl acetate and chromatography (silica gel, step gradient: 0-5% methanol/ethyl acetate) gave the title compound as a solid after trituration with ether (65mg). MS (+ve ion electrospray) 377 (MH⁺, 58%) and 148 (100%); ¹H NMR δ(CD₃OD): 8.6 (1H, m), 8.5 (1H, m), 8.3 (1H, m), 8.1 (1H, d, J=6Hz), 7.5 (1H, d, J=6Hz), 7.4-7.6 (5H, m), 5.6 and 6.1 (1H, 2m), 4.6 (2H, s), and 3.7 and 4.2 (2H, 2m).

### Example 50: (S)-N-[1-(4-Methoxyphenyl)-2-(3-quinolylmethanesulfonyl)ethyl]-N-hydroxyformamide

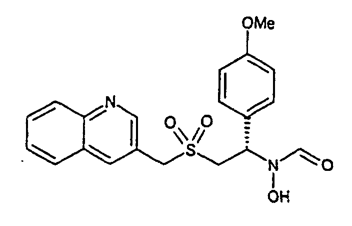

**Step 1: (S)-2-Bromo-1-(4-methoxyphenyl)ethanol -** (S)-2-Methyl-CBS-oxazaborolidine (50ml of a 1M solution in toluene) and borane dimethyl sulfide complex (11ml of a 2M solution in toluene) were mixed together under argon for 15 min. The reaction was then cooled to -30°C and 2-bromo-1-(4-methoxyphenyl)ethanone (11.45g) in toluene (30ml) was added dropwise over *circa* 20 min to maintain the reaction temperature at < -20°C. After 1h methanol (30ml) was added carefully, with consequential hydrogen evolution. The reaction was allowed to warm to rt, evaporated and then re-evaporated from toluene (30ml). The residue was diluted with EtOAc and washed with dilute HCl and saturated sodium hydrogen carbonate solution. Evaporation of the EtOAc and chromatography (silica gel, step gradient: 5-45% EtOAc/hexane) gave the subtitle product (10.61 g). ¹H NMR δ(CDCl₃), 7.24 (2H, d, J=8.2Hz), 6.9 (2H, d, J=8.2Hz), 4.9 (1H, m), 3.8 (3H, s), 3.58-3.5 (2H, m).
**Step 2: (S)-1-(4-Methoxyphenyl)-2-(3-quinolylmethanesulfanyl)ethanol and (R)-2-(4-methoxyphenyl)-2-(3-quinolylmethanesulfanyl)ethanol -** To 3-acetylthiomethylquinoline (1g) in methanol (10ml) was added sodium methoxide (0.25g). After 15min at rt (S)-2-bromo-1-(4-methoxyphenyl)ethanol (1g) in methanol (10ml) was added, and after 6h the solution was partitioned between aqueous saturated sodium hydrogen carbonate and EtOAc. The organic layer was washed with saturated brine solution, separated, and dried (MgSO₄) prior to evaporation and chromatography (silica gel, step gradient: 20-100% ether/petroleum ether followed by EtOAc/petroleum ether 50-80%) to give the subtitle compounds - (S)-1-(4-methoxyphenyl)-2-(3-quinolylmethanesulfanyl)ethanol (1.05g) [¹H NMR δ(CDCl₃), 8.84 (1H, d, J=2Hz), 8.08 (1H, d, J=8.8Hz), 8 (1H, d, J=2Hz), 7.77 (1H, dd, J=8 and 1.2Hz), 7.68 (1H, m), 7.54 (1H, m), 7.23 (2H, d, J=6.4Hz), 6.86 (2H, d, J=6.4Hz), 4.75 (1H, m), 3.86 (2H, m), 3.78 (3H, s) 2.9 (1H, brs) 2.75 (2H, m)], and (R)-2-(4-methoxyphenyl)-2-(3-quinolylmethanesulfanyl)ethanol (0.326g) [¹H NMR δ(CDCl₃), 8.71 (1H, d, J=1.9Hz), 8.04 (1H, d, J=8.4Hz), 7.87 (1H, d, J=1.9Hz), 7.73-7.5 (3H, m), 7.21 (2H, d, J=6.7Hz), 6.82 (2H, d, J=6.7Hz), 3.86-3.6 (8H, m) and 2.82 (1H, brs)].
**Step 3: (S)-*N*-[1-(4-Methoxyphenyl)-2-(3-quinolylmethanesulfanyl)ethyl]-N,O-bis-tert-butoxycarbonyl-hydroxylamine -** (S)-1-(4-Methoxyphenyl)-2-(3-quinolylmethanesulfanyl)ethanol (1g) and (R)-2-(4-methoxyphenyl)-2-(3-quinolylmethanesulfanyl)ethanol (0.3g) were combined in THF/toluene (20ml, 1:1) under argon and treated with N,O-bis-tert-butoxycarbonylhydroxylamine (2.8g) and 1,1'-azobis(N,N-dimethylformamide) (1.72g). After cooling to 0°C tributylphosphine (2.46ml) was added rapidly *via* syringe and the reaction then allowed to warm to rt. After 4h the reaction was quenched with water (10ml) diluted with EtOAc and washed with brine. After filtering through a short plug of silica gel the EtOAc layer was evaporated and the residue chromatographed (silica gel, step gradient: 20-100% ether/petroleum ether) to give the subtitle compound as a foam (1.045g).
**Step 4: (S)-*N*-[1-(4-Methoxyphenyl)-2-(3-quinolylmethanesulfonyl)ethyl]-N,O-bis-tert-butoxycarbonyl-hydroxylamine -** (S)-*N*-[1-(4-Methoxyphenyl)-2-(3-quinolylmethanesulfanyl)ethyl]-N,O-bis-tert-butoxycarbonylhydroxylamine (1.045g) in EtOAc (15ml) at 0°C was treated with MCPBA (65% purity, 1.026g). After 15min the reaction was quenched with dimethylsulfide (2ml), diluted with EtOAc and washed with saturated sodium hydrogen carbonate solution. Evaporation of the EtOAc layer and chromatography (silica gel, step gradient: 30-100% ether/petroleum ether, then 80% ether/EtOAc) gave the subtitle compound as a foam (0.65g).
**Step 5: (S)-*N*-[1-(4-Methoxyphenyl)-2-(3-quinolylmethanesulfonyl)ethyl]-*N*-hydroxyformamide -** (S)-*N*-[1-(4-Methoxyphenyl)-2-(3-quinolylmethanesulfonyl)ethyl]-N,O-bis-tert-butoxycarbonylhydroxylamine (0.614g) was dissolved into 90% TFA/water (10ml) and left for 2h. Toluene (30ml) was added and the reaction was evaporated and re-evaporated from toluene (2 x 30ml), dissolved into EtOAc and treated with saturated sodium hydrogen carbonate solution until the EtOAc layer was neutral. The organic layer was evaporated and redissolved in a mixture of acetic anhydride and formic acid (15ml, 1:3 premixed for 10min). After 2h the reaction was evaporated from toluene (3 x 30ml), triturated with ether, and redissolved in methanol (10ml). After 30min the solution was evaporated and re-evaporated from toluene/methanol (3 x 20ml). A portion containing half of the crude residue was purified by preparative HPLC eluting with a water/acetonitrile gradient containing 0.1%TFA to give the TFA salt of the title compound (0.088g). MS electrospray (+ve ion) 401 (MH⁺), 801 (2MH⁺); MS electrospray (-ve ion) 399 (M-H⁻), 799 (2M-H⁻); ¹H NMR δ (CD₃OD), 9.1 (1H, br s), 8.85 (1H, br s), 8.33 (1H, br d), 8.14 (2H, d, J=8.9Hz), 8.02 (1H, m), 7.82 (1H, m), 7.39 (2H, br m), 6.92 (2H, d, J=8.9Hz), 6.12-6.05 and 5.62-5.5 (1H, br m x2, rotamers), 4.77 (2H, s), 4.46-4.12 and 3.89-3.6 (2H, br AB x 2, rotamers) and 3.77 (3H, s).

### Example 51: (S)-N-[1-(3,4-Dihydro-2H-benzo[b][1,4]dioxepin-7-yl)-2-(3- quinolylmethanesulfonyl)ethyl]-N-hydroxyformamide

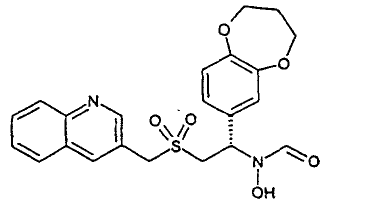

2-Bromo-1-(3,4-dihydro-2*H*-benzo*[b]*[1,4]dioxepin-7-yl)ethanone was converted into the title compound using the procedures of Example 50 steps 1-5. Final final purification was carried out by chromatography (silica gel, step gradient :10-50% EtOAc/petroleum ether). MS electrospray (+ve ion) 443 (MH⁺), 885 (2MH⁺); MS electrospray (-ve ion) 441 (M-H⁻), 883 (2M-H⁻); ¹H NMR δ [CD₃OD], 8.88 (1H, br s), 8.39 (1H, br s), 8.3 (1H, br s), 8.03 (1H, d, J=8.4Hz), 7.93 (1H, d, J=8Hz), 7.8 (1H, m), 7.6 (1H, m), 7.1-6.9 (3H, m), 6.01 and 5.48 (1H, br m x2, rotamers), 4.65 (2H, s), 4.3-3.5 (6H, m), 2.2-2.1 (2H, m).

### Example 52:(S)-N-[1-(3-Quinolylmethanesulfonyl)-2-pentyl]-N-hydroxyformamide

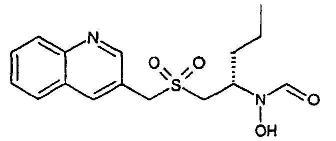

**Step 1: (S)-2-N-t-Butoxycarbonylaminopentanol -** (S)-2-N-t-Butoxycarbonylaminopentanoic acid (6.2g) was converted into the subtitle compound (5.6g) by the method of M. Ho et al., *Tet. Lett.* 1993, 34(41), 6513. ¹H NMR δ(CDCl₃):4.64 (1H,s), 3.45-3.78 (3H,m), 2.61 (1H,s), 1.3-1.52 (13H, m), 0.93 (3H, t, J=7.5Hz).
**Step 2: (S)-2-N-t-Butoxycarbonylaminopentyl thioacetate -** An ice-cold solution of triphenylphosphine (14.2g) in THF (150ml) was treated dropwise with diisopropyl azodicarboxylate (10.65ml) and stirred for 30 min. A solution of (S)-2-N-t-butoxycarbonylaminopentanol (5.5g) and thioacetic acid (3.86ml) in THF (20ml) was added dropwise. The reaction was allowed to gain rt overnight and evaporated. The residue was extracted with hexane (500ml), evaporated and flash chromatographed (silica gel, step gradient: 50-100% MDC/hexane then 5% EtOAc/MDC) to give the subtitile compound (6.3g). MS (APCI +ve ion) 284(MNa ⁺); ¹H NMR δ(CDCl₃): 4.43 (1H, broad d), 3.73 (1H, m), 2.93-3.14 (2H, m), 2.35 (3H, s), 1.18-1.51 (13H, m),0.91 (3H, t, J=7.3Hz),
**Step 3: (S)-2-(N-t-Butoxycarbonylamino)-1-(3-quinolylmethanesulfanyl)pentane -** A stirred mixture of (S)-2-N-t-butoxycarbonylaminopentyl thioacetate (1.15g) and 3-chloromethylquinoline hydrochloride (0.86g) in methanol (60ml) at rt was treated dropwise with 1 M sodium hydroxide (8.47ml) and stirred for 4h. The mixture was evaporated to low volume and diluted with saturated sodium hydrogen carbonate solution (10ml). After extraction with MDC (2x20ml), the combined extracts were dried (MgSO₄) and evaporated. The residue was flash chromatographed (silica gel, step gradient: 15-40% EtOAc/MDC) to give the subtitle compound (1.4g). MS (APCI +ve ion) 383(MNa ⁺); ¹H NMR δ(CDCl₃): 8.9 (1H, m), 8.1 (2H, m), 7.79 (1H, d, J=8 Hz), 7.69 (1H, t, J=8Hz), 7.51(1H, t, J=8Hz), 4.45 (1H, broad d), 3.92 (2H, s), 3.83 (1H, m), 2.53 (2H, d, J=6Hz), 1.2-1.6 (13H, m), 0.9 (3H, t, J=7.5Hz).
**Step 4: (S)-2-(N-t-Butoxycarbonylamino)-1-(3-quinolylmethanesulfonyl)pentane -** An ice-cold solution of (S)-2-(N-t-butoxycarbonylamino)-1-(3-quinolylmethanesulfanyl)pentane (1.37g) in MDC (30ml) was treated with 50% 3-chloroperoxybenzoic acid (2.63g) and the mixture stirred for 1h. The reaction mixture was quenched with 10% sodium thiosulfate (10ml) followed by saturated sodium bicarbonate (10ml) solution. After 5 min the organic phase was collected, dried (MgSO₄) and evaporated. The residue was flash chromatographed (silica gel; step gradient: 60-90% EtOAc/hexane) to give the subtitle compound (0.85g). MS (APCI +ve ion) 415 (MNa ⁺); ¹H NMR δ(CDCl₃): 8.9 (1H, m), 8.3 (2H, d, J=2Hz), 8.1 (1H d, J=8 Hz), 7.77 (1H, t, J=8Hz), 7.58 (1H, t, J=8Hz), 4.85 (1H, broad ), 4.5 (2H, m), 4.1 (1H, m), 3.0-3.3 (2H,m), 1.2-1.8 (13H,m), 0.9 (3H, t, J=7.5Hz).
**Step 5: (S)-2-Amino-1-(3-quinolylmethanesulfonyl)pentane dihydrochloride - A** stirred solution of (S)-2-(N-t-butoxycarbonylamino)-1-(3-quinolylmethanesulfonyl)pentane (0.83g) in MDC (15ml) at rt was treated with 4M hydrogen chloride in dioxan (10ml). After 1.5h the mixture was evaporated to give the subtitle compound (0.77g). MS (APCI+ve ion) 293 (MH ⁺), ¹H NMR δ(DMSO-d6) 9.04 (1H, m), 8.64 (1H, m), 8.24 (3H, broad s), 8.15 (2H, m), 7.86 (1H, t, J=8Hz), 7.76 (1H, t, J=8Hz), 5.01 (2H, s), 3.4-3.77 (3H, m), 1.7 (2H, m), 1.38 (2H, m), 0.87 (3H, t, J=7.3Hz).
**Step 6: (S)-2-(N-Cyanomethylamino)-1-(3-quinolylmethanesulfonyl)pentane -** A mixture of (S)-2-amino-1-(3-quinolylmethanesulfonyl)pentane dihydrochloride (0.76g), bromoacetonitrile (0.35ml) and N-ethyldiisopropylamine (1.44ml) in acetonitrile (15ml) was refluxed for 16h, cooled and evaporated. The residue was partitioned between MDC (15ml) and saturated sodium hydrogen carbonate solution (15ml). The organic phase was collected and the aqueous re-extracted with MDC (15ml). The combined MDC phases were dried (MgSO₄) and evaporated. The residue was flash chromatographed (silica gel, step gradient: 70-100% EtOAc/hexane) to give the subtitle compound (0.5g). MS (APCI +ve ion) 354(MNa ⁺); ¹H NMR δ(CDCl₃): 8.9 (1H, m), 8.3 (1H, d, J=2Hz), 8.14 (1H, d, J=8 Hz), 7.87 (1H, d, J=8Hz), 7.78 (1H, t, J=8Hz), 7.64 (1H, t, J=8Hz), 4.53 (2H, s), 3.68 (2H, m), 3.41 (1H, m), 3.0 (2H, m), 2.03 (1H, m), 1.2-1.7 (4H, m), 0.93 (3H, t, J=7.3Hz).
**Step 7: (S)-N-[1-(3-Quinolylmethanesulfonyl)-2-pentyl]hydroxylamine -** An ice-cold stirred solution of (S)-2-(N-cyanomethylamino)-1-(3-quinolylmethanesulfonyl)pentane (269mg) in MDC (10ml) was treated with pure 3-chloroperoxybenzoic acid (281mg). After 45 min the reaction was quenched with 10% sodium thiosulphate (5ml) followed by saturated sodium hydrogen carbonate (5ml) solution. After 5 min the organic layer was collected and the aqueous re-extracted with MDC (5ml). The combined MDC phases were dried (MgSO₄) and evaporated. The residue was redissolved in methanol (10ml) treated with hydroxylamine hydrochloride (1.12g) and heated at 60°C for 1.5h. The cooled solution was evaporated, then dissolved in water (5ml) and saturated sodium hydrogen carbonate solution (5ml). After extraction with MDC (2x5ml), the combined extracts were dried (MgSO₄) and evaporated. The residue was flash chromatographed (silica gel, step gradient: 1-4% methanol/ MDC) to give the subtitle compound (71mg). MS (APCI +ve ion) 309(MH⁺); ¹H NMR δ(CDCl₃): 8.9 (1H, m), 8.3 (1H, d, J=2Hz), 8.14 (1H, d, J=8Hz), 7.85 (1H, d, J=8Hz), 7.75 (1H, t, J=8Hz), 7.6 (1H, t, J=8Hz), 5.59 (1H, broad), 4.97 (1H, s)4.5 (2H, m), 3.4 (2H, m), 2.96 (1H, m), 1.2-1,8 (4H,m), 0.92 (3H, t, J=7.3Hz).
**Step 8: (S)-N-[1-(3-Quinolylmethanesulfonyl)-2-pentyl]-N-hydroxyformamide -** (S)-N-[1-(3-Quinolylmethanesulfonyl)-2-pentyl]hydroxylamine (68mg) was dissolved in a premixed solution of acetic anhydride (2ml) and formic acid (6ml) and stood overnight. The reaction was evaporated and then re-evaporated from chloroform (2x5ml). The residue was redissolved in methanol (5ml), treated with potassium carbonate (152mg) and stirred for 45 min and then evaporated. The residue was redissolved in water (5ml) and the pH adjusted to 7 with 1M hydrochloric acid. After extraction with MDC (2x5ml), the combined extracts were dried (MgSO₄) and evaporated. The residue was chromatographed (acid washed silica gel, step gradient: 0-70% EtOAc/MDC) to give the title compound (71mg). MS (APCI +ve ion) 337 (MH⁺); ¹H NMR δ[(CD₃)₂CO] 8.9 (1H, m), 8.8 and 8.3 (1H, 2xs rotamers), 8.77 and 8.4 (1H, 2xs rotarners), 8.25 (1H,m), 7.95 (1H,m), 7.85 (1H, d, J=8Hz), 7.66 (1H, t, J=8Hz), 7.49 (1H, t, J=8Hz), 4.85 and 4.2(1H, 2xs rotamers) 3.05-3.66 (2H, m), ,1.15-1,85 (4H, m), 0.77 (3H, t, J=7.3Hz).

### Example 53: :(S)-N-[1-(3-Quinolylmethanesulfonyl)-2-butyl]-N-hydroxyformamide

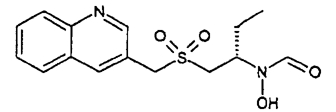

**Step 1: (S)-2-N-t-Butoxycarbonylaminobutanol -** (S)-2-N-t-Butoxycarbonylaminobutanoic acid (8.22g) was converted into the subtitle compound (7.5g) by the method of M. Ho et al., Tet. Lett. 1993, 34(41), 6513. ¹H NMR δ(CDCl₃):4.65 (1H, s), 3.44-3.76, (3H, m), 2.60 (1H, s), 1.3-1.55 (11H, m), 0.93 (3H, t, J=7.5Hz).
**Step 2: (S)-2-N-t-Butoxycarbonylaminobutyl thioacetate -** (S)-2-N-t-Butoxycarbonylaminobutanol (7.45g) was converted into the subtitle compound (6.68g) as described in Example 52 step 2. MS (APCI +ve ion) 265 (MNH₄⁺); ¹H NMR δ(CDCl₃): 4.45 (1H, broad d), 3.64 (1H, m), 2.93-3.17 (2H, m), 2.35 (3H, s), 1.44 (9H, m),1.26 (2H, m), 0.94 (3H, t, J=7Hz).
**Step 3: (S)-2-(N-t-Butoxycarbonylamino)-1-(3-quinolylmethanesulfanyl)butane -** (S)-2-N-t-Butoxycarbonylaminobutyl thioacetate (1.09g) was converted into the subtitle compound (1.06g) as described in Example 52 step 3. MS (APCI +ve ion) 369 (MNa ⁺); ¹H NMR δ(CDCl₃): 8.9 (1H, m), 8.1 (2H, d, J=2Hz), 7.8 (1H, d, J=8 Hz), 7.69 (1H, t, J=8Hz), 7.54 (1H, t, J=8Hz), 4.4(1H, broad d), 3.92 (2H, s), 3.73 (1H, m), 2.53 (2H, d, J=6Hz), 1.2-1.6 (11H, m), 0.91 (3H, t, J=7.5Hz).
**Step 4: (S)-2-(N-t-Butoxycarbonylamino)-1-(3-quinolylmethanesulfonyl)butane** - (S)-2-(N-t-Butoxycarbonylamino)-1-(3-quinolylmethanesulfanyl)butane (1.05g) was converted into the subtitle compound (0.73g) as described in Example 52 step 4. MS (APCI +ve ion) 401 (MNa⁺); ¹H NMR δ(CDCl₃): 8.93 (1H, m), 8.33 (2H, m), 8.13(1H, d, J=8 Hz), 7.77 (1H, t, J=8Hz), 7.59 (1H, t, J=8Hz), 4.83 (1H, broad ), 4.5 (2H, m), 4.05 (1H, m), 3.0-3.3 (2H, m), 1.6-1.9 (2H, m), 1.47 (9H, s), 0.97 (3H, t, J=7.5Hz).
**Step 5: (S)-2-Amino-1-(3-quinolylmethanesulfonyl)butane dihydrochloride -** (S)-2-(N-t-Butoxycarbonylamino)-1-(3-quinolylmethanesulfonyl)butane (0.72g) was converted into the subtitle compound (0.67g) as described in Example 52 step 5: MS (APCI +ve ion) 279 (MH ⁺), ¹H NMR δ (DMSO-d6): 9.1 (1H, m),8.75 (2H, m), 8.34 (3H, broad s), 8.2 (2H,m), 7.97 (1H, t, J=8Hz), 7.8(1H, t, J=8Hz), 5.05 (2H, s ), 3.41-3.73 (3H, m), 1.76 (2H, m), 0.95 (3H, t, J=7.3Hz).
**Step 6: (S)-2-(N-Cyanomethylamino)-1-(3-quinolylmethanesulfonyl)butane - (S)-2**-Amino-1-(3-quinolylmethanesulfonyl)butane dihydrochloride (0.61g).) was converted into the subtitle compound (0.48g) as described in Example 52 step 6. MS (APCI +ve ion) 318(MH ⁺); ¹H NMR δ(CDCl₃): 8.91 (1H, m), 8.31 (1H, d, J=2Hz) 8.14 (1H, d, J=8Hz), 7.87 (1H, d, J=8Hz), 7.78 (1H, t, J=8Hz), 7.61 (1H, t, J=8Hz), 4.54 (2H, s), 3.68 (2H, m), 3.38 (1H, m), 3.0 (2H, m), 2.07 (1H, m), 1.5-1.73 (2H, m), 0.91 (3H, t, J=7.3Hz).
**Step 7: (S)-N-[1-(3-Quinolylmethanesulfonyl)-2-butyl]hydroxylamine -** (S)-2-(N-Cyanomethylamino)-1-(3-quinolylmethanesulfonyl)butane (436mg) was converted into the subtitle compound (46mg) as described in Example 52 step 7. MS (APCI +ve ion) 295(MH⁺); ¹H NMR δ(CDCl₃): 8.91 (1H,m), 8.32 (1H, d, J=2Hz), 8.13 (1H, d, J=8 Hz), 7.86 (1H, d, J=8Hz), 7.79 (1H, t, J=8Hz), 7.61 (1H, t, J=8Hz), 5.64 (1H, broad), 4.68 (1H,s), 4.52 (2H, m),3.4 (2H, m), 2.89 (1H, m),1.4-1.8 (2H, m), 0.96 (3H, t, J=7.3Hz).
**Step 8: (S)-N-[1-(3-Quinolylmethanesulfonyl)-2-butyl]-N-hydroxyformamide -** (S)-N-[1-(3-Quinolylmethanesulfonyl)-2-butyl]hydroxylamine (82mg) was converted into the title compound (46mg) as described in Example 52 step 8. MS (APCI +ve ion) 323 (MH⁺); ¹H NMR δ[(CD₃)₂CO] 8.8 (1H, s),8.75 and 8.32 (1H, 2xs rotamers), 8.65 and 8.5 (1H, 2xs rotamers), 8.33 (1H, m), 7.95(1H, m), 7.67 (1H, t, J=8Hz), 7.5 (1H, t, J=8Hz), 4.77 and 4.14 (1H, 2xs rotamers) 4.52 (2H, m),3.05-3.66 (2H, m), 1.5-1.8 (2H, m), 0.77 (3H, t, J=7.3Hz).

### Example 54: :(S)-N-[1-(3-Quinolylmethanesulfonyl)-4-methyl-2-pentyl]-N- hydroxyformamide

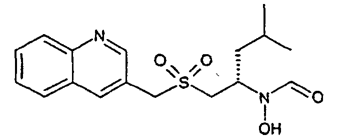

**Step 1: (S)-2-N-t-butoxycarbonylamino-4-methylpentyl thioacetate -** (S)-2-N-t-Butoxycarbonylamino-4-methylpentanol (10.08g)) was converted into the subtitle compound (6.68g) as described in Example 52 step 2: ¹H NMR δ(CDCl₃): 4.39 (1H, broad d), 3.83 (1H, m), 2.93-3.18 (2H, m), 2.35 (3H, s), 1.44 (9H, m),1.28 (3H, m),0.9(6H, d, J=6.4Hz).
**Step 2: (S)-2-(N-t-Butoxycarbonylamino)-4-methyl-1-(3-quinolylmethanesulfanyl)pentane -** (S)-2-N-t-Butoxycarbonylamino-4-methylpentyl thioacetate (1.42g) was converted into the subtitle compound (1.4g) as described in Example 52 step 3. MS (electrospray +ve ion) 375(MH ⁺); ¹H NMR δ (CDCl₃): 8.87 (1H, m), 8.22 (1H, d, J=2Hz), 8.0 (1H, d, J=8Hz), 7.91 (1H, d, J=8Hz), 7.73 (1H, t, J=8Hz), 7.59 (1H, t, J=8Hz), 6.71 (1H, d, J=9Hz), 3.96 (2H, s), 3.65 (1H, m), 2.41 (2H, m), 1.15-1.6 (12H, m), 0.81 (6H, d, J=6.4Hz).
**Step 3: (S)-2-(N-t-Butoxycarbonylamino)-4-methyl-1-(3-quinolylmethanesulfonyl)pentane -** (S)-2-(N-t-Butoxycarbonylamino)-4-methyl-1-(3-quinolylmethanesulfanyl)pentane (1.24g) was converted into the subtitle compound (1.07g) as described in Example 52 step 4. ¹H NMR δ(CDCl₃): 8.94 (1H, m), 8.34 (1H, d, J=2Hz), 8.14 (1H, d, J=8Hz), 7.85 (1H, d, J=8Hz), 7.76 (1H, t, J=8Hz), 7.59 (1H, t, J=8Hz), 4.78 (1H, d, J=5Hz), 4.53 (2H, m), 4.20 (1H, m), 2.95-3.35 (2H, m), 1.42-1.78 (3H, m), 1.47 (9H, s), 0.92 (6H, d, J=6.4Hz).
**Step 4: (S)-2-Amino-4-methyl-1-(3-quinolylmethanesulfonyl)pentane dihydrochloride** - (S)-2-(N-t-Butoxycarhonylamino)-4-methyl-1-(3-quinolylmethanesulfonyl)pentane (1.05g) was converted into the subtitle compound (0.9g) as described in Example 52 step 5: MS (APCI +ve ion) 307 (MH ⁺); ¹H NMR δ (DMSO-d6): 9.07 (1H, m), 8.68 (1H, m), 8.33 (3H, broad s), 8.17 (2H, m), 7.94 (1H, t, J=8Hz), 7.77 (1H, t, J=8Hz), 5.04 (2H, s), 3.63 (3H, m), 1.8 (3H, m), 0.85 (6H, d, J=6.4Hz).
**Step 5: (S)-2-(N-Cyanomethylamino)-4-methyl-1-(3-quinolylmethanesulfonyl)pentane -** (S)-2-Amino-4-methyl-1-(3-quinolylmethanesulfonyl)pentane dihydrochloride (0.89g) was converted into the subtitle compound (0.76g) as described in Example 52 step 6. MS (APCI +ve ion) 346 (MH ⁺); ¹H NMR δ(CDCl₃): 8.91 (1H, m), 8.31 (1H, d, J=2Hz), 8.13 (1H, d, J=8Hz), 7.87 (1H, d, J=8Hz), 7.78 (1H, t, J=8Hz), 7.63 (1H, t, J=8Hz), 4.54 (2H, s), 3.68 (2H,m), 3.42 (1H, m), 3.01 (2H, m), 1.9(1H, m), 1.22-1.7 (3H, m), 0.91 (6H, d, J=6.4Hz).
**Step 6: (S)-N-[1-(3-Quinolylmethanesulfonyl)-4-methyl-2-pentyl]hydroxylamine -** (S)-2-(N-Cyanomethylamino)-4-methyl-1-(3-quinolylmethanesulfonyl)pentane (359mg) was converted into the subtitle compound (104mg) as described in Example 52 step 7. MS (APCI +ve ion) 323 (MH⁺); ¹H NMR δ(CDCl₃): 8.91(1H, m), 8.32 (1H, d, J=2Hz), 8.13 (1H, d, J=8 Hz), 7.86 (1H, d, J=8Hz), 7.79 (1H, t, J=8Hz), 7.61 (1H, t, J=8Hz), 5.2-5.8 (1H, broad), 5.08 (1H, s), 4.52 (2H, m), 3.4-3.6 (2H, m), 2.85 (1H, m), 1.2-1.8 (3H, m), 0.91 (6H, m).
**Step 7: (S)-N-[1-(3-Quinolylmethanesulfonyl)-4-methyl-2-pentyl]-N-hydroxyformamide -** (S)-N-[1-(3-Quinolylmethanesulfonyl)-4-methyl-2-pentyl]hydroxylamine (104mg) was converted into the title compound (51mg) as described in Example 52 step 8. MS (APCI +ve ion) 351 (MH⁺); ¹H NMR δ[(CD₃)₂CO] 8.8, 8.25 and 7.95 (5H,3xm, rotamers), 7.85 (1H, d, J=8Hz), 7.66 (1H, t, J=8Hz), 7.51 (1H, t, J=8Hz), 4.97 and 4.27 (1H, 2xs rotamers), 4.55 (2H, m), 3.05-3.7 (2H, m), 1.2-1.8 (3H,m), 0.77 (3H, m).

### Example 55: (S)-N-[1-(3-Quinolylmethanesulfonyl)-3-methoxy-2-propyl]-N- hydroxyformamide

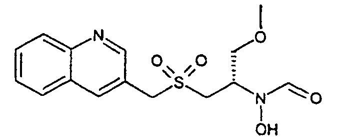

**Step 1: (R)-2-N-t-Butoxycarbonylamino-3-methoxypropanol -** (S)-2-N-t-butoxycarbonylamino-3-methoxypropanoic acid (2.96g) was converted into the subtitle compound (2.04g) by the method of M. Ho et al., *Tet. Lett*. 1993, 34(41), 6513. ¹H NMR δ(CDCl₃): 5.15 (1H, s), 3.45-4.2 (6H, m), 3.36 (3H, s), 2.71 (1H, s), 1.45 (9H, m).
**Step 2: (S)-2-N-t-Butoxycarbonylamino-3-methoxypropyl thioacetate -** An ice-cold solution of triphenylphosphine (5.19g) in THF (80ml) was treated dropwise with diisopropyl azodicarboxylate (3.9ml) and stirred for 30 min. A solution of (S)-2-t-butoxycarbonylamino-3-methoxypropanol (2.03g) and thioacetic acid (1.4ml) in THF (20ml) was added dropwise. The reaction was allowed to gain rt overnight and evaporated. The residue was extracted with hexane (500ml), evaporated and flash chromatographed (silica gel, step gradient: 40-60% EtOAc/hexane) to give the subtitile compound (1.06g). MS (APCI +ve ion) 286 (MNa ⁺); ¹H NMR δ(CDCl₃): 4.9 (1H, broad), 3.87 (1H, m), 3.42 (2H, m), 3.35 (3H, s), 3.08 (2H, m), 2.35 (3H,s), 1.44 (9H, s).
**Step 3: (S)-2-(N-t-Butoxycarbonylamino)-1-(3-quinolylmethanesulfanyl)-3-methoxypropane -** A stirred mixture of (S)-2-N-t-butoxycarbonylamino-3-methoxypropyl thioacetate (1.05g) and 3-chloromethylquinoline hydrochloride (0.81g) in methanol (50ml) at rt was treated dropwise with 1M sodium hydroxide (7.98ml) and stirred for 4h. The mixture was evaporated to low volume and diluted with saturated sodium hydrogen carbonate solution (10ml). After extraction with MDC (2x20ml), the combined extracts were dried (MgSO₄) and evaporated. The residue was flash chromatographed (silica gel, step gradient: 15-30% EtOAc/MDC) to give the subtitle compound (0.9g). MS (APCI +ve ion) 363 (MH ⁺); ¹H NMR δ(CDCl₃): 8.9 (1H, m), 8.1 (2H, m), 7.79 (1H, d, J=8Hz), 7.69 (1H, t, J=8Hz), 7.51 (1H, t, J=8Hz), 4.95 (1H, broad d), 3.91 (3H, m), 3.4, 3.61 (2H, 2xm), 3.32 (3H, s), 2,59 (2H, m) 1.46 (9H, s).
**Step 4: (S)-2-(N-t-Butoxycarbonylamino)-1-(3-quinolylmethanesulfonyl)-3-methoxypropane-** An ice-cold solution of (S)-2-(N-t-butoxycarbonylamino)-1-(3-quinolylmethanesulfanyl)-3-methoxypropane (0.9g) in MDC (30ml) was treated with 50% 3-chloroperoxybenzoic acid (1.72g) and the mixture stirred for 30 min. The reaction mixture was quenched with 10% sodium thiosulfate (10ml) followed by saturated sodium bicarbonate (10ml) solution. After 5 min the organic phase was collected, dried (MgSO₄) and evaporated. The residue was flash chromatographed (silica gel, step gradient: 1-4% MeOH/MDC) to give the subtitle compound (0.74g). MS (APCI +ve ion) 395 (MH ⁺); ¹H NMR δ(CDCl₃): 8.92 (1H, m), 8.36 (1H, d, J=2Hz), 8.13 (1H, d, J=8 Hz), 7.82 (1H, d, J=8Hz), 7.75 (1H, t, J=8Hz), 7.58 (1H, t, J=8Hz), 5.2 (1H, broad ), 4.55 (2H, m), 4.3 (1H, m), 3.7-3.5 (2H, m), 3.37 (3H, s), 3.24 (2H, d, J=6.3Hz), 1.48 (9H, s)
**Step 5: (S)-2-Amino-1-(3-quinolylmethanesulfonyl)-3-methoxypropane dihydrochloride -** A stirred solution of (S)-2-(N-t-butoxycarbonylamino)-1-(3-quinolylmethanesulfonyl)-3-methoxypropane (0.74g) in MDC (10ml) at rt was treated with 4M hydrogen chloride in dioxan (10ml). After 1h the mixture was evaporated to give the subtitle compound (0.69g). MS (APCI +ve ion) 295 (MH ⁺); ¹H NMR δ (DMSO-d6): 9.06 (1H, m), 8.69 (1H, m), 8.32 (3H, broad s), 8.18 (2H, m), 7.91 (1H, t, J=8Hz), 7.77 (1H, t, J=8Hz), 5.05 (2H, m), 3.91 (1H, m), 3.63 (2H, m),3.32 (3H, s).
**Step 6: (S)-2-(N-Cyanomethylamino)-1-(3-quinolylmethanesulfonyl)-3-methoxypropane -** A mixture of (S)-2-amino-1-(3-quinolylmethanesulfonyl)pentane dihydrochloride (0.69g), bromoacetonitrile (0.31ml) and N-ethyldiisopropylamine (1.29ml) in acetonitrile (20ml) was refluxed for 16h, cooled and evaporated. The residue was partitioned between MDC (20ml) and saturated sodium hydrogen carbonate solution (20ml). The organic phase was collected and the aqueous re-extracted with MDC (10ml). The combined MDC phases were dried (MgSO₄) and evaporated. The residue was flash chromatographed (silica gel, step gradient: 70-100% EtOAc/hexane) to give the subtitle compound (0.52g). MS (APCI+ve ion) 334 (MH⁺); ¹H NMR δ(CDCl₃): 8.9 (1H, m), 8.3 (1H, d, J=2Hz), 8.14 (1H, d, J=8Hz), 7.87 (1H, d, J=8Hz), 7.78 (1H, t, J=8Hz), 7.62 (1H, t, J=8Hz), 4.55 (2H, s), 3.7 (2H, m), 3.0-3.65 (5H, m), 3.34 (3H, s), 2.23 (1H, m),
**Step 7: (S)-N-[1-(3-Quinolylmethanesulfonyl)-3-methoxy-2-propyl]hydroxylamine -** An ice-cold stirred solution of (S)-2-(N-cyanomethylamino)-1-(3-quinolylmethanesulfonyl)-3-methoxypropane (323mg) in MDC (10ml) was treated with 70% 3-chloroperoxybenzoic acid (478mg). After 45 min the reaction was quenched with 10% sodium thiosulphate (5ml) followed by saturated sodium hydrogen carbonate (5ml) solution. After 5 min the organic layer was collected and the aqueous re-extracted with MDC (10ml). The combined MDC phases were dried (MgSO₄) and evaporated. The residue was redissolved in methanol (10ml), treated with hydroxylamine hydrochloride (1.3g) and heated at 60°C for 1.5h. The cooled solution was evaporated, then dissolved in water (10ml) and saturated sodium hydrogen carbonate solution (10ml). After extraction with MDC (2x5ml), the combined extracts were dried (MgSO₄) and evaporated. The residue was flash chromatographed (silica gel, step gradient: 2-8% methanol/MDC) to give the subtitle compound (200mg). MS (APCI +ve ion) 311 (MH⁺); ¹H NMR δ(CDCl₃): 8.9 (1H, m), 8.32 (1H, d, J=2Hz), 8.13 (1H, d, J=8 Hz), 7.85 (1H, d, J=8Hz), 7.77 (1H, t, J=8Hz), 7.6 (1H, t, J=8Hz), 5.25 (1H, broad), 4.54 (2H, m), 3.71 (1H, m), 3.57 (2H, m), 3.34 (2H, m), 3.36 (3H, s), 3.0 (1H, m).
**Step 8: (S)-N-[1-(3-Quinolylmethanesulfonyl)-3-methoxy-2-propyl]-N-hydroxyformamide ―** (S)-N-[1-(3-Quinolylmethanesulfonyl)-3-methoxy-2-propyl]hydroxylamine (195mg) was dissolved in a premixed solution of acetic anhydride (1ml) and formic acid (3ml) and stood overnight. The reaction was evaporated then re-evaporated from chloroform (2x5ml). The residue was redissolved in methanol (5ml), treated with potassium carbonate (260mg), stirred for 15min and then evaporated. The residue was redissolved in water (5ml) and the pH adjusted to 7 with 1M hydrochloric acid. After extraction with MDC (2x5ml), the combined extracts were dried (MgSO₄) and evaporated. The residue was chromatographed (acid washed silica gel, step gradient: 2-5% MeOH/MDC) to give the title compound (122mg). MS (APCI +ve ion) 339 (MH⁺); ¹H NMR δ[(CD₃)₂CO] 8.93 (1H, m), 8.8 and 8.3 (1H, 2xs rotamers), 8.3 and 7.95 (1H,2xs rotamers), 8.3 (1H, m), 7.95 (1H, m), 7.85 (1H, d, J=8Hz), 7.66 (1H, t, J=8Hz), 7.51(1H, t, J=8Hz), 5.05 and 4.4 (1H, 2xs rotamers), 4.6 (2H, m), 3.1-3.65 (4H, m), 3.2 (3H, s).

### Example 56: (S)-N-[1-(3-Quinolylmethanesulfonyl)-3-methyl-2-butyl]-N-hydroxyformamide.

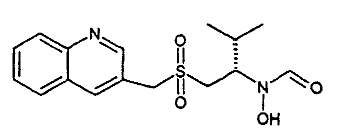

**Step 1 : 1-Bromo-3-methylbutan-2-one -** Prepared from 3-methylbutan-2-one as described in Example 32 step 1.
**Step 2 : 1-Bromo-3-methylbutan-2-ol -** A solution of 1-bromo-3-methylbutan-2-one (3.5g) in dry THF (30ml) was cooled in an ice bath and treated dropwise with a 1M solution of borane in THF (30ml). The reaction mixture was then allowed to stir at rt for 2 h followed by the addition of excess saturated sodium hydrogen carbonate. The solution was then extracted into EtOAc (2x) and washed with water (2x). The EtOAc layer was dried (MgSO₄) and evaporated to give the subtitle compound as an oil (2.84g).
**Step 3 : 3-Methyl-1-(3-quinolylmethanesulfanyl)-butan-2-ol -** A solution of 3-acetylthiomethylquinoline (2.1g) in dry MeOH (30ml) was treated with a 0.5M solution of NaOCH₃ in MeOH (20ml) for 30 min followed by the addition of 1-bromo-3-methylbutan-2-ol (1.5g). The reaction mixture was stirred at rt overnight. The crude reaction mixture was absorbed onto silica gel and chromatographed (silica gel, step gradient: 3-12% EtOAc/petroleum ether) to give the subtitle compound as a yellow solid (1.3g). MS electrospray (+ve ion) 262.29 (M+H⁺); ¹H NMR δ(DMSO-d6): 8.88 (1H, d, J=2.4Hz), 8.22 (1H, d, J=2.4Hz), 8.01 (1H, d, J=8.4Hz), 7.80 (1H, d, J=8.4Hz), 7.74 (1H, t, J=8.4Hz), 7.67(1H, t, J=8Hz) ,4.65 (1H, b, s), 3.97 (2H, s), 4.05-4.00 (1H, m), 3.98 (2H, ABq), 2.52-2.39 (2H, m), 1.67-1.61 (1H, m), 0.78 (3H, d, J=6Hz), 0.77 (3H, d, J=6Hz).
**Step 4 : 3-Methyl-1-(3-quinolylmethanesulfonyl)-butan-2-ol -** A solution of 3-methyl-1-(3-quinolylmethanesulfanyl)-butan-2-ol (1.2g) in dry MDC (30ml) was cooled to 0°C followed by the addition of MCPBA (65%) (2.31g) portionwise and allowed to stir at 0°C for 30 min. The reaction mixture was then washed with a solution of Na₂SO₃ (2g) in 100ml saturated sodium hydrogen carbonate (2x) followed by saturated sodium carbonate (2x). The MDC layer was dried (MgSO₄) and absorbed on to silica gel and chromatographed (silica gel, step gradient: 10-100% EtOAc/hexane) to give the subtitle compound as a yellow solid (0.5g). MS electrospray (+ve ion ) 294 (M+H⁺), 310 (MH⁺ + NH₃), ¹H NMR δ(DMSO-d6) 8.88 (1H, d, J=2.4Hz), 8.22 (1H, d, J=2.4Hz), 8.04 (1H, d, J=8.4Hz), 7.98 (1H, d, J=8.4Hz), 7.74 (1H, t, J=8.4 Hz), 7.67 (1H, t, J=8 Hz) ,5.35 (1H, d, J= 5.6Hz), 4.77 (2H, ABq), 3.91-3.86 (1H, m), 3.3-3.06 (2H, m), 1.75-1.67 (1H, m), 0.78 (3H, d, J=6Hz), 0.77 (3H, d, J=6Hz).
**Step 5 : (E)-1-(3-Quinolylmethanesulfonyl)-3-methylbut-1-ene -** 3-Methyl-1-(3-quinolylmethanesulfonyl)-butan-2-ol (0.3g) in dry MDC (10ml) was treated with Et₃N (1.42ml). The reaction mixture was then cooled to 0°C followed by the dropwise addition of methanesulfonyl chloride (0.16ml). The reaction was stirred at 0°C for 2 h and then partitioned with saturated sodium hydrogen carbonate (2x). The MDC layer was dried (MgSO₄) absorbed on to silica gel and chromatographed (silica gel, step gradient: 5-55% EtOAc/hexane) to give the subtitle compound as a white solid (0.16g). MS electrospray (+ve ion ) 275 (M+H⁺), ¹H NMR δ(DMSO-d6): 8.81 (1H, d, J=2 Hz), 8.31 (1H, d, J=2Hz), 8.04 (1H, d, J=8.4 Hz), 7.98 (1H, d, J=8.4Hz), 7.74 (1H, t, J=8.4Hz), 7.67 (1H, t, J=8 Hz), 6.55 (1H, d, J= 16.4Hz), 6.41 (1H, m), 4.71 (2H, s), 2.45-2.41 (1H, m), 0.89 (6H, d, J=6.8Hz).
**Step 6 : *N***-**[1-(3-Quinolylmethanesulfonyl)-3-methyl-2-butyl]hydroxylamine -** A solution of (E) - 1-(3-Quinolylmethanesulfonyl)-3-methylbut-1-ene (0.16g) in dry THF (2ml) was treated with hydroxylamine (50wt% solution in water, 2ml) and allowed to stir at rt for 3h. Excess solvent was removed by evaporation and azeotroped first with toluene then MeOH to give the subtitle compound as a white solid (110mg). MS electrospray (+ve ion ) 309(M+H⁺); ¹H NMR δ(DMSO-d6) 8.89 (1H, d, J=2.4Hz), 8.4 (1H, d, J=2.4Hz), 8.05 (1H, d, J=8.4 Hz), 8.02 (1H, d J=8.4Hz), 7.81(1H, t, J=7.2Hz), 7.67 (1H, t, J=7.2Hz), 7.34 (1H, s), 4.88-4.79 (2H, m) 3.16-3.09 (2H, m), 2.05-2.0 (1H, m), 0.89 (3H, d, J=6.8Hz), 0.59 (3H, d, J=6.8Hz).
**Step 7: (S)-*N*-[1-(3-Quinolylmethanesulfonyl)-3-methyl-2-butyl]-*N*-hydroxyformamide***-N*-[1-(3-Quinolylmethanesulfonyl)-3-methyl-2-butyl]hydroxylamine (0.11g) was treated with formic acid (2.5ml) and acetic anhydride (0.75ml) and left to stir at rt for 2h. The reaction mixture was stripped to dryness and taken up into methanol followed by the addition of K₂CO₃ (0.24g). After stirring at rt for 45min methanol was removed by evaporation and the residue was partitioned between MDC and water. The MDC layer was dried (MgSO₄) and evaporated to give a crude racemic mixture which was separated into single enantiomers using preparative HPLC (chiralpak AD isocratic ethanol/hexane 25:75, 235nm). The slower running component was collected to give the title compound as a white solid (40mg). Chiral purity: 99.8%ee. MS electrospray (+ve ion) 336 (M+H⁺), ¹H NMR δ(DMSO-d6) 9.76(1H, s), 8.4 (1H, s), 8.37 (1H, s), 8.06-8.00 (2H, m), 7.81 (1H, t, J=7.2Hz), 7.66 (1H, t, J=7.2 Hz), 4.80-4.68 (2H, ABq,) 3.99-3.95(1H, m), 3.61-3.42(1H, m) 3.42-3.34 (1H, m),1.9-1.84 (1H, m ), 0.94 (3H, d J=6.8 Hz), 0.88 (3H, d, J=6.8Hz).

### Example 57: (S)-N-[1-(3-Quinolylmethanesufonyl)-3-isopropoxy-2-propyl]-N-hydroxyformamide.

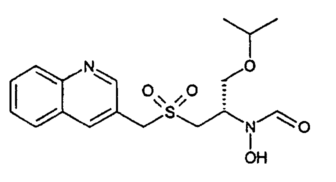

**Step 1: (S)-Aziridine-1, 2-dicarboxylic acid 1-*tert*-butyl ester 2-methyl ester** - (S)-1-Trityl aziridine-2-carboxylic acid methyl ester (J. Baldwin, *Tetrahedron* 1993, 49 (28), 6309) (39.89g) was dissolved in chloroform (300ml) and stirred at rt. p-Toluene sulfonic acid (220.96g) was dissolved in methanol (400ml) and added slowly to this solution and stirred at rt for 2.75 h. The reaction mixture was evaporated to a minimum volume and partitioned between ethyl acetate (500ml) and water (1300ml). The aqueous layer was washed with ethyl acetate (2 x 400ml). The aqueous layer was then neutralised with sodium bicarbonate and extracted with dichloromethane (4 x 300ml) and ethyl acetate (2 x 300ml). The combined organic extracts were dried (sodium sulfate) and filtered, and immediately treated with di-t-butyldicarbonate (48.03g) and DMAP (10mg). The reaction volume was evaporated to approximately half and the reaction allowed to stir overnight. The reaction was then evaporated to a minimum and the residue purified by chromatography (silica gel, step gradient: 0-40% ether/petroleum ether) to give the subtitle compound (10.95g). ¹H NMR δ (CDCl₃): 3.77 (3H, s), 3.03 (1H, m), 2.53 (1H, m), 2.41 (1H, s), 1.45 (9H, s)..
**Step 2: (S)-2-N-tert-Butoxycarbonylamino-3-isopropoxypropanoic acid methyl ester** - (S)-Aziridine-1,2-dicarboxylic acid 1-*tert*-butyl ester 2-methyl ester (4.0g) was dissolved in chloroform (59ml) and isopropanol (70ml) and then boron trifluoride etherate (6 drops) was added and the reaction stirred at rt overnight. A further aliquot of boron trifluoride etherate (6 drops) was then added followed by another 4 drops 4 h later. The reaction was then stirred overnight. The reaction mixture was then evaporated to a minimum and purified by chromatography (silica gel, step gradient 0-30% ether/petroleurn ether) to give the subtitle compound (3.76g). ¹H NMR δ (CDCl₃), 5.35 (1H, d, 7.2Hz), 4.40 (1H, m), 3.82 (1H, m), 3.75 (3H, s), 3.63 (1H, m), 3.54 (1H, m), 1.45 (9H, s), 1.10 (6H, m).
**Step 3: (R)-2-N-tert-Butoxycarbonylamino-3-isopropoxypropanol -** (S)-2-N-tert-Butoxycarbonylamino-3-isopropoxypropanoic acid methyl ester (3.76g) was dissolved in ether (300ml) and cooled to 0°C and then treated with DIBAL (72.1ml, 1M solution in THF) and the reaction stirred at 0°C for 2.5h. The reaction was then allowed to warm to rt and stirred overnight. The reaction was then cooled in an ice bath, treated with a saturated aqueous solution of sodium potassium tartrate (300ml) and stirred vigorously for 30 min (mechanical stirrer). The organic layer was then separated and washed with 1M aqueous sodium hydroxide solution (100ml) and brine (2 x 100ml). The organic layer was then dried (MgSO₄) and evaporated to a yellow oil which was purified by chromatography (silica gel, step gradient: 0-90% ether/petroleum ether) to give the subtitle compound as a clear oil (2.03g). ¹H NMR δ (CDCl₃): 5.17 (1H, broad), 4.85-3.45 (6H, m), 2.82 (1H, broad), 1.47 (9H, s), 1.18 (6H, m)
**Step 4: (S)-1-Bromo-2-(N-tert-Butoxycarbonylamino)-3-isopropoxypropane -** (R)-2-N-tert-Butoxycarbonylamino-3-isopropoxypropanol (2.03g) was dissolved in MDC (30ml) and then triethylamine (1.33ml) was added. Triphenylphosphine dibromide (3.68g) was added, portionwise, and the reaction stirred at rt overnight. The reaction mixture was evaporated to a minimum volume and then re-dissolved in ethyl acetate (180ml) and washed with 10%wt. aqueous citric acid solution (2 x 80ml) and brine (2 x 100ml). The organic layer was then dried (MgSO₄) and evaporated and the crude purified by chromatography (silica gel, step gradient: 0-15% ether/petroleum ether) to yield the subtitle compound as a clear oil which crystallised in the freezer (1.25g). ¹H NMR δ (CDCl₃), 4.95 (1H, broad), 3.90 (1H, broad), 3.69-3.40 (5H, m), 1.45 (9H, s), 1.14 (6H, d, 6.1Hz)
**Step 5: (S)-2-(N-tert-Butoxycarbonylamino)-1-(3-quinolylmethanesulfanyl)-3-isopropoxypropane -** (S)-1-Bromo-2-(N-tert-butoxycarbonylamino)-3-isopropoxypropane was converted into the subtitle compound using the procedure described in Example 6, step 1. ¹H NMR δ (CDCl₃): 8.91-7.48 (6H, aromatics), 4.97 (1H, broad), 3.90-3.78 (3H, m), 3.67-3.40 (3H, m), 2.61 (2H, m), 1.46 (9H, s), 1.11 (6H, m).
**Step 6: (S)-2-(N-tert-Butoxycarbonylamino)-1-(3-quinolylmethanesulfonyl)-3-isopropoxypropane -** (S)-2-(N-tert-Butoxycarbonylamino)-1-(3-quinolylmethanesulfanyl)-3-isopropoxypropane was converted into the subtitle compound using the procedure described in Example 6, step 4. MS electrospray (+ve ion) 423 (MH⁺); ¹H NMR δ (CDCl₃), 8.96-7.51 (6H, aromatics) , 5.19 (1H, broad), 4.64 (1H, m), 4.51 (1H, m), 4.28 (1H, m), 3.71 (1H, m), 3.61 (2H, m), 3.24 (2H, m), 1.48 (9H, s), 1.13 (6H, m).
**Step 7: (S)-2-Amino-1-(3-quinolylmethanesulfonyl)-3-isopropoxypropane dihydrochloride -** (S)-2-(N-tert-Butoxycarbonylamino)-1-(3-quinolylmethanesulfonyl)-3-isopropoxypropane was converted into the subtitle compound using the procedure described in Example 29, step 2.
**Step 8: (S)-2-N-(Cyanomethyl)amino-1-(3-quinolylmethanesulfonyl)-3-isopropoxypropane -** (S)-2-Amino-1-(3-quinolylmethanesulfonyl)-3-isopropoxypropane dihydrochloride was converted into the subtitle compound using the procedure described in Example 29, step 3. MS electrospray (+ve ion) 362 (MH⁺); ¹H NMR δ (CDCl₃), 8.92-7.58 (6H, aromatics), 5.30 (2H, s), 3.72 (2H, d), 3.66-3.42 (4H, m), 3.23 (2H, m), 1.10 (6H, m).
**Step 9: (S)-*N*-[1-(3-Quinolylmethanesufonyl)-3-isopropoxy-2-propyl]hydroxylamine** - (S)-2-N-(Cyanomethyl)amino-1-(3-quinolylmethanesulfonyl)-3-isopropoxypropane was converted into the subtitle compound using the procedure described in Example 29, step 4. MS electrospray (+ve ion) 339 (MH⁺); ¹H NMR δ (CDCl₃), 8.94-7.25 (6H, aromatics), 5.80 (1H, broad), 4.79 (1H, broad), 4.55 (2H, ABq), 3.62-3.0 (5H, m), 1.12 (6H, m).
**Step 10: (S)-*N*-[1-(3-Quinolylmethanesufonyl)-3-isopropoxy-2-propyl]-N-hydroxyformamide -** (S)-*N*-[1-(3-Quinolylmethanesufonyl)-3-isopropoxy-2-propyl]hydroxylamine was converted into the title compound using the procedure described in Example 6, step 6. Chiral purity: >98%ee. MS electrospray (+ve ion) 367 (MH⁺), ¹H NMR δ (DMSO-d6): 10.00-7.64 (8H, aromatics + OH and formyl, rotamers), 4.92 and 4.37 (1H, 2 x m, broad), 4.81 (2H, ABq), 3.56 (5H, m), 1.06 (6H, m).

### Example 58: (S)-N-[1-(Benzo[b]thiophen-5-ylmethanesulfonyl)-3-isopropoxy-2- propyl]-N-hydroxyformamide

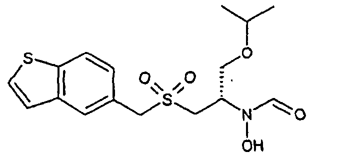

The title compound was prepared using the procedures described in Example 57, steps 1-10. Chiral purity: >98%ee. MS electrospray (+ve ion) 372 (MH⁺). ¹H NMR δ (DMSO-d6), 10.00-7.35 (7H, aromatics + OH and formyl, rotamers), 4.90 and 4.29 (1H, 2 x m, broad), 4.68 (2H, m), 3.59-3.15 (5H, m), 1.03 (6H, d, 6.04Hz).

### Example 59: (S)-N-[1-(4-Methoxyphenyl)-2-(5-thieno[2,3-b]pyridylmethanesulfonyl)ethyl]-N-hydroxyformamide

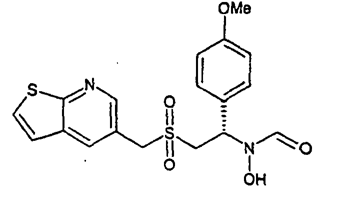

**Step 1: (S)-1-(4-Methoxyphenyl)-2-(5-thieno*[2,3-b]*pyridylmethanesulfanyl)ethanol and (R)-2-(4-methoxyphenyl)-2-(5-thieno*[2,3-b]*pyridylmethanesulfanyl)ethanol -** To 5-acetylthiomethylthieno*[2,3-b]*pyridine (1.32g) in methanol (12ml) was added aqueous sodium hydroxide (3.0ml, 2M). After 10min (S)-2-bromo-1-(4-methoxyphenyl)ethanol (Prepared as described in Example 50 step 1) (1.38g) was added, and after another 30min the solution was partitioned between saturated aqueous ammonium chloride and diethyl ether. The organic layer was dried (MgSO₄) and evaporated and the residue chromatographed (silica gel, step gradient: 25-100% ethyl acetate/hexane) to give a 2:5 mixture of the two subtitle compounds (1.16g). ¹H NMR δ(CDCl₃) 8.4 and 8.5 (1H, 2d, J=2Hz), 7.9 and 8.0 (1H, 2d, J=2Hz), 7.5 (1H, m), 7.2-7.3 (3H, m), 6.8 (2H, m), 4.8 (0.3H, m), 3.6-3.9 (7H, m), and 2.7 (0.6H, m).
**Step 2: (S)-N-[2-(5-Thieno*[2,3-b]*pyridylmethanesulfanyl)-1-(4-methoxyphenyl)ethyl]-N,O-bis-tert-butoxycarbonylhydroxylamine** - The mixture of (S)-1-(4-methoxyphenyl)-2-(5-thieno*[2,3-b]*pyridylmethanesulfanyl)ethanol and (R)-2-(4-methoxyphenyl)-2-(5-thieno*[2,3-b]*pyridylmethanesulfanyl)ethanol (1.16g) in toluene (20ml) under argon was treated with N,O-bis-tert-butoxycarbonyl-hydroxylamine (1.2g) and 1,1'-azobis(N,N-dimethylformamide) (1.2g). After cooling to 0°C tributylphosphine (1.8ml) was added dropwise and the reaction then allowed to warm to rt. After 1.5h the reaction was evaporated and the residue chromatographed (silica gel, step gradient: 10-50% ethyl acetate/hexane) to give the subtitle compound as a gum (1.2g). MS (+ve ion electrospray) 547 (MH⁺, 28%), and 314 (100%).
**Step 3: (S)-N-[2-(5-Thieno*[2,3-b]*pyridylmethanesulfonyl)-1-(4-methoxyphenyl)ethyl]-N,O-bis-tert-butoxycarbonylhydroxylamine -** (S)-N-[2-(5-Thieno*[2,3-b]*pyridyl methanesulfanyl)-1-(4-methoxyphenyl)ethyl]-N,O-bis-tert-butoxycarbonylhydroxylamine (1.2g) in dichloromethane (20ml) at 0°C was treated with MCPBA (65% purity, 1.1g). After 30min the reaction was washed with saturated sodium hydrogen carbonate solution containing 1% sodium sulfite. Evaporation of the organic layer and chromatography (silica gel, step gradient: 25-75% ethyl acetate/hexane) gave the subtitle compound as a foam (0.45g). MS (+ve ion electrospray) 579 (MH⁺, 87%), and 346 (100%).
**Step 4: (S)-*N*-[1-(4-Methoxyphenyl)-2-(5-thieno*[2,3-b]*pyridylmethanesulfonyl)ethyl]-*N*-hydroxyformamide -** A solution of (S)-N-[2-(5-thieno*[2,3-b]*pyridylmethanesulfonyl)-1-(4-methoxyphenyl)ethyl]-N,O-bis-tert-butoxycarbonylhydroxylamine (0.45g) in dichoromethane (10ml) was treated with trifluoroacetic acid (5ml). After 30min the solution was evaporated and redissolved in formic acid (10ml) and acetic anhydride (3ml) was added. After a further 1.5h the solution was again evaporated and redissolved in methanol (10ml). Potassium carbonate (0.4g) was then added and after 15min water was added and the pH adjusted to 7 with 1M hydrochloric acid. Extraction with ethyl acetate and chromatography (silica gel, step gradient: 50-100% ethyl acetate/hexane) gave the title compound as a solid after trituration with ether (30mg). MS (+ve ion electrospray) 813 (M₂H⁺, 35%) and 148 (100%); ¹H NMR δ(CD₃OD): 8.6 (1H, m), 8.3 (2H, m), 7.8 (1H, d, J=6Hz), 7.4 (3H, m), 6.9 (2H, d, J=11Hz), 5.6 and 6.1 (1H, 2m), 4.6 (2H, s), 3.7-4.2 (2H, m), and 3.8 (3H, s).

### Example 60: (S)-N-[1-(4-Methoxyphenyl)-2-(6-thieno[3,2-b]pyridylmethanesulfonyl)ethyl]-N-hydroxyformamide

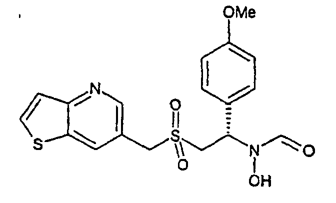

**Step 1: (S)-1-(4-Methoxyphenyl)-2-(6-thieno*[3,2-b]*pyridylmethanesulfanyl)ethanol and (R)-2-(4-methoxyphenyl)-2-(6-thieno*[3,2-b]*pyridylmethanesulfanyl)ethanol -** To 6-acetylthiomethylthieno*[3,2-b]*pyridine (0.8g) in methanol (10ml) was added aqueous sodium hydroxide (1.8ml, 2M). After 10min (S)-2-bromo-1-(4-methoxyphenyl)ethanol (Prepared as described in Example 50 step 1) (0.83g) was added, and after another 30min the solution was partitioned between saturated aqueous ammonium chloride and diethyl ether. The organic layer was dried (MgSO₄) and evaporated and the residue chromatographed (silica gel, step gradient: 0-3% methanol/ethyl acetate) to give a 1:2 mixture of the two subtitle compounds (0.7g). ¹H NMR δ(CDCl₃): 8.4 and 8.5 (1H, 2d, J=2Hz), 8.0 and 8.1 (1H, 2d, J=2Hz), 7.7 (1H, m), 7.5 (1H, m), 7.2-7.3 (2H, m), 6.8 (2H, m), 4.8 (0.3H, m), 3.6-3.9 (7H, m), and 2.8 (0.7H, m).
**Step 2: (S)-N-[2-(6-Thieno*[3,2-b]*pyridylmethanesulfanyl)-1-(4-methoxyphenyl)ethyl]-N,O-bis-tert-butoxycarbonylhydroxylamine -** The mixture of (S)-1-(4-methoxyphenyl)-2-(6-thieno*[3,2-b]*pyridylmethanesulfanyl)ethanol and (R)-2-(4-methoxyphenyl)-2-(6-thieno*[3,2-b]*pyridylmethanesulfanyl)ethanol (0.7g) in toluene (10ml) under argon was treated with N,O-bis-tert-butoxycarbonyl-hydroxylamine (0.75g) and 1,1'-azobis(N,N-dimethylformamide) (0.7g). After cooling to 0°C tributylphosphine (1.05ml) was added dropwise and the reaction then allowed to warm to rt. After 1.5h the reaction was evaporated and the residue chromatographed (silica gel, step gradient: 25-100% ethyl acetate/hexane) to give the subtitle compound as a gum (0.9g). MS (+ve ion electrospray) 547 (MH⁺, 29%), and 314 (100%).
**Step 3: (S)-N-[2-(6-Thieno*[3,2-b]*pyridylmethanesulfonyl)-1-(4-methoxyphenyl)ethyl]-N,O-bis-tert-butoxycarbonylhydroxylamine -** (S)-N-[2-(6-Thieno*[3,2*-*b]*pyridylmethanesulfanyl)-1-(4-methoxyphenyl)ethyl]-N,O-bis-tert-butoxycarbonylhydroxylamine (0.9g) in dichloromethane (15ml) at 0°C was treated with MCPBA (65% purity, 0.85g). After 30min the reaction was washed with saturated sodium hydrogen carbonate solution containing sodium sulfite 1%. Evaporation of the organic layer and chromatography (silica gel, step gradient: 25-100% ethyl acetate/hexane) gave the subtitle compound as a foam (0.3g). MS (+ve ion electrospray) 579 (MH⁺, 100%).
**Step 4: (S)-*N*-[1-(4-Methoxyphenyl)-2-(6-thieno*[3,2-b]*pyridylmethanesulfonyl)ethyl]-*N*-hydroxyformamide -** A solution of (S)-N-[2-(6-thieno*[3,2-b]*pyridyl methanesulfonyl)-1-(4-methoxyphenyl)ethyl]-N,O-bis-tert-butoxycarbonylhydroxylamine (0.3g) in dichoromethane (6ml) was treated with trifluoroacetic acid (2ml). After 30min the solution was evaporated and redissolved in formic acid (10ml) and acetic anhydride (3ml) was added. After a further 1.5h the solution was again evaporated and redissolved in methanol (10ml). Potassium carbonate (0.2g) was then added and after 15min water was added and the pH adjusted to 7 with 1M hydrochloric acid. Extraction with ethyl acetate and chromatography (silica gel, step gradient: 0-6% methanol/ ethyl acetate) gave the title compound as a solid after trituration with ether (75mg). MS (+ve ion electrospray) 813 (M₂H⁺, 22%) and 148 (100%); ¹H NMR δ(CD₃OD), 8.7 (1H, bs), 8.5 (1H, bs), 8.3 (1H, bs), 8.1 (1H, d, J=6Hz), 7.5 (1H, d, J=6Hz), 7.4 (2H, bd, J=11Hz), 6.9 (2H, d, J=11Hz),5.6 and 6.1 (1H, 2m), 4.6 (2H, s), 3.6-4.3 (2H, m), and 3.8 (3H, s).

### Example 61: (S)-N-[(2-(Benzo[b]thiophen-5-yl-methanesulfonyl)-1-(3-oxo-3,4-dihydro-2H-benz[1,4]oxazin-6-yl)ethyl]-N- hydroxyformamide

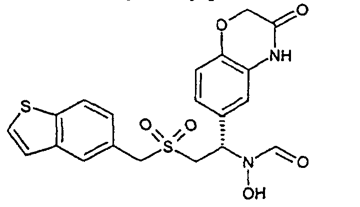

**Step 1: 2-(Benzo*[b]*thiophen-5-ylmethanesulfanyl)-1-(3-oxo-3,4-dihydro-*2H*-benz[1,4]oxazin-6-yl)ethanone -** Prepared according to the procedure described in Example 6, step 1.
**Step 2: 2-(Benzo*[b]*thiophen-5-ylmethanesulfanyl)-1-(3-oxo-4-trimethylsilylethyl-3,4-dihydro-*2H*-benz[1,4]oxazin-6-yl)ethanone** - A solution of 2-(benzo*[b]*thiophen-5-ylmethanesulfanyl)-1-(3-oxo-3,4-dihydro-*2H*-benz[1,4]oxazin-6-yl)ethanone (1.07g) in dry MDC (5ml) was treated with tributylphosphine (1.42ml) and trimethylsilylethanol (0.82ml). The reaction mixture was then allowed to stir for 10min, followed by addition of 1,1'-azobis(N,N-dimethylformamide) (1.00g) and left for 3 h. The reaction mixture was then redissolved in EtOAc (25ml) and washed with water (2x25ml) and brine (20ml). The organic layer was dried (MgSO₄), evaporated and chromatographed (silica gel, step gradient 5-100% ether/petroleum ether) to afford the subtitle compound (214mg).
**Step 3: (S)-*N*-[(2-(Benzo*[b]*thiophen-5-yl-methanesulfonyl)-1-(3-oxo-3,4-dihydro-*2H*-benz[1,4]oxazin-6-yl)ethyl]-*N*-hydroxyformamide** - Prepared according to the procedures described in Example 6, steps 2-6. ¹H NMR δ (MeOH-d₄), 8.23 (1H, s), 7.94-7.92 (3H, m), 7.62 (1H, m), 7.40-7.38 (2H, m), 7.02 (1H, m,) 7.01 (1H, m), 5.95-5.46 (1H, s x2, rotamers), 4.56 (2H, s), 3.51-3.46 (2H, m).

### Example 62: (S)-N-[1-(4-Methoxyphenyl)-2-(3-isoquinolylmethanesulfonyl)ethyl]-N-hydroxyformamide

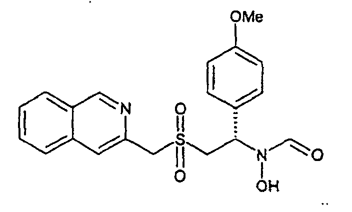

3-Acetylthiomethylisoquinoline (prepared from 3-methylisoquinoline by the procedures of Preparation 6, Method B) was converted into the title compound by the procedures of Example 50 steps 1-5, but with final purification by chromatography (silica gel, step gradient :10-50% EtOAc/petroleum ether). MS electrospray (+ve ion) 401 (MH⁺), 801 (2MH⁺); MS electrospray (-ve ion) 399 (M-H⁻), 799 (2M-H⁻); ¹H NMR δ (CD₃OD), 9.26 (1H, br s), 8.38 and 8.336 (1H, br s x2, rotamers), 8.13 (1H, d, J=8Hz), 7.96 (2H, m), 7.84 (1H, m), 7.74 (1H, m), 7.38 (2H, br d), 6.92 (2H, br d), 6.14 and 5.54 (1H, br d x2, rotamers), 4.8-4.6 (2H, m), 4.26-4.08 (1H, m, rotamers), 3.78 (3H, s) and 3.65-3.47(1H, m, rotamers).

### Example 63: (S)-N-[1-(3,4-Dihydro-2H-benzo[b][1,4]dioxepin-7-yl)-2-(3-isoquinolylmethanesulfonyl)ethyl]-N-hydroxyformamide

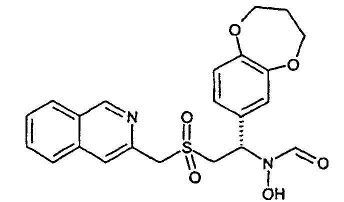

3-Acetylthiomethylisoquinoline (prepared from 3-methylisoquinoline by the procedures of Preparation 6, Method B) was converted into the title compound by the procedures of Example 50 steps 1-5, but with final purification by chromatography (silica gel, step gradient : 10-50% EtOAc/petroleum ether). MS electrospray (+ve ion) 443 (MH⁺), 885 (2MH⁺); MS electrospray (-ve ion) 441 (M-H⁻), 883 (2M-H⁻); ¹H NMR δ (CD₃OD), 9.28 (1H, br s), 8.38 and 8.32 (1H, br s x2, rotamers), 8.14 (1H, d, J=8Hz), 7.97 (2H, m), 7.84 (1H, m), 7.74 (1H, m), 7.07 (1H, m), 7.0 (1H, m), 6.94 (1H,m), 6.08 and 5.5 (1H, br d x2, rotamers), 4.9-4.62 (2H, m), 4.28-4.06 (5H, m), 3.65-48 (1H, m) and 2.15 (2H,m).

### Example 64: (S)-N-[1-(4-Methoxyphenyl)-2-(2-quinoxalylmethanesulfonyl)ethyl]-N-hydroxyformamide

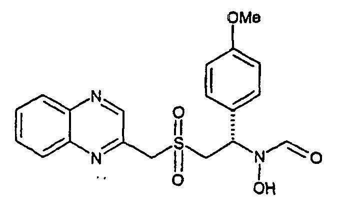

2-Acetylthiomethylquinoxaline (prepared from 2-methylquinoxaline by the procedures of Preparation 6, Method B) was converted into the title compound by the procedures of Example 50 steps 1-5, but with final purification by chromatography (silica gel, step gradient : 10-50% EtOAc/petroleum ether). MS electrospray (+ve ion) 402 (MH⁺), 803 (2MH⁺); MS electrospray (-ve ion) 400 (M-H⁻), 801 (2M-H⁻); ¹H NMR δ (CD₃OD): 8.97 (1H, br s), 8.47 (1H, s), 8.12 (1H, m), 8.03 (1H, br s), 7.85 (2H, m), 7.4 (2H, m), 6.94 (2H, m), 6.16 and 5.57 (1H, br d x2, rotamers), 4.8 (2H, m, obscured by H₂O), 4.38-4.05 (1H, m x2, rotamers), 3.9-3.75 (4H, m).

### Example 65: (S)-N-[2-(2-Fluorobenzo[b]thiophen-5-ylmethanesulfonyl)-1-(4- methylphenyl)ethyl]-N-hydroxyformamide

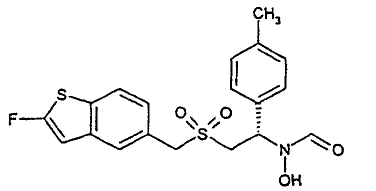

The title compound was prepared from 2-fluoro-5-acetylthiomethylbenzo*[b]*thiophene using procedures described in Example 6 steps 1-6. MS electrospray (+ve ion) 407.9 (M+H⁺), 429.9 (M+Na⁺), 815.0 (2M+ H⁺), 832.1 (2M H⁺ + NH₃ ); ¹H NMR δ(CDCl₃): 8.47-8.21 (1H, s x2, rotamers), 7.76-7.69 (2H, m), 7.41-7.31 (1H, m ), 7.19-7.13 (4H, m), 6.74 (1H, s), 5.95-5.35 (1H, s x2, rotamers), 4.45-4.26 (2H, m), 4.15-3.26 (2H, m), 2.38-2.31 (3H, 2s).

### Example 66: (S)-N-[2-(2-Fluorobenzo[b]thiophen-5-ylmethanesulfonyl)-1-(4-methoxyphenyl)ethyl]-N-hydroxyformamide

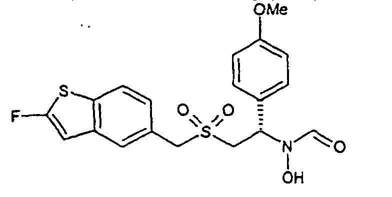

The title compound was prepared from 2-fluoro-5-acetylthiomethylbenzo*[b]*thiophene using procedures described in Example 6, steps 1-6. MS electrospray (+ve ion), 440.9 (M H⁺ + NH₃ ), 864.0 (2M H⁺ + NH₃ ), 868.9 (2M+Na⁺); ¹H NMR δ(CDCl₃): 8.45-8.21 (1H, rotamers), 7.76-7.69 (2H, m), 7.41-7.31 (1H, m ), 6.95-6.85 (2H, m), 6.74 (1H, s), 5.95-5.35 (1H, s x2, rotamers), 4.45-4.26 (2H, m), 4.15-3.26 (2H, m), 3.77 (3H, s).

### Example 67: (S)-N-[1-(3-Methylthiophen-2-yl)-2-(3-quinolylmethanesulfonyl)ethyl]-N-hydroxyformamide.

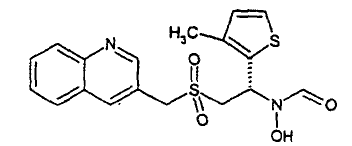

2-Bromo-1-(3-methylthiophen-2-yl)ethanone was prepared from 1-(3-methylthiophen-2-yl)ethanone using the procedure of Example 35 step 1, and was converted into the title compound using the procedures of Example 50, steps 1-5. MS electrospray (+ve ion) 391.0 (M+H⁺), 413.9 (M+Na⁺), 781.0 (2M+H⁺) ; ¹H NMR δ (MeOH-d₄), 8.83 (1H, s), 8.39 (1H, s), 8.06 (1H, m), 7.95 (1H, m), 7.82 (1H, m), 7.71-7.61 (2H, m), 7.34 (1H, d), 6.88 (1H, d), 6.45-5.85 (1H, rotamers), 4.64 (2H, s), 4.22-3.66 (2H, br), 2.28 (3H, s).

### Example 68: (S)-N-[2-(3-Quinolylmethanesulfonyl)-1-(3-methoxycarbonyl-4-methoxyphenyl)ethyl]-N-hydroxyformamide.

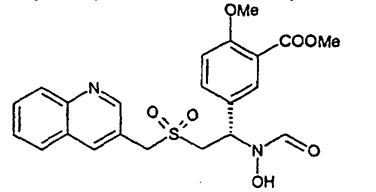

**Step 1: Methyl 5-acetyl-2-methoxybenzoate.** A solution of methyl 5-acetylsalicylate (1.94g) in dry DMF (12ml) was treated with potassium carbonate (1.38g) and iodomethane (0.93 ml) and the suspension was stirred at rt for 5h. The reaction mixture was then diluted with ether and excess 2M HCl. The organic layer was washed with water (6x), dried (MgSO₄) and evaporated to afford the subtitle compound as a white solid (1.31g).
**Step 2: Methyl 5-(2'-bromoacetyl)-2-methoxybenzoate.** Prepared from methyl 5-acetyl-2-methoxybenzoate using the method described in Example 40.
**Step 3: (S)-*N*-[2-(3-Quinolylmethanesulfonyl)-1-(3-methoxycarbonyl-4-methoxyphenyl)ethyl]-*N*-hydroxyformamide.** The title compound was prepared using the procedures described in Example 42 from methyl 5-(2'-bromoacetyl)-2-methoxybenzoate and 3-acetylthiomethylquinoline and obtained as a pale crisp foam. MS electrospray (+ve ion) 459 (MH⁺), (-ve ion) 915 (2M-H), 396. ¹H NMR δ(DMSO-d6, 353 °K) : 8.85 (1H, d, J=1Hz), 8.33 (1H, d, J=1Hz), 8.21 (1H, s), 8.03 (1H, d, J=8.7Hz), 7.97 (1H, d, J=8Hz), 7.80 (1H, dd, J=8 and 8Hz), 7.69 (1H, d, J= 1Hz), 7.63 (1H, dd, J=8 and 8Hz), 7.58 (1H, dd, J=8 and 1Hz), 7.13 (1H, d, J=8.7Hz), 5.71 (1H, br s), 4.74 (2H, s), 4.02 (1H, dd, J= 14.8 and 8.3Hz), 3.82 (3H, s), 3.80 (3H, s), 3.78 (1H, dd, J= 14.8 and 4.9Hz).

### Example 69: (S)-N-[2-(3-Quinolylmethanesulfonyl)-1-(3-carboxy-4-methoxyphenyl)ethyl]-N-hydroxyformamide.

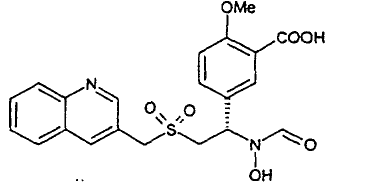

The title compound was prepared from (S)-*N*-[2-(3-quinolylmethanesulfonyl)-1-(3-methoxycarbonyl-4-methoxyphenyl)ethyl]-*N*-hydroxyformamide (Example 68) by treatment with aqueous sodium sulphide following the procedure described for Example 36, and was obtained as a white solid. MS electrospray (+ve ion) 445 (MH⁺). ¹H NMR δ(DMSO-d6, 353°K) : 8.85 (1H, d, J=1 Hz), 8.35 (1H, d, J=1 Hz), 8.27 (1H, s), 8.01 (1H, d, J=8.7 Hz), 7.97 (1H, d, J=8.0 Hz), 7.78 (1H, dd, J=8 and 8 Hz), 7.70 (1H, d, J=1 Hz), 7.63 (1H, dd, J=8 and 8 Hz), 7.54 (1H, dd, J=8 and 1 Hz), 7.10 (1H, d, J= 8.7 Hz), 5.74 (1H, br s), 4.75 (2H, ABq), 4.07 (1H, dd, J=14.6 and 8.1 Hz), 3.82 (3H, s), 3.76 (1H, dd, J= 14.6 and 4.8 Hz).

### Abbreviations

Bn ― Benzyl
(S)-CBS - (S)-2-methyl-CBS-oxazaborolidine
DMF ― N,N-dimethylformamide
EtOAc ― ethyl acetate
h ― hour
min ― minutes
MCPBA ― meta chloroperoxybenzoic acid
MDC ― dichloromethane
Petroleum ether refers to the fraction boiling at 40-60°C
rt ― Room temperature
THF ― tetrahydrofuran
NBS-N-bromosuccinimide
AIBN-Azoisobutyronitrile
DMAP- N,N-dimethylaminopyridine
DIBAL- Di-isobutylaluminium hydride

### HPLC Conditions

Preparative separations were carried out on a Biotage Flex HPLC eluting with solvent A (0.1% TFA in water) and solvent B (0.1% TFA in acetonitrile)

Analytical and preparative chiral HPLC separations were carried out using ChiralPak AD columns and ethanol/hexane as eluant.

## Claims

1. A compound of formula (I): 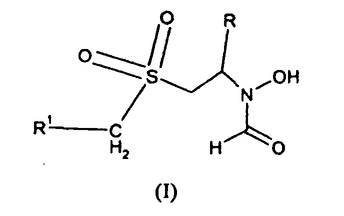 wherein
R is selected from phenyl optionally substituted by up to three groups selected independently from halogen, (C1-6)alkoxy, di-N-(C1-6)alkylaminosulfonyl, (C1-6)acylamino, aryl(C1-6)alkoxycarbonylamino, amino, (C1-6)alkoxycarbonyl, carboxy, hydroxy and (C1-6)alkyl or two groups which together form a fused ring; benzothiophene, thiophene optionally substituted by (C1-6)alkyl, carboxy or (C1-6)alkoxycarbonyl; furanyl; pyrazole optionally substituted by up to two (C1-6)alkyl groups; (C1-6)alkyl optionally substituted by (C1-6)alkoxy, aryl(C1-6)alkoxy or phenyl; isoxazole optionally substituted by (C1-6)alkyl; benzodioxan; benzodioxepine; and benzoxazine; and
R¹ is bicyclyl or heterobicyclyl, wherein bicyclyl or heterobicyclyl means fused bicyclic rings containing 4-7 ring atoms in each ring and wherein heterobicyclyl rings contain up to 4 heteroatoms in each ring selected from oxygen, nitrogen and sulphur.

2. A compound of formula (IA): 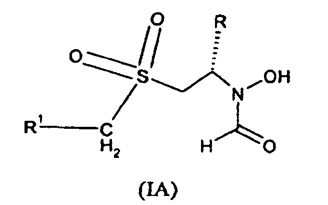 wherein
R and R¹ are as defined in claim 1.

3. A compound according to claim 1 or clam 2 wherein R¹ is selected from benzothiophene optionally substituted by fluorine; indanyl; benzofuranyl; quinolyl; naphthyl; benzothiazole; thienopyridyl; isoquinolyl; and quinoxalyl.

4. A compound according to any preceding claim wherein R is selected from phenyl optionally substituted by one or two groups independently selected from chlorine, fluorine, -OCH₃, -SO₂N(CH₃)₂, -NHCOCH₃, NHCO₂CH₂Ph, amino, ethoxycarbonyl, methoxycarbonyl, carboxy, hydroxy and methyl; benzothiophen-2-yl; thiophen-2-yl optionally substituted by methyl, carboxy or methoxycarbonyl; pyrazol-3-yl optionally substituted by methyl and/or t-butyl; furan-2-yl; furan-3-yl; methyl, ethyl, *n*-propyl, isopropyl, isobutyl or neopentyl each optionally substituted by methoxy, isopropoxy, benzyloxy or phenyl; isoxazol-3-yl optionally substituted by methyl; 3-oxo-3,4-dihydro-*2H*-benz[1,4]oxazin-6-yl; 3,4-dihydro-2*H*-benzo[b][1,4]dioxepin-7-yl; and 2,3-dihydrobenzo[1,4]dioxin-6-yl.

5. A compound according to any preceding claim wherein R¹ is selected from benzothiophene-5-yl optionally substituted by F; indan-2-yl; benzofuran-2-yl; benzofuran-5-yl; benzofuran-6-yl; quinolin-3-yl; 2-naphthyl; benzothiazol-6-yl; thieno[2,3-b]pyridin-5-yl; thieno[3,2-b]pyridin-6-yl; isoquinolin-3-yl; and quinoxalin-2-yl.

6. A compound according to any preceding claim wherein R is selected from phenyl; phenyl substituted by one or two groups independently selected from chlorine, fluorine, -OCH₃, -SO₂N(CH₃)₂, -NHCOCH₃, NHCO₂CH₂Ph, amino, ethoxycarbonyl, methoxycarbonyl, carboxy, hydroxy and methyl; thiophen-2-yl optionally substituted by methyl; 3-oxo-3,4-dihydro-*2H*-benz[1,4]oxazin-6-yl; 3,4-dihydro-2*H*-benzo[b][1,4]dioxepin-7-yl; or 2,3-dihydrobenzo[1,4]dioxin-6-yl; methyl optionally substituted by methoxy or isopropoxy; ethyl; *n*-propyl; isopropyl; and isobutyl.

7. A compound according to any preceding claim wherein R¹ is benzothiophen-5-yl; quinolin-3-yl; thieno[2,3-b]pyridin-5-yl; or thieno[3,2-b]pyridin-6-yl.

8. A compound according to any preceding claim wherein R is substituted or unsubstituted phenyl, such as phenyl substituted by two groups which together form a fused ring, and/or R¹ is benzothiophen-5-yl, 3-quinolinyl, thieno[2,3-b]pyridin-5-yl or thieno[3,2-b]pyridin-6-yl.

9. A compound selected from the group consisting of
*N*-(2-(Benzo[b]thiophen-5-ylmethanesulfonyl)-1-(3,4-dichlorophenyl)-ethyl]-*N*-hydroxyformamide,
*N*-[2-(Benzo[b]thiophen-5-ylmethanesulfonyl)-1-phenyl-ethyl]-*N*-hydroxyformamide, *N*-[2-(Benzo[b]thiophen-5-ylmethanesulfonyl)-1-(4-methoxyphenyl)-ethyl]-*N*-hydroxy-formamide, *N*-[2-(Benzo[b]thiophen-5-ylmethanesulfonyl)-1-(2,3-dihydrobenzo[1,4]dioxin-6-yl)-ethyl]-*N*-hydroxyformamide,
*N*-[2-(Benzo[*b*]thiophen-5-ylmethanesulfonyl)-1-(3,4-dihydro-2*H*-benzo[*b*][1,4]dioxepin-7-yl)-ethyl]-*N*-hydroxyformamide,
(S)-*N*-[2-(Benzo*[b]*thiophen-5-yl-methanesulfonyl)-1-(4-methoxyphenyl)-ethyl]-*N*-hydroxyformamide,
(S)-*N*-[2-(Benzo*[b]*thiophen-5-yl-methanesulfonyl)-1-(phenyl)-ethyl]-*N*-hydroxyformamide,
(S)-*N*-[2-(Benzo[b]thiophen-5-ylmethanesulfonyl)-1-(2,3-dihydrobenzo[1,4]dioxin-6-yl)-ethyl]-*N*-hydroxyformamide,
(S)-*N*-[2-(Benzo[b]thiophen-5-ylmethanesulfonyl)-1-(3,4-dichlorophenyl)-ethyl]-*N*-hydroxyformamide,
(S)-*N*-[2-(Benzo[*b*]thiophen-5-ylmethanesulfonyl)-1-(3,4-dihydro-2*H*-benzo[*b*][1,4]dioxepin-7-yl)-ethyl]-*N*-hydroxyformamide,
(S)-*N*-[2-(Benzo*[b]*thiophen-5-yl-methanesulfonyl)-1-(2,4-difluorophenyl)-ethyl]-*N*-hydroxyformamide,
(S)-*N*-[2-(Benzo*[b]*thiophen-5-yl-methanesulfonyl)-1-(3-hydroxyphenyl)-ethyl]-*N*-hydroxyformamide,
(S)-*N*-[2-(Benzo*[b]*thiophen-5-yl-methanesulfonyl)-1-(4-dimethylaminosulfonylphenyl)-ethyl]-*N*-hydroxyformamide,
(S)-*N*-[2-(Benzo*[b]*thiophen-5-yl-methanesulfonyl)-1-(3-methoxyphenyl)-ethyl)-*N*-hydroxyformamide,
(S)-*N*-[2-(Benzo*[b]*thiophen-5-yl-methanesulfonyl)-1-(3-fluorophenyl)-ethyl]-*N*-hydroxyformamide,
(S)-*N*-[2-(Benzo*[b]*thiophen-5-yl-methanesulfonyl)-1-(4-fluorophenyl)-ethyl]-*N*-hydroxyformamide,
(S)-*N*-[2-(Benzo*[b]*thiophen-5-yl-methanesulfonyl)-1-(benzo*[b]*thiophen-2-yl)-ethyl]-*N*-hydroxyformamide,
(S)-*N*-[2-(Benzo*[b]*thiophen-5-yl-methanesulfonyl)-1-(3-methylthiophen-2-yl)ethyl]-*N*-hydroxyformamide,
(S)-*N*-[2-(Benzo*[b]*thiophen-5-yl-methanesulfonyl)-1-(1-t-butyl-5-methylpyrazol-3-yl)ethyl]-*N*-hydroxyformamide,
(S)-*N*-[2-(Benzo*[b]*thiophen-5-yl-methanesulfonyl)-1-(furan-2-yl)ethyl]-*N*-hydroxyformamide,
(S)-*N*-[2-(Benzo*[b]*thiophen-5-yl-methanesulfonyl)-1-(furan-3-yl)ethyl]-*N*-hydroxyformamide,
(S)-N-[1-Phenyl-2-(2-indanylmethanesulfonyl)ethyl]-N-hydroxyformamide,
(S)-N-[1-Phenyl-2-(benzo*[b]*furan-5-yl-methanesulfonyl)ethyl]-*N*-hydroxyformamide,
(S)-*N*-[1-Phenyl-2-(2-fluoro-benzo*[b]*thiophen-5-yl-methanesulfonyl)ethyl]-*N*-hydroxyformamide,
(S)-*N*-[1-Phenyl-2-(3-fluoro-benzo*[b]*thiophen-5-yl-methanesulfonyl)ethyl]-*N*-hydroxyformamide,
(S)-*N*-[1-Phenyl-2-(3-quinolylmethanesulfonyl)ethyl]-*N*-hydroxyformamide,
(S)-*N*-[2-(Benzo*[b]*furan-5-yl-methanesulfonyl)-1-(4-methoxy-phenyl)-ethyl]-*N*-hydroxyformamide,
(S)-*N*-[1-Benzo*[b]*thiophen-5-yl-methanesulfonyl)-2-propyl]-*N*-hydroxyformamide,
(S)-*N*-[1-Benzyloxy-3-(benzo*[b]*thiophen-5-yl-methanesulfonyl)-2-propyl]-*N*-hydroxyformamide,
(S)-*N*-[1-(Benzo*[b]*thiophen-5-yl-methanesulfonyl)-3-phenyl-2-propyl]-*N*-hydroxyformamide,
(S)-*N*-[1-(Benzo*[b]*thiophen-5-yl-methanesulfonyl)-4,4-dimethyl-2-pentyl]-*N*-hydroxyformamide,
(S)-*N*-[1-(Benzo*[b]*thiophen-5-yl-methanesulfonyl)-3-methyl-2-butyl]-*N*-hydroxyformamide;
N-[ 1-(Benzo*[b]*thiophen-5-yl-methanesulfonyl)-4-methyl-2-pentyl]-*N*-hydroxyformamide; (S)-*N*-[2-(Benzo*[b]*thiophen-5-yl-methanesulfonyl)-1-(5-methylisoxazol-3-yl)-ethyl]-*N*-hydroxyformamide; (S)-*N*-[1-(4-Acetamidophenyl)-2-(benzo*[b]*thiophen-5-yl-methanesulfonyl)ethyl]-*N*-hydroxyformamide; (S)-*N*-[2-(Benzo*[b]*thiophen-5-yl-methanesulfonyl)-1-(5-carboxythiophen-2-yl)ethyl]-*N*-hydroxyformamide; (S)-*N*-[2-(Indan-2-yl-methanesulfonyl)-1-(5-methoxycarbonylthiophen-2-yl)ethyl]-*N*-hydroxyformamide; (S)-*N*-[2-(Indan-2-ylmethanesulfonyl)-1-(3-benzyloxycarbonylaminophenyl)ethyl]-*N*-hydroxyformamide;
(S)-*N*-[2-(Indan-2-ylmethanesulfonyl)-1-(3-aminophenyl)ethyl]-*N*-hydroxyformamide; (S)-*N*-[2-(Benzo*[b]*thiophen-5-ylmethanesulfonyl)-1-(4-ethoxycarbonylphenyl)ethyl]-*N*-hydroxyformamide; (S)-*N*-[2-(Benzo*[b]*thiophen-5-ylmethanesulfonyl)-1-(4-carboxyphenyl)ethyl]-*N*-hydroxyformamide; (S)-*N*-[2-(3-Quinolylmethanesulfonyl)-1-(4-ethoxycarbonylphenyl)ethyl]-*N*-hydroxyformamide;
(S)-*N*-[2-(3-Quinolylmethanesulfonyl)-1-(4-carboxyphenyl)ethyl]-*N*-hydroxyformamide; (S)-N-[1-Phenyl-2-(benzo*[b]*furan-6-ylmethanesulfonyl)ethyl]-N-hydroxyformamide; (S)-N-[1-Phenyl-2-(2-naphthylmethanesulfonyl)ethyl]-N-hydroxyformamide; (S)-N-[1-Phenyl-2-(benzothiazol-6-ylmethanesulfonyl)ethyl]-N-hydroxyformamide; (S)-N-[1-Phenyl-2-(benzo*[b]*furan-2-ylmethanesulfonyl)ethyl]-N-hydroxyformamide; (S)-*N*-[1-Phenyl-2-(5-thieno*[2,3-b]*pyridylmethanesulfonyl)ethyl]-*N*-hydroxyformamide; (S)-*N*-[1-Phenyl-2-(6-thieno*[3,2-b]*pyridylmethanesulfonyl)ethyl]-*N*-hydroxyformamide; (S)-*N*-[1-(4-Methoxyphenyl)-2-(3-quinolylmethanesulfonyl)ethyl]-*N*-hydroxyformamide; (S)-*N*-[1-(3,4-Dihydro-2*H*-benzo*[b]*[1,4]dioxepin-7-yl)-2-(3-quinolylmethanesulfonyl)ethyl]-*N*-hydroxyformamide; (S)-N-[1-(3-Quinolylmethanesulfonyl)-2-pentyl]-N-hydroxyformamide; (S)-N-[1-(3-Quinolylmethanesulfonyl)-2-butyl]-N-hydroxyformamide; (S)-N-[1-(3-Quinolylmethanesulfonyl)-4-methyl-2-pentyl]-N-hydroxyformamide; (S)-N-[1-(3-Quinolylmethanesulphonyl)-3-methoxy-2-propyl]-N-hydroxyformamide; (S)-*N*-[1-(3-Quinolylmethanesulfonyl)-3-methyl-2-butyl]-*N*-hydroxyformamide; (S)-*N*-[1-(3-Quinolylmethanesulfonyl)-3-isopropoxy-2-propyl]-N-hydroxyformamide;(S)-*N*-[1-(Benzo[*b*]thiophen-5-ylmethanesulfonyl)-3-isopropoxy-2-propyl]-N-hydroxyformamide; (S)-*N*-[1-(4-Methoxyphenyl)-2-(5-thieno*[2,3-b]*pyridylmethanesulfonyl)ethyl]-*N*-hydroxyformamide; (S)-*N*-[1-(4-Methoxyphenyl)-2-(6-thieno*[3,2-b]*pyridylmethanesulfonyl)ethyl]-*N*-hydroxyformamide; (S)-*N*-[(2-(Benzo*[b]*thiophen-5-yl-methanesulfonyl)-1-(3-oxo-3,4-dihydro-*2H*-benz[1,4]oxazin-6-yl)ethyl]-*N*-hydroxyformamide;(S)-*N*-[1-(4-Methoxyphenyl)-2-(3-isoquinolylmethanesulfonyl)ethyl]-*N*-hydroxyformamide; (S)-*N*-[1-(3,4-Dihydro-2*H*-benzo*[b]*[1,4]dioxepin-7-yl)-2-(3-isoquinolylmethanesulfonyl)ethyl]-*N*-hydroxyformamide; (S)-*N*-[1-(4-Methoxyphenyl)-2-(2-quinoxalylmethanesulfonyl)ethyl]-*N*-hydroxyformamide; (S)-*N*-[2-(2-Fluorobenzo*[b]*thiophen-5-yl-methanesulfonyl)-1-(4-methylphenyl)ethyl]-*N*-hydroxyformamide; (S)-*N*-[2-(2-Fluorobenzo*[b]*thiophen-5-yl-methanesulfonyl)-1-(4-methoxyphenyl)ethyl]-*N*-hydroxyformamide; (S)-*N*-[1-(3-Methylthiophen-2-yl)-2-(3-quinolylmethanesulfonyl)ethyl]-*N*-hydroxyformamide; (S)-*N*-[2-(3-Quinolylmethanesulfonyl)-1-(3-methoxycarbonyl-4-methoxyphenyl)ethyl]-*N*-hydroxyformamide; and (S)-*N*-[2-(3-Quinolylmethanesulfonyl)-1-(3-carboxy-4-methoxyphenyl)ethyl]-*N*-hydroxyformamide.

10. Use of a compound according to any preceding claim for the production of a medicament for the treatment or prophylaxis of disorders in which the overproduction of s-CD23 is implicated.

11. A pharmaceutical composition for the treatment or prophylaxis of disorders in which the overproduction of s-CD23 is implicated which comprises a compound according to any one of claims 1 to 9 and optionally a pharmaceutically acceptable carrier therefor.

12. A process for preparing a compound according to any one of claims 1 to 9 which process comprises:
(a) deprotecting a compound of formula (II): 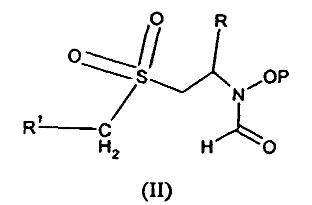 wherein R and R¹ are as defined hereinabove, and P is a protecting group, or
(b) converting a compound of formula (I) to a different compound of formula
(I) as defined hereinabove, or
(c) formylating a compound of formula (III) 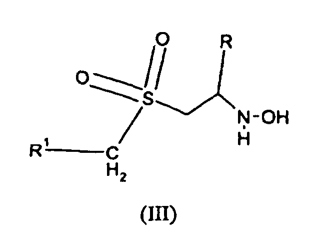 wherein R and R¹ are as defined hereinabove, or
(d) oxidising a compound of formula (X) 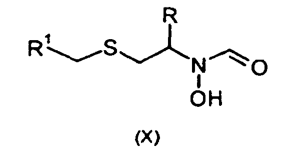
wherein R and R¹ are as defined hereinabove to give a compound of formula (I) as defined hereinabove.

13. A compound of formula (II): 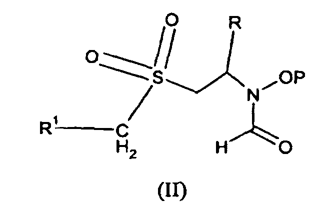 wherein R and R¹ are as defined hereinabove, and P is a protecting group.

14. A compound of formula (III): 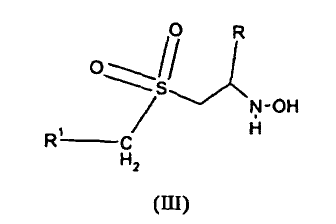 wherein R and R¹ are as defined hereinabove.

15. A compound of formula (X) 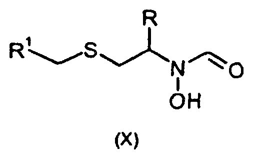 wherein R and R¹ are as defined hereinabove.

## Patentansprüche

1. Verbindung der Formel (I): 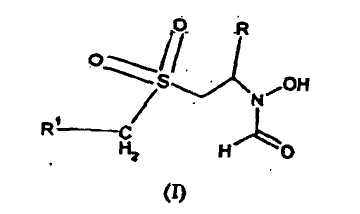 wobei
R ausgewählt ist aus Phenyl, das gegebenenfalls mit is zu drei Resten substituiert ist, die unabhängig voneinander ausgewählt sind aus Halogen, (C1-6)-Alkoxy, Di-N-(C1-6)-alkylaminosulfonyl, (C1-6)-Acylamino, Aryl(C1-6)-alkoxycarbonylamino, Amino, (C1-6)-Alkoxycarbonyl, Carboxy, Hydroxy und (C1-6)-Alkyl oder zwei Resten, die zusammen einen kondensierten Ring bilden; Benzothiophen, Thiophen, das gegebenenfalls durch (C1-6)-Alkyl, Carboxy oder (C1-6)-Alkoxycarbonyl substituiert ist; Furanyl; Pyrazol, das gegebenenfalls durch bis zu zwei (C1-6)-Alkylreste substituiert ist; (C1-6)-Alkyl, das gegebenenfalls durch (C1-6)-Alkoxy, Aryl(C1-6)-alkoxy oder Phenyl substituiert ist; Isoxazol, das gegebenenfalls durch (C1-6)-Alkyl substituiert ist; Benzodioxan; Benzodioxepin und Benzoxazin; und
R¹ Bicyclyl oder Heterobicyclyl ist, wobei Bicyelyl oder Heterobicyclyl kondensierte bicyclische Ringe mit 4-7 Ringatomen in jedem Ring bedeuten und wobei die Heterobicyclylringe bis zu 4 Heteroatome, ausgewählt aus Sauerstoff, Stickstoff und Schwefel, in jedem Ring enthalten.

2. Verbindung der Formel (IA): 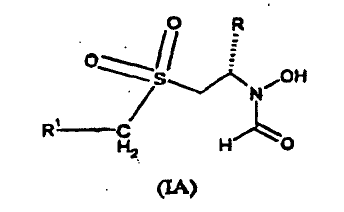 wobei
R und R¹ wie in Anspruch 1 definiert sind.

3. Verbindung gemäß Anspruch 1 oder Anspruch 2, wobei R¹ aus Benzothiophen, das gegebenenfalls durch Fluor substituiert ist; Indanyl; Benzofuranyl; Chinolyl; Naphthyl; Benzothiazol; Thienopyridyl; Isochinolyl und Chinoxalyl ausgewählt ist.

4. Verbindung gemäß einem der vorstehenden Ansprüche, wobei R ausgewählt ist aus Phenyl, das gegebenenfalls durch einen oder zwei Reste substituiert ist, die unabhängig voneinander ausgewählt sind aus Chlor, Fluor, -OCH₃, -SO₂N(CH₃)₂, -NHCOCH₃, NHCO₂CH₂Ph, Amino, Ethoxycarbonyl, Methoxycarbonyl, Carboxy, Hydroxy und Methyl; Benzothiophen-2-yl; Thiophen-2-yl, das gegebenenfalls durch Methyl, Carboxy oder Methoxycarbonyl substituiert ist; Pyrazol-3-yl, das gegebenenfalls durch Methyl und/oder t-Butyl substituiert ist; Furan-2-yl; Furan-3-yl; Methyl, Ethyl, n-Propyl, Isopropyl, Isobutyl oder Neopentyl, welche jeweils gegebenenfalls durch Methoxy, Isopropoxy, Benzyloxy oder Phenyl substituiert sind; Isoxazol-3-yl, das gegebenenfalls durch Methyl substituiert ist; 3-Oxo-3,4-dihydro-2H-benz[1,4]oxazin-6-yl; 3,4-Dihydro-2H-benzo[b][1,4]dioxepin-7-yl; und 2,3-Dihydrobenzo[1,4]dioxin-6-yl.

5. Verbindung gemäß einem der vorstehenden Ansprüche, wobei R¹ ausgewählt ist aus Benzothiophen-5-yl, das gegebenenfalls durch F substituiert ist; Indan-2-yl; Benzofuran-2-yl; Benzofuran-5-yl; Benzofuran-6-yl; Chinolin-3-yl; 2-Naphthyl; Benzothiazol-6-yl; Thicno[2,3-b]pyridin-5-yl; Thieno[3,2-b]pyridin-6-yl; Isochinolin-3-yl und Chinoxalin-2-yl.

6. Verbindung gemäß einem der vorstehenden Ansprüche, wobei R ausgewählt ist aus Phenyl; Phenyl, das durch einen oder zwei Reste substituiert ist, die unabhängig voneinander ausgewählt sind aus Chlor, Fluor, -OCH₃, -SO₂N(CH₃)₂, -NHCOCH₃, NHCO₂CH₂Ph, Amino, Ethoxycarbonyl, Methoxycarbonyl, Carboxy, Hydroxy und Methyl; Thiophen-2-yl, das gegebenenfalls durch Methyl substituiert ist; 3-Oxo-3,4-dihydro-2H-benz[1,4]oxazin-6-yl; 3,4-Dihydro-2H-benzo[b][1,4]dioxepin-7-yl, oder 2,3-Dihydrobenzo[1,4]dioxin-6-yl; Methyl, das gegebenenfalls durch Methoxy oder Isopropoxy substituiert ist; Ethyl; n-Propyl; Isopropyl und Isobutyl.

7. Verbindung gemäß einem der vorstehenden Ansprüche, wobei R¹ Benzothiophen-5-yl; Chinolin-3-yl; Thieno[2,3-b]pyridin-5-yl; oder Thieno[3,2-b]pyridin-6-yl ist.

8. Verbindung gemäß einem der vorstehenden Ansprüche, wobei R substituiertes oder unsubstituiertes Phenyl ist, wie Phenyl, das durch zwei Reste, die zusammen einen kondensierten Ring bilden, substituiert ist, und/oder R¹ Benzothiophen-5-yl, 3-Chinolinyl, Thieno[2,3-b]pyridin-5-yl oder Thieno[3,2-b]pyridin-6-yl ist.

9. Verbindung, ausgewählt aus
N-[2-(Benzo[b]thiophen-5-ylmethansulfonyl)-1-(3,4-dichlorphenyl)-ethyl]-N-hydroxyformamid,
N-[2-(Benzo[b]thiophen-5-ylmethansulfonyl)-1-phenyl-ethyl]-N-hydroxyformamid,
N-[2-(Benzo[b]thiophen-5-ylmethansulfonyl)-1-(4-methoxyphenyl)-ethyl]-N-hydroxyformamid, N-[2-(Benzo[b]thiophen-5-ylmethansulfonyl)-1-(2,3-dihydrobenzo[1,4]dioxin-6-yl)-ethyl]-N-hydroxyformamid,
N-[2-(Benzo[b]thiophen-5-ylmethansulfonyl)-1-(3,4-dihydro-2H-benzo[b][1,4]dioxepin-7-yl)-ethyl]-N-hydroxyformamid,
(S)-N-[2-(Benzo[b]thiophen-5-yl-methansulfonyl)-1-(4-methoxyphenyl)-ethyl]-N-hydroxyformamid,
(S)-N-[2-(Benzo[b]thiophen-5-yl-methansulfonyl)-1-(phenyl)-ethyl]-N-hydroxyformamid,
(S)-N-[2-(Benzo[b]thiophen-5-ylmethansulfonyl)-1-(2,3-dihydrobenzo[1,4]dioxin-6-yl)-ethyl]-N-hydroxyformamid,
(S)-N-[2-(Benzo[b]thiophen-5-ylmethansulfonyl)-1-(3,4-dichlorphenyl)-ethyl]-N-hydroxyformamid,
(S)-N-[2-(Benzo[b]thiophen-5-ylmethansulfonyl)-1-(3,4-dihydro-2Hbenzo[b][1,4]dioxepin-7-yl)-ethyl]-N-hydroxyformamid,
(S)-N-[2-(Benzo[b]thiophen-5-yl-methansulfonyl)-1-(2,4-difluorphenyl)-ethyl]-N-hydroxyformamid,
(S)-N-[2-(Benzo[b]thiophen-5-yl-methansulfonyl)-1-(3-hydroxyphenyl)-ethyl]-N-hydroxyformamid,
(S)-N-[2-(Benzo[b]thiophen-5-yl-methansulfonyl)-1-(4-dimethylaminosulfonylphenyl)-ethyl]-N-hydroxyformamid,
(S)-N-[2-(Benzo[b]thiophen-5-yl-methansulfonyl)-1-(3-methoxyphenyl)-ethyl]-N-hydroxyformamid,
(S)-N-[2-(Benzo[b]thiophen-5-yl-methansulfonyl)-1-(3-fluorphenyl)-ethyl]-N-hydroxyformamid,
(S)-N-[2-(Benzo[b]thiophen-5-yl-methansulfonyl)-1-(4-fluorophenyl)-ethyl]-N-hydroxyformamid;
(S)-N-[2-(Benzo[b]thiophen-5-yl-methansulfonyl)-1-(benzo[b]thiophen-2-yl)-ethyl]-N-hydroxyformamid,
(S)-N-[2-(Benzo[b]thiophen-5-yl-methansulfonyl)-1-(3-methylthiophen-2-yl)ethyl]-N-hydroxyformamid,
(S)-N-[2-(Benzo[b]thiophen-5-yl-methansulfonyl)-1-(1-t-butyl-5-methylpyrazol-3-yl)ethyl]-N-hydroxyformamid,
(S)-N-[2-(Benzo[b]thiophen-5-yl-methansulfonyl)-1-(furan-2-yl)ethyl]-N-hydroxyformamid,
(S)-N-[2-(Benzo[b]thiophen-5-yl-methansulfonyl)-1-(furan-3-yl)ethyl]-N-hydroxyformamid,
(S)-N-[1-Phenyl-2-(2-indanylmethansulfonyl)ethyl]-N-hydroxyformamid,
(S)-N-[1-Phenyl-2-(benzo[b]furan-5-yl-methansulfonyl)ethyl]-N-hydsoxyformamid,
(S)-N-[1-Phenyl-2-(2-fluor-benzo[b]thiophen-5-yl-methansulfonyl)ethyl]-N-hydroxyformamid,
(S)-N-[1-Phenyl-2-(3-fluor-benzo[b]thiophen-5-yl-methansulfonyl)ethyl]-N-hydroxyformamid,
(S)-N-[1-Phenyl-2-(3-chinolylmethansulfonyl)ethyl]-N-hydroxyformamid,
(S)-N-[2-(Benzo[b]furan-5-yl-methansulfonyl)-1-(4-methoxyphenyl)-ethyl]-N-hydroxyformamid,
(S)-N-[1-Benzo[b]thiophen-5-yl-methansulfonyl)-2-propyl]-N-hydroxyformamid,
(S)-N-[1-Benzyloxy-3-(benzo[b]thiophen-5-yl-methansulfonyl)-2-propyl]-N-hydroxyformamid,
(S)-N-[1-(Benzo[b]thiophen-5-yl-methansalfonyl)-3-phenyl-2-propyl]-N-hydroxyformamid,
(S)-N-[1-(Benzo[b]thiophen-5-yl-methansulfonyl)-4,4-dimethyl-2-pentyl]-N-hydroxyformamid,
(S)-N-[1-(Benzo[b]thiophen-5-yl-methansulfonyl)-3-methyl-2-butyl]-N-hydroxyformamid, N-[ 1-(Benzo[b]thiophen-5-yl-methansulfonyl)-4-methyl-2-pentyl]-N-hydroxyformamid;
(S)-N-[2-(Benzo[b]thiophen-5-yl-methansulfonyl)-1-(5-methylisoxazol-3-yl)-ethyl]-N-hydroxyformamid; (S)-N-[1-(4-Acetamidophenyl)-2-(benzo[b]thiophen-5-yl-methansulfonyl)ethyl]-N-hydroxyformamid; (S)-N-[2-(Benzo[b]thiophen-5-yl-methansulfonyl)-1-(5-carboxythiophen-2-yl)ethyl]-N-hydroxyformamid; (S)-N-[2-(Indan-2-ylmethansulfonyl)-1-(5-methoxycarbonylthiophen-2-yl)ethyl]-N-hydroxyformamid; (S)-N-[2-(Indan-2-ylmethansulfonyl)-1-(3-benzyloxycarbonylaminophenyl)ethyl]-N-hydroxyformamid; (S)-N-[2-(Indan-2-ylmethansulfonyl)-1-(3-aminophenyl)ethyl]-N-hydroxyformamid; (S)-N-[2-(Benzo[b]thiophen-5-ylmethansulfonyl)-1-(4-ethoxycarbonylphenyl)ethyl]-N-hydroxyformamid; (S)-N-[2-(Benzo[b]thiophen-5-ylmethansulfonyl)-1-(4-carboxyphenyl)ethyl]-N-hydroxyformamid; (S)-N-[2-(3-Chinolylmethansulfonyl)-1-(4-ethoxycarbonylphenyl)ethyl]-N-hydroxyformamid; (S)-N-[2-(3-Chinolylmethansulfonyl)-1-(4-carboxyphenyl)ethyl]-N-hydroxyformamid; (S)-N-[1-Phenyl-2-(benzo[b]furan-6-ylmethansulfonyl)ethyl]-N-hydroxyformamid; (S)-N-[1-Phenyl-2-(2-naphthylmethansulfonyl)ethyl]-N-hydroxyformamid; (S)-N-[1-Phenyl-2-(benzothiazol-6-ylmethansulfonyl)ethyl]-N-hydroxyformamid; (S)-N-[1-Phenyl-2-(benzo[b]furan-2-ylmethansulfonyl)ethyl]-N-hydroxyformamid; (S)-N-[1-Phenyl-2-(5-thieno[2,3-b]pyridylmethansulfonyl)ethyl]-N-hydroxyformamid; (S)-N-[1-Phenyl-2-(6-thieno[3,2-b]pyridylmethansulfonyl)ethyl]-N-hydroxyformamid; (S)-N-[1-(4-Methoxyphenyl)-2-(3-chinolylmethansulfonyl)ethyl]-N-hydroxyformamid; (S)-N-[1-(3,4-Dihydro-2H-benzo[b][1,4]dioxepin-7-yl)-2-(3-chinolylmethansulfonyl)ethyl]-N-hydroxyformamid; (S)-N-[1-(3-Chinolylmethansulfonyl)-2-pentyl]-N-hydroxyformamid;
(S)-N-[1-(3-Chinolylmethansulfonyl)-2-butyl]-N-hydroxyformamid; (S)-N-[1-(3-Chinolylmethansulfonyl)-4-methyl-2-pentyl]-N-hydroxyformamid; (S)-N-[1-(3-Chinolylmethansulfonyl)-3-methoxy-2-propyl]-N-hydroxyformamid; (S)-N-[1-(3-Chinolylmethansulfonyl)-3-methyl-2-butyl]-N-hydroxyformamid; (S)-N-[1-(3-Chinolylmethansulfonyl)-3-isopropoxy-2-propyl]-N-hydroxyformamid; (S)-N-[1-(Benzo[b]thiophen-5-ylmethansulfonyl)-3-isopropoxy-2-propyl]-N-hydroxyformamid; (S)-N-[1-(4-Methoxyphenyl)-2-(5-thieno[2,3-b]pyridylmethansulfonyl)ethyl]-N-hydroxyformamid; (S)-N-[1-(4-Methoxyphenyl)-2-(6-thieno[3,2-b]pyridylmethansulfonyl)ethyl]-N-hydroxyformamid; (S)-N-[(2-(Benzo[b]thiophen-5-yl-methansulfonyl)-1-(3-oxo-3,4-dihydro-2H-benz[1,4]oxazin-6-yl)ethyl]-N-hydroxyformamid; (S)-N-[1-(4-Methoxyphenyl)-2-(3-isochinolylmethansulfonyl)ethyl]-N-hydroxyformamid; (S)-N-[1-(3,4-Dihydro-2H-benzo[b][1,4]dioxepin-7-yl)-2-(3-isochinolylmethansulfonyl)ethyl]-N-hydroxyformamid; (S)-N-[1-(4-Methoxyphenyl)-2-(2-chinoxalylmethansulfonyl)ethyl]-N-hydroxyformamid;
(S)-N-[2-(2-Fluorbenzo[b]thiophen-5-yl-methansulfonyl)-1-(4-methylphenyl)ethyl]-N-hydroxyformamid; (S)-N-[2-(2-Fluorbenzo[b]thiophen-5-yl-methansulfonyl)-1-(4-methoxyphenyl)ethyl]-N-hydroxyformamid; (S)-N-[1-(3-Methylthiophen-2-yl)-2-(3-chinolylmethansulfonyl)ethyl]-N-hydroxyformamid; (S)-N-[2-(3-Chinolylmethansulfonyl)-1-(3-methoxycarbonyl-4-methoxyphenyl)ethyl]-N-hydroxyformamid; und (S)-N-[2-(3-Chinolylmethansulfonyl)-1-(3-carboxy-4-methoxyphenyl)ethyl]-N-hydroxyformamid.

10. Verwendung einer Verbindung nach einem der vorstehenden Ansprüche zur Herstellung eines Medikaments zur Behandlung oder Prophylaxe von Störungen, die die Überproduktion von s-CD23 einschließen.

11. Arzneimittel zur Behandlung oder Prophylaxe von Störungen, die die Überproduktion von s-CD23 einschließen, welches eine Verbindung nach einem der Ansprüche 1 bis 9 und gegebenenfalls einen pharmazeutisch verträglichen Träger dafür umfasst.

12. Verfahren zur Herstellung einer Verbindung nach einem der Ansprüche 1 bis 9, wobei das Verfahren umfasst:
(a) Entschützen einer Verbindung der Formel (II) 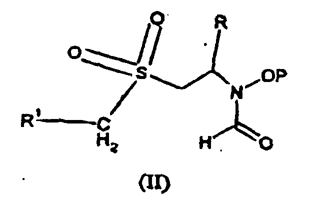 wobei R und R¹ wie vorstehend definiert sind, und P eine Schutzgruppe ist, oder
(b) Umwandeln einer Verbindung der Formel (1) in eine andere Verbindung der Formel (I) wie vorstehend definiert, oder
(c) Formylieren einer Verbindung der Formel (III): 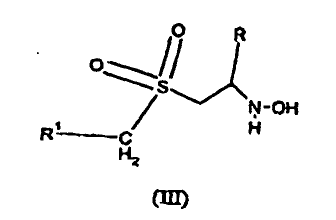 wobei R und R¹ wie vorstehend definiert sind, oder
(d) Oxidieren einer Verbindung der Formel (X) 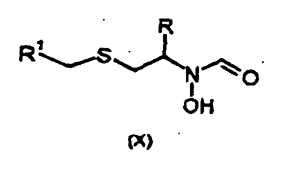
wobei R und R¹ wie vorstehend definiert sind, um eine Verbindung der Formel (I) wie vorstehend definiert zu erhalten.

13. Verbindung der Formel (II): 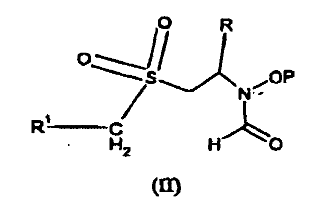 wobei R und R¹ wie vorstehend definiert sind, und P eine Schutzgruppe ist.

14. Verbindung der Formel (III): 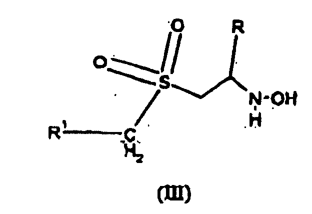 wobei R und R¹ wie vorstehend definiert sind.

15. Verbindung der Formel (X): 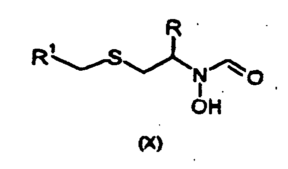 wobei R und R¹ wie vorstehend definiert sind.

## Revendications

1. Composé de formule (I) : 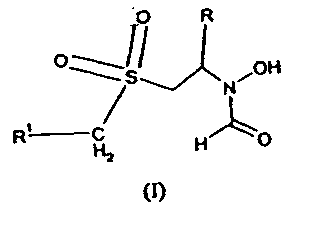 dans laquelle
R est choisi entre des groupes phényle facultativement substitué avec jusqu'à trois groupes choisis indépendamment entre des groupes halogéno, alkoxy en C₁ à C₆, di-N-(alkyle en C₁ à C₆)aminosulfonyle, acylamino en C₁ à C₆, aryl(alkoxy en C₁ à C₆)carbonylamino, amino, (alkoxy en C₁ à C₆)carbonyle, carboxy, hydroxy et alkyle en C₁ à C₆ ou bien deux groupes qui, conjointement, forment un noyau condensé ; benzothiophène, thiophène facultativement substitué avec un substituant alkyle en C₁ à C₆, carboxy ou (alkoxy en C₁ à C₆)carbonyle ; furannyle ; pyrazole facultativement substitué avec jusqu'à deux groupes alkyle en C₁ à C₆ ; alkyle en C₁ à C₆ facultativement substitué avec un substituant alkoxy en C₁ à C₆, aryl(alkoxy en C₁ à C₆) ou phényle ; isoxazole facultativement substitué avec un substituant alkyle en C₁ à C₆, benzodioxanne ; benzodioxépine ; et benzoxazine ; et
R¹ représente un groupe bicyclyle ou hétérobicyclyle, le terme "bicyclyle" ou "hétérobicyclyle" désignant des noyaux bicyclyle condensés contenant 4 à 7 atomes dans chaque noyau, et les noyaux hétérobicyclyle contenant dans chaque noyau jusqu'à 4 hétéroatomes choisis entre des atomes d'oxygène, d'azote et de soufre.

2. Composé de formule (IA) : 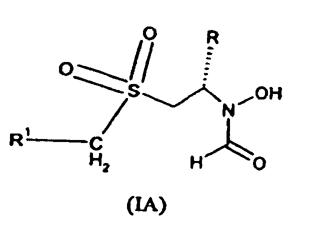 dans laquelle
R et R¹ répondent aux définitions figurant dans la revendication 1.

3. Composé suivant la revendication 1 ou la revendication 2, dans lequel R¹ est choisi entre des groupes benzothiophène facultativement substitué avec un substituant fluoro ; indanyle ; benzofurannyle ; quinolyle ; naphtyle ; benzothiazole ; thiénopyridyle ; isoquinolyle ; et quinoxalyle.

4. Composé suivant l'une quelconque des revendications précédentes, dans lequel R est choisi entre des groupes phényle facultativement substitué avec un ou deux groupes choisis indépendamment entre des groupes chloro, fluoro, -OCH₃, -SO₂N(CH₃)₂, -NHCOCH₃, NHCO₂CH₂Ph, amino, éthoxycarbonyle, méthoxycarbonyle, carboxy, hydroxy et méthyle ; benzothiophène-2-yle ; thiophène-2-yle facultativement substitué avec un substituant méthyle, carboxy ou méthoxycarbonyle ; pyrazole-3-yle facultativement substitué avec un substituant méthyle et/ou tertiobutyle ; furanne-2-yle ; furanne-3-yle ; méthyle, éthyle, n-propyle, isopropyle, isobutyle ou néopentyle chacun facultativement substitué avec un substituant méthoxy, isopropoxy, benzyloxy ou phényle ; isoxazole-3-yle facultativement substitué avec un substituant méthyle ; 3-oxo-3,4-dihydro-*2H*-benz[1,4]oxazine-6-yle ; 3,4-dihydro-*2H*-benzo[b] [1,4]dioxépine-7-yle ; et 2,3-dihydrobenzo[1,4]dioxine-6-yle.

5. Composé suivant l'une quelconque des revendications précédentes, dans lequel R¹ est choisi entre des groupes benzothiophène-5-yle facultativement substitué avec un substituant F ; indane-2-yle ; benzofuranne-2-yle ; benzofuranne-5-yle ; benzofuranne-6-yle ; quinoléine-3-yle ; 2-naphtyle ; benzothiazole-6-yle ; thiéno[2,3-b]pyridine-5-yle ; thiéno[3,2-b]pyridine-6-yle ; isoquinoléine-3-yle ; et quinoxaline-2-yle.

6. Composé suivant l'une quelconque des revendications précédentes, dans lequel R est choisi entre des groupes phényle ; phényle substitué avec un ou deux groupes choisis indépendamment entre des groupes chloro, fluoro, -OCH₃, -SO₂N(CH₃)₂, -NHCOCH₃, NHCO₂CH₂Ph, amino, éthoxycarbonyle, méthoxycarbonyle, carboxy, hydroxy et méthyle ; thiophène-2-yle facultativement substitué avec un substituant méthyle ; 3-oxo-3,4-dihydro-*2H*-benz[1,4]oxazine-6-yle ; 3,4-dihydro-*2H*-benzo[b][1,4]dioxépine-7-yle ; ou 2,3-dihydrobenzo[1,4]dioxine-6-yle ; méthyle facultativement substitué avec un substituant méthoxy ou isopropoxy ; éthyle ; *n*-propyle ; isopropyle ; et isobutyle.

7. Composé suivant l'une quelconque des revendications précédentes, dans lequel R¹ représente un groupe benzothiophène-5-yle ; quinoléine-3-yle ; thiéno[2,3-b]pyridine-5-yle ; ou thiéno[3,2-b]pyridine-6-yle.

8. Composé suivant l'une quelconque des revendications précédentes, dans lequel R représente un groupe phényle substitué ou non substitué, tel qu'un groupe phényle substitué avec deux groupes qui, conjointement, forment un noyau condensé, et/ou R¹ représente un groupe benzothiophène-5-yle, 3-quinolinyle, thiéno[2,3-b]pyridine-5-yle ou thiéno[3,2-b]pyridine-6-yle.

9. Composé choisi dans le groupe consistant en
*N*-[2-(benzo[b]thiophène-5-ylméthanesulfonyl)-1-(3,4-dichlorophényl)-éthyl]-*N*-hydroxyformamide,
*N*-[2-(benzo[b]thiophène-5-ylméthanesulfonyl)-1-phényléthyl]-*N*-hydroxyformamide,
N- [2- (benzo[b]thiophène-5-ylméthanesulfonyl)-1- (4-méthoxyphényl)-éthyl]-*N*-hydroxyformamide,
*N*-[2-(benzo[b]thiophène-5-ylméthanesulfonyl)-1-(2,3-dihydrobenzo-[1,4]dioxine-6-yl)éthyl]-*N*-hydroxyformamide,
N-[2-(benzo[b]thiophène-5-ylméthanesulfonyl)-1-(3,4-dihydro-2*H*-benzo[b][1,4]dioxépine-7-yl)éthyl]-*N*-hydroxyformamide,
(S)-*N*-[2-(benzo*[b]*thiophène-5-ylméthanesulfonyl)-1-(4-méthoxyphényl)éthyl]-*N*-hydroxyformamide,
(S)-*N*-[2-(benzo*[b]*thiophène-5-ylméthanesulfonyl)-1-(phényl)éthyl]-N-hydroxyformamide,
(S)-*N*-[2-(benzo[b]thiophène-5-ylméthanesulfonyl)-1-(2,3-dihydrobenzo[1,4]dioxine-6-yl)éthyl]-*N*-hydroxyformamide,
(S)-*N*-[2-(benzo[b]thiophène-5-ylméthanesulfonyl)-1-(3,4-dichlorophényl)éthyl]-*N*-hydroxyformamide,
(S)-*N*-[2-(benzo*[b]*thiophène-5-ylméthanesulfonyl)-1-(3,4-dihydro-*2H*-benzo*[b]*[1,4]dioxépine-7-yl)éthyl-*N*-hydroxyformamide,
(S)-*N*-[2-(benzo*[b]*thiophène-5-ylméthanesulfonyl)-1-(2,4-difluorophényl)éthyl]-*N*-hydroxyformamide,
(S)-*N*-[2-(benzo*[b]*thiophène-5-ylméthanesulfonyl)-1-(3-hydroxyphényl)éthyl]-*N*-hydroxyformamide,
(S)-*N*-[2-(benzo*[b]*thiophène-5-ylméthanesulfonyl)-1-(4-diméthylaminosulfonylphényl)éthyl]-*N*-hydroxyformamide,
(S)-*N*-[2-(benzo*[b]*thiophène-5-ylméthanesulfonyl)-1-(3-méthoxyphényl)éthyl]-*N*-hydroxyformamide,
(S)-*N*-[2-(benzo*[b]*thiophène-5-ylméthanesulfonyl)-1-(3-fluorophényl)-éthyl]-*N*-hydroxyformamide,
(S)-*N*-[2-(benzo*[b]*thiophène-5-ylméthanesulfonyl)-1-(4-fluorophényl)-éthyl]-*N*-hydroxyformamide,
(S)-*N*-[2-(benzo*[b]*thiophène-5-ylméthanesulfonyl)-1-(benzo*[b]*thiophène-2-yl)éthyl]-*N*-hydroxyformamide,
(S)-*N*-[2-(benzo*[b]*thiophène-5-ylméthanesulfomyl)-1-(3-méthylthiophène-2-yl)éthyl]-*N*-hydroxyformamide,
(S)-*N*-[2-(benzo*[b]*thiophène-5-ylméthanesulfonyl)-1-(1-tertiobutyl-5-méthylpyrazole-3-yl)éthyl]-*N*-hydroxyformamide,
(S)-*N*-[2-(benzo*[b]*thiophène-5-ylméthanesulfonyl)-1-(furanne-2-yl)éthyl]-*N*-hydroxyformamide,
(S)-*N*-[2-(benzo*[b]*thiophène-5-ylméthanesulfonyl)-1-(furanne-3-yl)éthyl]-*N*-hydroxyformamide,
(S)-N-[1-phényl-2-(2-indanylméthanesulfonyl)éthyl]-*N*-hydroxyformamide,
(S)-N-[1-phényl-2-(benzo*[b]*furanne-5-ylméthanesulfonyl)éthyl]-*N*-hydroxyformamide,
(S)-*N*-[1-phényl-2-(2-fluorobenzo*[b]*thiophène-5-ylméthanesulfonyl)-éthyl]-*N*-hydroxyformamide,
(S)-*N*-[1-phényl-2-(3-fluorobenzo*[b]*thiophène-5-ylméthanesulfony)-éthyl]-*N*-hydroxyformamide,
(S)-*N*-[1-phényl-2-(3-quinolylméthanesulfonyl)éthyl]-*N*-hydroxyformamide,
(S)-*N*-[2-(benzo *[b]*furanne-5-ylméthanesulfonyl)-1-(4-méthoxyphényl)-éthyl]-*N*-hydroxyformamide,
(S)-*N*-[1-(benzo*[b]*thiophène-5-ylméthanesulfonyl)-2-propyl]-*N*-hydroxyformamide,
(S)-*N*-[1-(benzyloxy-3-(benzo*[b]*thiophène-5-ylméthanesulfonyl)-2-propyl]-*N*-hydroxyformamide,
(S)-*N*-[1-(benzo*[b]*thiophène-5-ylméthanesulfonyl)-3-phényl-2-propyl]-*N*-hydroxyformamide,
(S)-*N*-[1-(benzo*[b]*thiophène-5-ylméthanesulfonyl)-4,4-diméthyl-2-pentyl]-*N*-hydroxyformamide,
(S)-*N*-[1-(benzo*[b]*thiophène-5-ylméthanesulfonyl)-3-méthyl-2-butyl]-*N*-hydroxyformamide ;
*N*-[1-benzo*[b]*thiophène-5-yl-méthanesulfonyl)-4-méthyl-2-pentyl]-*N*-hydroxyformamide ;
(S)-*N*-[2-(benzo*[b]*thiophène-5-ylméthanesulfonyl)-1-(5-méthylisoxazole-3-yl)éthyl]-*N*-hydroxyformamide ;
(S)-*N*-[1-(4-acétamidophényl)-2-(benzo*[b]*thiophène-5-yl-méthanesulfonyl)éthyl]-*N*-hydroxyformamide ;
(S)-*N*-[2-(benzo*[b]*thiophène-5-ylméthanesulfonyl)-1-(5-carboxythiophène-2-yl)éthyl]-*N*-hydroxyformamide ;
(S)-*N*-[2-(indane-2-ylméthanesulfonyl)-1-(5-méthoxycarbonylthiophène-2-yl)éthyl]-*N*-hydroxyformamide ;
(S)-*N*-[2-(indane-2-ylméthanesulfonyl)-1-(3-benzyloxycarbonylaminophényl)éthyl]-*N*-hydroxyformamide ;
(S)-*N*-[2-(indane-2-ylméthanesulfonyl)-1-(3-aminophényl)éthyl]-*N*-hydroxyformamide ;
(S)-*N*-[2-(benzo*[b)*thiophène-5-ylméthanesulfonyl)-1-(4-éthoxycarbonylphényl)éthyl]-*N*-hydroxyformamide ;
(S)-*N*-[2-(benzo *[b)*thiophène-5-ylméthanesulfonyl)-1-(4-carbonylphényl)éthyl]-*N*-hydroxyformamide ;
(S)-*N*-[2-(3-quinolylméthanesulfonyl)-1-(4-éthoxycarbonylphényl)éthyl]-*N*-hydroxyformamide ;
(S)-*N*-[2-(3-quinolylméthanesulfonyl)-1-(4-carboxyphényl)éthyl]-*N*-hydroxyformamide ;
(S)-N-[1-phényl-2-(benzo*[b]*furanne-6-ylméthanesulfonyl)éthyl]-*N*-hydroxyformamide ;
(S)-N-[1-phényl-2-(2-napthylméthanesulfonyl)éthyl]-*N*-hydroxyformamide ;
(S)-N-[1-phényl-2-(benzothiazole-6-ylméthanesulfonyl)éthyl]-*N*-hydroxyformamide ;
(S)-N-[1-phényl-2-(benzo*[b]*furanne-2-ylméthanesulfonyl)éthyl]-*N*-hydroxyformamide ;
(S) -*N*- [1-phényl-2-(5-thiéno*[2,3-b]*pyridylméthanesulfonyl)-éthyl]-*N*-hydroxyformamide ;
(S)-*N*-[1-phényl-2-(6-thiéno*[3,2-b*]pyridylméthanesulfonyl)-éthyl]-*N*-hydroxyformamide ;
(S)-*N*-[1-(4-méthoxyphényl)-2-(3-quinolylméthanesulfonyl)éthyl]-*N*-hydroxyformamide ;
(S) -*N*- [1-(3,4-dihydro-2*H*-benzo*[b]*[1,4]dioxépine-7-yl)-2- (3-quinolylméthanesulfonyl)éthyl]-*N*-hydroxyformamide ;
(S) -N- [1-(3-quinolylméthanesulfonyl)-2-pentyl]-N-hydroxyformamide ;
(S)-N-[1-(3-quinolylméthanesulfonyl)-2-butyl]-N-hydroxyformamide ;
(S)-N-[1-(3-quinolylméthanesulfonyl)-4-méthyl-2-pentyl]-N-hydroxyformamide ;
(S)-N-[1-(3-quinolylméthanesulfonyl)-3-méthoxy-2-propyl]-N-hydroxyformamide ;
(S)-*N*-[1-(3-quinolylméthanesulfonyl)-3-méthyl-2-butyl]-*N*-hydroxyformamide ;
(S)-*N*-[1-(3-quinolylméthanesulfonyl)-3-isopropoxy-2-propyl]-*N*-hydroxyformamide ;
(S)-*N*-[1-benzo*[b]*thiophène-5-ylméthanesulfonyl)-3-isopropoxy-2-propyl]-*N*-hydroxyformamide ;
(S)-*N*-[1-(4-méthoxyphényl)-2-(5-thiéno*[2,3-b]*pyridylméthanesulfonyl)éthyl]-N-hydroxyformamide ;
(S)-*N*-[1-(4-méthoxyphényl)-2-(6-thiéno*[3,2-b]*pyridylméthanesulfonyl)éthyl]-N-hydroxyformamide ;
(S)-*N*-[(2-(benzo[b]thiophène-5-ylméthanesulfonyl)-1-(3-oxo-3,4-dihydro-2*H*-benz[1,4]oxazine-6-yl)éthyl]-*N*-hydroxyformamide ;
(S)-*N*-[1-(4-méthaxyphényl)-2-(3-isoquinolylméthane-sulfonyl)éthyl]-*N*-hydroxyformamide ;
(S)-*N*-[1-(3,4-dihydro-2*H*-benzo*[b]*[1,4]dioxépine-7-yl)-2-(3-isoquinolylméthanesulfonyl)éthyl]-*N*-hydroxyformamide ;
(S)-*N*-[1-(4-méthoxyphényl)-2-(2-quinoxalylméthanesulfonyl)éthyl]-*N*-hydroxyformamide ;
(S)-*N*-[2-(2-fluorobenzo*[b]*thiophène-5-ylméthanesulfonyl)-1-(4-méthylphényl)éthyl]-*N*-hydroxyformamide ;
(S)-*N*-[2-(2-fluorobenzo*[b]*thiophène-5-ylméthanesulfonyl)-1-(4-méthylphényl)éthyl]-*N*-hydroxyformamide ;
(S)-*N*-[1-(3-méthylthiophène-2-yl)-2-(3-quinolylméthanesulfonyl)éthyl-*N*-hydroxyformamide ;
(S)-*N*-[2-(3-quinolylméthanesulfonyl)-1-(3-méthoxycarbonyl-4-méthoxyphényl)éthyl]-*N*-hydroxyformamide ; et
(S)-*N*-[2-(3-quinolylméthanesulfonyl)-1-(3-carboxy-4-méthoxyphényl)-éthyl]-*N*-hydroxyformamide ;

10. Utilisation d'un composé suivant l'une quelconque des revendications précédentes pour la production d'un médicament destiné au traitement ou à la prophylaxie d'affections dans lesquelles la surproduction de s-CD23 est impliquée.

11. Composition pharmaceutique pour le traitement ou la prophylaxie d'affections dans lesquelles la surproduction de s-CD23 est impliquée, qui comprend un composé suivant l'une quelconque des revendications 1 à 9 et éventuellement un support pharmaceutiquement acceptable à cette fin.

12. Procédé pour la préparation d'un composé suivant l'une quelconque des revendications 1 à 9, procédé qui comprend :
(a) la suppression de la protection d'un composé de formule (II) : 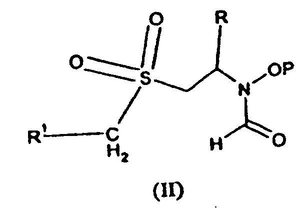 dans laquelle R et R¹ répondent aux définitions précitées et P représente un groupe protecteur, ou
(b) la conversion d'un composé de formule (I) en un composé différent de formule (I) répondant à la définition précitée, ou
(c) la formylation d'un composé de formule (III) 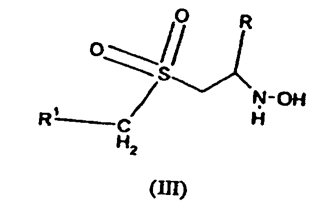 dans laquelle R et R¹ répondent aux définitions précitées, ou
(d) l'oxydation d'un composé de formule (X) 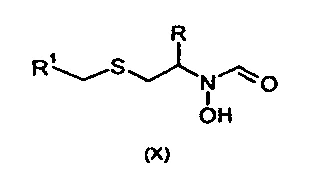
dans laquelle R et R¹ répondent aux définitions précitées, ce qui donne un composé de formule (I) répondant à la définition précitée.

13. Composé de formule (II) : 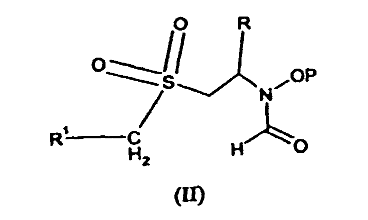 dans laquelle R et R¹ répondent aux définitions précitées et P représente un groupe protecteur.

14. Composé de formule (III) : 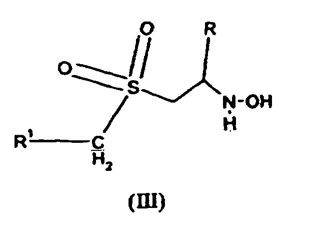 dans laquelle R et R¹ répondent aux définitions précitées.

15. Composé de formule (X) : 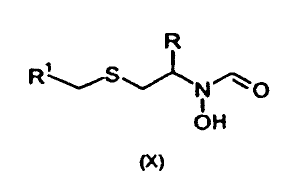 dans laquelle R et R¹ répondent aux définitions précitées.
